# EUROPEAN PATENT APPLICATION

(11) **EP 4 144 349 A1**
(43) Date of publication of application: **08.03.2023**
(21) Application number: 20892292.2
(22) Date of filing: 30.11.2020
(51) Int. Cl.: A61K 31/198, A61K 31/085, A61P 9/00, C12Q 1/6869, C12Q 1/6883, C12N 15/113, C12N 15/85, A01K 67/027

(54) **RNA INTERFERENCE-INDUCING NUCLEIC ACID COMPRISING 8-OXOGUANINE, MODIFIED NUCLEIC ACID BINDING TO MICRORNA COMPRISING 8-OXOGUANINE, AND USES THEREOF**

(30) Priority: 28.11.2019 KR 20190156147
(71) Applicant: Korea University Research and Business Foundation, Seoul 02841 (KR)
(72) Inventor: CHI, Sung Wook, Seoul 06362 (KR); SEOK, Hee Young, Seoul 06337 (KR)
(74) Representative: Elkington and Fife LLP
(86) International application number: PCT/KR2020/017341
(87) International publication number: WO 2021/107747

(57) **Abstract**

In the present invention, it has been confirmed that, when an RNA interference-inducing nucleic acid including at least one 8-oxoguanine (o⁸G) in 1st to 9th nucleotides from the 5'-end of at least one single strand of a double strand of a nucleic acid, and a modified nucleic acid that specifically binds to microRNA and in which at least one guanine (G) from among the 1st to 9th nucleotides from the 5'-end are modified with 8-oxoguanine (o⁸G), are produced and administered to cells or mice, various pathophysiological phenomena are induced.

In addition, the positions where G>T modifications occur have been identified in cDNA produced through the reverse transcription of microRNA in which guanine (G) is oxidatively modified with 8-oxoguanine (o⁸G) by oxidative stress in a seed region of microRNA, to confirm the positions where oxidative modification to 8-oxoguanine has occurred.

## Description

### Technical Field

The present invention relates to an RNA interference-inducing nucleic acid including 8-oxoguanine, a modified nucleic acid that specifically binds to a microRNA including 8-oxoguanine, and a pharmaceutical composition using the same, a recombinant animal model, a drug screening method, a diagnosis method of a disease, and a method for controlling pathophysiological phenomena.

### Background Art

When a cell undergoes pathophysiological changes, oxidative stress generally occurs, thereby generating reactive oxygen species (ROS). The generated reactive oxygen species modify various biological constructs due to their great reactivity, and RNA is the most easily modified among them. In particular, the most easily occurring among the oxidatively modified RNA bases is that the guanine (G) base is oxidatively modified and transformed into 8-oxoguanine (o8G).

On the other hand, heart disease is one of the three major causes of death among adults in Korea, including cancer, and it starts with various pathological stresses in the course of its onset that cause changes in the size of the cardiac tissue and cause cardiac hypertrophy. The cardiac hypertrophy is characterized by an increase in the size of cardiomyocytes and an increase in the rate of protein synthesis. The cardiac hypertrophy gradually induces myocardial fibrosis and heart failure, eventually resulting in cardiac failure (or heart failure). In particular, in the case of heart failure, since a mortality rate is very high at around 50 %, studies to ultimately prevent or develop a treatment for myocardial hypertrophy are in progress, and thus it is necessary to establish a disease model for this.
Korean Patent Publication No. 1481007

### Disclosure

### Technical Problem

The present inventors have confirmed that when oxidative modification of guanine (G) base to 8-oxoguanine (o⁸G) in the seed region of microRNA due to oxidative stress occurs, it binds to the target sequence through a o⁸G:A arrangement, and also has confirmed a position where oxidative modification to 8-oxoguanine has occurred in the produced cDNA by identifying a position where guanine (G) is modified to thymine (T), i.e., G>T modification occurs in the cDNA produced through reverse transcription of the microRNA. Therefore, it has been confirmed that various pathophysiological phenomena are induced by an RNA interference-inducing nucleic acid in which guanine (G) is modified to 8-oxoguanine (o⁸G) in the nucleotides of a microRNA, when being administered to a cell or mice, and based on this, the present invention has been completed.

Accordingly, the present invention provides an RNA interference-inducing nucleic acid, which includes at least one 8-oxoguanine (o⁸G) in the 1st to 9th nucleotides from a 5'-end of at least one single strand of double strands thereof.

In addition, the present invention provides a method for identifying a position of 8-oxoguanine (o⁸G).

In addition, the present invention provides a modified nucleic acid that specifically binds to a microRNA in which at least one guanine (G) of the 1st to 9th nucleotides from a 5'-end is modified to 8-oxoguanine (o⁸G), and a recombinant vector including a gene encoding the modified nucleic acid.

In addition, the present invention provides a pharmaceutical composition for treating cardiac hypertrophy including the modified nucleic acid or the recombinant vector, and a pharmaceutical composition for treating liver cancer or glioblastoma including the RNA interference-inducing nucleic acid.

Another embodiment provides a pharmaceutical composition for preventing or treating cardiac hypertrophy including an antioxidant as an active ingredient, and a method for providing information for diagnosing cardiac hypertrophy.

Another embodiment provides a method for producing an animal model resistant to cardiac hypertrophy and an animal model resistant to cardiac hypertrophy.

Another embodiment provides a method for screening a candidate substance for the treatment of cardiac hypertrophy.

The technical objects to be achieved by the present invention are not limited to the objects mentioned above, and other objects not mentioned will be clearly understood by those of ordinary skill in the art to which the present invention belongs from the description below.

### Technical Solution

The present invention provides an RNA interference-inducing nucleic acid including at least one 8-oxoguanine (o⁸G) in the 1st to 9th nucleotides from a 5'-end of at least one single strand of double strands of a nucleic acid.

An embodiment of the present invention provides an RNA interference-inducing nucleic acid in which the 1st to 9th nucleotides from the 5'-end includes a base sequence of a microRNA.

In an embodiment of the present invention, the microRNA may be at least one of the following microRNAs of Group 1:
[Group 1]
miR-1, miR-184, let-7f-5p, miR-1-3p, miR-122, let-7, and miR-124.

In another embodiment of the present invention, the RNA interference-inducing nucleic acid may recognize a target site in which the o⁸G:A arrangement occurs at the position of 8-oxoguanine (o⁸G).

In another embodiment of the present invention, the at least one single strand of double-strands of a nucleic acid may include at least one of the following polynucleotides of Group 2:
[Group 2]
a polynucleotide of SEQ ID NO: 1 (5'p-Uo⁸GGAAUGUAAAGAAGUAUGUAU-3');
a polynucleotide of SEQ ID NO: 2 (5'p-UGo⁸GAAUGUAAAGAAGUAUGUAU-3');
A polynucleotide of SEQ ID NO: 3 (5'p-UGGAAUo^{B}GUAAAGAAGUAUGUAU-3');
A polynucleotide of SEQ ID NO: 65 (5'p-Uo⁸Go⁸GAGUGUGACAAUGGUGUUUG-3');
a polynucleotide of SEQ ID NO: 66 (5'p-UGAo⁸GUAGUAGGUUGUAUAGdTdT-3'); and
a polynucleotide of SEQ ID NO: 67 (5'p-UAAo⁸GGCACGCGGUGAAUGCdTdT-3').

When the RNA interference-inducing nucleic acid according to the present invention is injected into cells or animals, myocardial hypertrophy, inhibition of migration of liver cancer cells, or apoptosis may be induced.

The present invention provides a composition including the aforementioned RNA interference-inducing nucleic acid and an antioxidant.

In addition, the present invention provides a method for identifying a position of 8-oxoguanine (o⁸G) which includes:
(a) extracting RNA from a cell;
(b) isolating RNA that includes 8-oxoguanine (o⁸G) from the extracted RNA by immunoprecipitation (IP) using an anti-o⁸G antibody;
(c) producing cDNA by reverse transcription of the isolated RNA that includes 8-oxoguanine (o⁸G) to produce and sequence a sequencing library for determining the position of 8-oxoguanine; and
(d) identifying the position at which guanine (G) is substituted with thymine (T) as the position where guanine (G) is modified to 8-oxoguanine (o⁸G).

In addition, the present invention provides a modified nucleic acid that specifically binds to a modified microRNA in which at least one guanine (G) in the 1st to 9th nucleotides from a 5'-end is modified to 8-oxoguanine (o⁸G),
wherein the modified nucleic acid includes a polynucleotide complementary to 6 or more consecutive polynucleotides starting from the second or third nucleotide from the 5'-end of the modified microRNA, and
the modified nucleic acid includes adenine (A) that binds to the at least one 8-oxoguanine (o⁸G) among the 1st to 9th nucleotides from the 5'-end of the microRNA.

In an embodiment of the present invention, in the microRNA that specifically binds to the modified nucleic acid, at least one guanine (G) of the 2nd, 3rd, and 7th nucleotides from the 5'-end of the microRNA may be modified to 8-oxoguanine (o⁸G), and the modified nucleic acid may include a polynucleotide complementary to 6 or more consecutive polynucleotides starting from either the 2nd or 3rd nucleotide from the 5'-end of the microRNA, and adenine (A) as a nucleotide at the position that binds to the at least one 8-oxoguanine among the 2nd, 3rd, or 7th nucleotides from the 5'-end of the microRNA.

In an embodiment of the present invention, the modified nucleic acid may include a base sequence of 5'-ACAUUCA-3' (SEQ ID NO: 4), 5'-ACAUUAC-3' (SEQ ID NO: 5), or 5'-AAAUUCC-3' (SEQ ID NO: 6).

In addition, the present invention provides a recombinant vector including a gene encoding the aforementioned modified nucleic acid.

In addition, the present invention provides a pharmaceutical composition for treating cardiac hypertrophy. The pharmaceutical composition for treating cardiac hypertrophy includes a modified nucleic acid that specifically binds to microRNA and in which at least one guanine (G) among the 1st to 9th nucleotides from the 5'-end is modified to 8-oxoguanine (o⁸G), or a recombinant vector including a gene encoding the modified nucleic acid, wherein the modified nucleic acid including a polynucleotide complementary to 6 or more consecutive polynucleotides starting from the second or the third nucleotide from the 5'-end of the microRNA, and adenine (A) as a nucleotide at the position that binds to the at least one 8-oxoguanine (o⁸G) among the 1st to 9th nucleotides from the 5'-end of the microRNA.

In an embodiment of the present invention, the microRNA may be miR-1, miR-184, let-7f-5p, or miR-1-3p.

As an embodiment of the present invention, the pharmaceutical composition for treating cardiac hypertrophy may further include an antioxidant.

In addition, the present invention provides a pharmaceutical composition for treating liver cancer or glioblastoma including an RNA interference-inducing nucleic acid that includes at least one 8-oxoguanine (o⁸G) in the 1st to 9th nucleotides from a 5'-end of at least one single strand of double strands thereof.

In an embodiment of the present invention, in the pharmaceutical composition for treating liver cancer, the microRNA may be miR-122.

In another embodiment of the present invention, in the pharmaceutical composition for treating glioblastoma, the microRNA may be let-7 or miR-124.

In another embodiment of the present invention, the pharmaceutical composition for treating liver cancer or glioblastoma may further include an antioxidant.

In addition, the present invention provides a pharmaceutical composition for preventing or treating cardiac hypertrophy including an antioxidant as an active ingredient,
wherein the antioxidant inhibits the oxidative modification of one or more guanine (G) to 8-oxoguanine (o⁸G) among the 1st to 9th nucleotides from the 5'-end of RNA.

In an embodiment of the present invention, the antioxidant may be N-acetylcysteine (NAC) or butylhydroxyanisole (BHA).

In another embodiment of the present invention, the RNA may be miR-1, miR-184, let-7f-5p, or miR-1-3p.

In addition, the present invention provides a method for providing information for diagnosing cardiac hypertrophy that includes:
determining whether a guanine (G) among nucleotides of a microRNA expressed in a cardiomyocyte of an animal is modified to 8-oxoguanine (o⁸G); and
classifying it as cardiac hypertrophy when a guanine (G) of the nucleotides of the microRNA is modified to 8-oxoguanine (o⁸G).

In an embodiment of the present invention, the nucleotides may be the 1st to 9th nucleotides from the 5'-end of the microRNA.

In another embodiment of the present invention, the nucleotides may be the 2nd, 3rd, and 7th nucleotides from the 5'-end of the microRNA.

In another embodiment of the present invention, the microRNA may be miR-1, miR-184, let-7f-5p, or miR-1-3p.

In addition, the present invention provides a method for producing a non-human animal model resistant to cardiac hypertrophy, and a non-human animal model resistant to cardiac hypertrophy produced by the method, which includes:
(a) operably linking the gene encoding the modified nucleic acid to a promoter to construct a recombinant vector;
(b) introducing the recombinant vector into a fertilized egg of an animal; and
(c) generating the fertilized egg after transplanting it into a surrogate mother to obtain a transgenic animal model.

In addition, the present invention provides a method for screening a candidate substance for the treatment of cardiac hypertrophy, which includes:
(a) treating a cardiomyocyte of an animal model of cardiac hypertrophy with a candidate substance;
(b) analyzing frequency of modification of guanine (G) to 8-oxoguanine (o⁸G) among nucleotides of microRNA expressed in the cardiomyocyte of the animal model of cardiac hypertrophy; and
(c) selecting the candidate substance as a cardiac hypertrophy therapeutic agent, when the frequency of modification of guanine (G) to 8-oxoguanine (o⁸G) decreases compared to the case in which the candidate substance is not treated,.

In an embodiment of the present invention, the candidate substance may be an antioxidant.

In another embodiment of the present invention, the microRNA may be miR-1.

In addition, the present invention provides a method for inhibiting cardiac hypertrophy including administering the modified nucleic acid to a subject.

In addition, the present invention provides a method for inhibiting liver cancer metastasis or a method for treating glioblastoma including administering the RNA interference-inducing nucleic acid to a subject.

In addition, the present invention provides a method for preventing or treating cardiac hypertrophy that includes administering a composition including an antioxidant as an active ingredient to a subject.

In addition, the present invention provides a use of the modified nucleic acid for inhibiting cardiac hypertrophy.

In addition, the present invention provides a use of the RNA interference-inducing nucleic acid for inhibiting liver cancer metastasis and treating liver cancer or glioblastoma.

In addition, the present invention provides a use for preventing or treating cardiac hypertrophy of a composition including an antioxidant as an active ingredient.

The present invention also provides a use of an antioxidant for the production of a medicament for use in the treatment of cardiac hypertrophy.

### Advantageous Effects

The RNA interference-inducing nucleic acid and modified nucleic acid that specifically binds to a microRNA in which at least one guanine (G) of the 1st to 9th nucleotides from the 5'-end is modified to 8-oxoguanine (o⁸G) according to the present invention may be used to control pathophysiological phenomena in a cell or animal.

Specifically, it can be used for diagnosis and development of therapeutic agents for cardiac hypertrophy, liver cancer, or glioblastoma.

### Description of the Drawings

FIG. 1A is a schematic view of an intraperitoneal (I.P) injection of 75 mg/kg isoproterenol (ISO) every 2 days for 29 days in mice (n=7).
FIG. 1B is a view confirming changes of mouse heart sizes by ISO and NAC treatment.
FIG. 1C is a view confirming the RNA oxidation action of ROS produced by ISO treatment.
FIG. 1D is a view showing the results of confirming that the o⁸G modification of microRNAs according to PE or ISO treatment in rCMC cells appeared together in the immunofluorescence staining of Ago2 and o⁸G.
FIG. 1E is a view showing the immunofluorescence staining results of o⁸G and Ago2 according to PE or ISO treatment in H9c2.
FIG. 1F is a view showing the results of dot blot analysis using o⁸G-specific antibodies in H9c2 and rCMC cells.
FIG. 1G is a view showing the results of northwestern analysis in PE-treated H9c2 cells (top of FIG. 1G) and ISO-treated mouse hearts (bottom of FIG. 1G).
FIG. 1H is a view showing the results of dot blot analysis using about 20 nt miRNA gel-extracted for ISO-treated mouse heart.
FIG. 2A shows a schematic view of the o⁸G sequencing method (o⁸G-miSeq).
FIG. 2B is a view showing the immunoprecipitation (IP) optimization process for o⁸G.
FIG. 2C is a view showing the amount of o⁸G by the optimized IP process.
FIG. 2D is a view confirming the G>T mutation in cDNA of oxidized miRNA indirectly (top of FIG. 2D) by sequence-specific cleavage of restriction enzyme sites and directly (middle and bottom of FIG. 2D) by sequencing.
FIG. 2E is a view showing the results of o⁸G-miSeq of oxidized miRNA in H9c2 cells.
FIG. 2F is a view showing the results of Volcano plot analysis in H9c2 cells.
FIG. 2G is a view showing the results of o⁸G-miSeq of oxidized miRNA in rCMC cells.
FIGS. 2H and 2i are views showing the results of o⁸G-miSeq analysis after exposing rCMC and H9c2 cells to serum deficiency.
FIG. 2J shows the results of o⁸G-miSeq of miRNA oxidized in H₂O₂-treated H9c2 cells by comparing them with Wang JX, 2015 (Wang, J. X. et al. Oxidative Modification of miR-184 Enables It to Target Bcl-xL and Bcl-w, Mol Cell 59, 50-61).
FIG. 3A is a view showing the relative amount of o⁸G of miRNA oxidatively induced by PE treatment in rCMC cells.
FIG. 3B is a view showing the relative amount of oxidatively induced miR-1 and the amount of miR-1 in o⁸G IP by ISO treatment in mouse heart.
FIGS. 3C and 3D are views confirming the target silencing effect through the o⁸G:A base arrangement of oxidized miR-1.
FIG. 3E is a view showing the analysis result of the luciferase reporter having a miR-1 oxo site in PE or H₂O₂ treated AC16.
FIG. 3F is a view showing the results of analyzing a double fluorescent protein (dFP) reporter having a miR-1 7oxo site in GFP using flow cytometry in H9c2 cells.
FIG. 3G is a view showing the results of analyzing a double fluorescent protein (dFP) reporter having a miR-1 seed site in GFP using flow cytometry in H9c2 cells.
FIG. 3H is a view showing miR-1 expression in AC16, H9c2, rCMC, and mouse heart.
FIG. 3I is a view showing the results of analyzing a double fluorescent protein (dFP) reporter having miR-1 7oxo, 3oxo, and 2oxo sites in GFP using flow cytometry in H9c2 cells.
FIG. 3J is a view showing the distribution of the size (log₁₀(FSC)) and GFP values (log₁₀(GFP)) of GFP including the miR-1 7oxo site in H9c2 cells.
FIG. 3K is a view showing the results of dFP reporter quantification using flow cytometry in H9c2 cells.
FIG. 3L is a view confirming the activity of miR-1:7o⁸G (RFP:GFP-7oxo vs. RFP:GFP, NT) using the dFP reporter assay in H9c2 cells.
FIG. 3M is a view showing the results of the dFP reporter analysis according to the NAC treatment in H9c2 cells.
FIG. 3N is a view showing the results of the dFP reporter analysis having a miR-1 7oxo site in RFP using flow cytometry in H9c2 cells.
FIG. 3O is a view showing the results of the dFP reporter analysis when considering only the limited cell population with the lowest reporter value (RFP) of 25 % in H9c2 cells.
FIG. 3P is a view showing the results of luciferase reporter analysis having a miR-1 seed site in the presence of 2o⁸G, 3o⁸G, 7o⁸G, and miR-1 in H9c2 cells.
FIG. 4A is a view confirming the effect of miR-1 expression in PE- or NAC-treated rCMC cells.
FIG. 4B is a view confirming the cell size in rCMC cells transfected with miR-1:o⁸G (miR-1:2o⁸G, miR-1:3o⁸G, miR-1:7o⁸G) or miR-1:2U, miR-1:3U, miR-1:7U according to an embodiment of the present invention.
FIG. 4C is a view (scale bar, 50 µm) showing the results of microscopic observation of H9c2 transfected with oxidized miR-1 (miR-1:2o⁸G, miR-1:3o⁸G, miR-1:7o⁸G) or miR-1:U (miR-1:2U, miR-1:3U, miR-1:7U).
FIG. 4D is a view confirming the increase in the expression of the cardiac hypertrophy marker ANP by PE and NAC treatment according to an embodiment of the present invention.
FIG. 4E is a view showing the size distribution of H9c2 cells transfected with miR-1:7U.
FIG. 4F is a view showing time-lapse images of rCMC cells (top of FIG. 4F) and H9c2 cells (bottom of FIG. 4F) transfected with miR-1:7o⁸G or miR-1:7U according to an embodiment of the present invention.
FIG. 4G shows the effect on cardiac hypertrophy in vivo (top of FIG. 4H) and results of checking the delivery to cardiac tissue through qPCR quantification (bottom of FIG. 4H) when miR-1:7o⁸G according to an embodiment of the present invention was injected into mice via the tail vein.
FIG. 4H is a view showing a mouse heart through in vivo delivery of miR-1:7o⁸G according to an embodiment of the present invention.
FIG. 4I is a view confirming the results of immunostaining of the interventricular septum (IS) and expression of cardiomyocytes and ANPs when miR-1:7o⁸G according to an embodiment of the present invention is injected into mice.
FIG. 5A is a view showing that the results of performing a luciferase reporter on a target site, which can be recognized when o⁸G modification of miR-122 occurs at the 2nd and 3rd bases in Huh7, a liver cancer cell, are compared with cells in which miR-122 is removed from Huh7 (Huh7: miR-122 KO).
FIG. 5B is a view showing the result of confirming the presence of the modified let-7 using a luciferase reporter for the 4oxo site which can be recognized when the 4th base of let-7 was modified with o⁸G in HS683, a glioma cell.
FIG. 5C is a view showing the result of confirming the presence of the modified miR-124 using a luciferase reporter for the 4oxo site that can be recognized when the fourth base of miR-124 is modified with o⁸G, when oxidative stress was applied by removing fetal calf serum from HS683, a glioma cell.
FIG. 5D is a view showing the results of observation showing that when miR-122 is oxidatively modified with o⁸G introduced into Huh7, a liver cancer cell, cell migration is changed and miR-122:2,3o⁸G shows more efficient effects when treated with antioxidants.
FIG. 5E is a view showing the results of confirming the function of apoptosis when let-7a:4o⁸G, in which the fourth base of let-7 is modified with o⁸G, is introduced in HS683, a glioma cell.
FIG. 5F is a view showing the results of confirming the function of apoptosis when mir-124:4o⁸G in which the fourth base of miR-124 is modified with o⁸G is introduced in HS683, a glioma cell.
FIG. 6A is a view showing the results of Ago HITS-CLIP derived from the left ventricle of the heart of a human cardiomyopathy patient.
FIG. 6B is a view confirming the frequency of o⁸G:A binding in the Ago-mRNA cluster.
FIG. 6C is a view showing the base sequences of anti-seed and anti-7oxo (top of FIG. 6C) and the results of luciferase reporter analysis (bottom of FIG. 6C) according to an embodiment of the present invention.
FIG. 6D is a view showing the miR-1 7oxo target inhibitory activity of anti-7oxo (9x) according to an embodiment.
FIG. 6E is a view confirming the cardiac hypertrophy inhibitory effect when anti-7oxo (4x) according to one embodiment is introduced into rCMC.
FIG. 6F is a view confirming the cardiac hypertrophy inhibitory effect that appears when anti-7oxo (4x) RNA or α-MHC 13x according to an embodiment is injected into ISO-treated mice.
FIG. 6G is a view confirming the cardiomyocyte size (top of FIG. 6G) and miR-1:7o⁸G target inhibitory effect (bottom of FIG. 6G) depending on the administration of anti-7oxo (α-MHC 13x) according to an embodiment.
FIG. 6H is a view confirming that the administration of anti-7oxo (4x or 13x) according to an embodiment has an effect on the increase of ROS in ISO-treated mouse heart.
FIG. 6I is a view showing a recombinant vector designed to produce a transformed mouse expressing anti-7oxo (α-MHC 13x) according to an embodiment.
FIG. 6J is a view confirming the expression of anti-7oxo (13x) in mice transformed with the recombinant vector of FIG. 5I.
FIG. 6K is a view confirming the size of the heart in mice transformed with anti-7oxo (13x) according to an embodiment.
FIG. 6L is a view confirming the overall inhibition of miR-1:7o⁸G target in mice transformed with anti-7oxo (13x) according to an embodiment.
FIG. 6M is a view confirming the reduction in the size of cardiomyocytes in the ventricular septum (IS) of mice transformed with anti-7oxo (13x) according to an embodiment.
FIG. 6N shows a schematic view of the site-specific oxidation of miR-1:7o⁸G induced by ROS and its cardiac hypertrophy induction process.
FIG. 7A is a view confirming that cardiac hypertrophy was successfully induced in an animal model of cardiac hypertrophy according to an embodiment for obtaining a plasma sample.
FIG. 7B is a view illustrating a process of conducting an experiment after securing a plasma sample in FIG. 6A.
FIG. 7C is a view showing the results of miRNA-1 measurement in plasma samples of cardiac hypertrophy and its control group.
FIG. 7D is a view confirming that oxidatively modified microRNA-1 (miR-1:o⁸G) is increased in cardiac hypertrophy as measured by o⁸G immunoprecipitation in plasma samples of cardiac hypertrophy and its control group.
FIG. 8A is a view confirming the reduction of PE-induced cardiac hypertrophy in H9c2 cells according to the treatment with an antioxidant (N-acetylcysteine, hereinafter NAC) through immunostaining.
FIG. 8B is a view showing the results of mouse echocardiography by ISO and NAC treatment.
FIG. 8C is a view showing the results of immunofluorescence staining of o⁸G in rCMC and H9c2 cells according to PE and NAC treatment.
FIG. 8D is a view showing the results of suppressing cardiomyocyte hypertrophy by treating H9c2 cells, which are treated with PE or ISO to induce cardiomyocyte hypertrophy, with BHA and Sigma-Aldrich's cell culture antioxidant.
FIG. 8E is a view confirming that when anti-miR-1-7oxo according to an embodiment for inhibiting miR-1:7o⁸G was introduced into H9c2 cells treated with PE to induce cardiomyocyte hypertrophy, in the case of treatment with NAC, an antioxidant, cardiomyocyte hypertrophy was completely suppressed.

### Best Mode for Implementing the Invention

As used herein, the term "nucleotide" includes a nitrogen-containing heterocyclic base, a sugar, and one or more phosphate groups. It is a monomer unit of a nucleic acid sequence.

In RNA, the sugar is ribose, and in DNA, it is deoxyribose which is a sugar that lacks the hydroxyl group present on ribose. The nitrogen-containing heterocyclic base may be a purine or pyrimidine base. The purine base includes adenine (A) and guanine (G), and modified derivatives or analogs thereof. The pyrimidine base includes cytosine (C), thymine (T), and uracil (U), and modified derivatives or analogs thereof. In the present invention, the sugar of the nucleotide is ribose, and the base includes adenine (A), guanine (G), cytosine (C), or uracil (U).

As used herein, the term "target site" refers to a target base sequence that binds to the 2nd to 8th base sequences based on the 5'-end of the microRNA.

As used herein, the term "seed region" refers to a base sequence between the 1st and 9th positions based on the 5'-end of the microRNA.

As used herein, the type of the "cell" may be a vertebrate, such as mammals including humans (humans, monkeys, mice, rats, hamsters, cattle, etc.), birds (chickens, ostriches, etc.), amphibians (frogs, etc.), and fish, or invertebrates, such as insects (such as silkworms, moths, fruit flies, etc.), plants, microorganisms such as yeast, and the like, and desirably mammals including humans, but are not limited thereto.

In the present invention, "cardiac hypertrophy" refers to a condition in which the myocardial fibers are enlarged, the heart weight is increased, and the ventricular wall is thickened, and it may be an early symptom of cardiac hypertrophy, heart failure, cardiac fibrosis, mitral valve atresia, aortic valve insufficiency, dilated cardiomyopathy, ischemic heart disease, ventricular septal defect, tricuspid valve insufficiency, pulmonary insufficiency, pulmonary hypertension, right ventricular myocardium infarction, cardiomyopathy involving the right ventricle, atrial septal defect, atrial fibrillation, hypertrophic cardiomyopathy, or infiltrative cardiomyopathy, but in addition to the above diseases, if the disease is related to cardiac hypertrophy, the type is not limited.

As used herein, the term "recombinant" refers to a cell in which a cell replicates a heterologous nucleic acid, expresses the nucleic acid, or expresses a peptide, a heterologous peptide, or a protein encoded by the heterologous nucleic acid. The recombinant cells may express genes or gene segments not found in the native form of the cell, either in sense or antisense form. In addition, the recombinant cells may express genes found in cells in a natural state, but the genes are modified and re-introduced into the cells by artificial means.

As used herein, the term "vector" refers to a DNA construct containing a DNA sequence operably linked to a suitable regulatory sequence capable of effecting the expression of DNA in a suitable host. The suitable regulatory sequences may include promoters to effect transcription, optional operator sequences to control transcription, sequences encoding suitable mRNA ribosome binding sites, and sequences that control termination of transcription and translation. The vector of the present invention is not particularly limited as long as it can replicate within a cell, and any vector known in the art may be used, for example, a plasmid, cosmid, phage particle, or viral vector.

In the present invention, the term "recombinant vector" may be used as an expression vector of a target polypeptide capable of expressing the target polypeptide with high efficiency in an appropriate host cell when the coding gene of the target polypeptide to be expressed is operably linked and the recombinant vector may be expressed in a host cell. The host cell may desirably be a eukaryotic cell, and an expression regulatory sequence such as a promoter, terminator, enhancer, etc., a sequence for membrane targeting or secretion, etc. may be appropriately selected according to the type of host cell and may be combined in various ways depending on the purpose.

In the present invention, the term "promoter" refers to a nucleic acid sequence that regulates gene expression as a DNA base sequence site to which transcriptional regulators bind, and is intended to induce overexpression of a target gene. For example, the recombinant vector of the present invention may include a promoter selected from an alpha MHC (αMHC) promoter, a beta MHC (βMHC) promoter, a Nkx2.5 promoter, a cardiac troponin T (TNNT2) promoter, a muscle creatine kinase (MCK) promoter, a human α-skeletal actin (HAS) promoter, and a murine stem cell virus (MSCV) promoter which is operably linked to regulate cardiomyocyte-specific expression. According to an embodiment of the present invention, the alpha MHC (αMHC) promoter may be operably linked, but the type of the promoter is not limited.

In the present invention, the term "cardiomyocyte-specific" refers to a characteristic that is not expressed or expressed to a low degree in cells other than cardiomyocytes, but is expressed to a very high degree in cardiomyocytes.

As used herein, the term "expression" refers to a process by which a polypeptide is produced from a structural gene, and the process includes transcription of the gene into mRNA and translation of the mRNA into a polypeptide(s).

In the present invention, the term "transformation" refers to a change in the genetic properties of an organism by directly introducing vector-form DNA into cells physiochemically, or by infecting a host cell with a virus to express a foreign gene, that is, transferring the gene into the host cell, and that is, it means introduction of a gene into a host cell so that it may be expressed in the host cell.

In the present invention, "antioxidant" is a substance that prevents excessive production of active oxygen, and serves to prevent oxidative stress in cells and tissues. The antioxidant neutralizes free radicals that cause harmful reactions in cells before they attack DNA or oxidize lipids. In addition, the antioxidant has high reactivity and reacts with harmful substances in the body to prevent chain reactions caused by active oxygen, thereby protecting cells.

In the present invention, "prevention" refers to any action that suppresses or delays the onset of a targeted pathophysiological phenomenon by administration of the composition according to the present invention.

In the present invention, "treatment" refers to any action in which the symptoms of a target disease are improved or beneficially changed by administration of the composition according to the present invention.

MicroRNA (miRNA) is a small RNA composed of about 21 bases, and functions to induce RNA interference that suppresses gene expression in the post-transcriptional stage of the target gene. The microRNA recognizes hundreds of target mRNAs through the base sequence of the seed region (positions 1-8) located mainly at the 5'-end, followed by base arrangement, and suppressing their expression to regulate biological functions. Therefore, when the modification of guanine (G) to 8-oxoguanine (o⁸G) occurs at the seed region of the microRNA, through the o⁸G:A base arrangement enabled due to this, a new target may be suppressed due to base arrangement with other mRNAs, thereby regulating other pathophysiological functions.

In other words, the oxidative modification occurring in the seed region may be a mechanism to regulate the recognition of a target by microRNA that occurs naturally or pathologically, and based on this, it is possible to develop a technology that can artificially induce or inhibit pathophysiological functions. It has been previously known that 8-oxoguanine modification in RNA mainly occurs in a group of diseases (degenerative disease) related to the aging process of the human body (degenerative disease), and heart disease is also deeply related to this. Therefore, a nucleic acid body based on 8-oxoguanine modification has a potential to regulate various diseases.

The present invention provides an RNA interference-inducing nucleic acid including at least one 8-oxoguanine (o⁸G) in the 1st to 9th nucleotides from the 5'-end of at least one single strand of double strands thereof.

In one single strand of the double strand of the nucleic acid, the 1st to 9th nucleotides from the 5'-end may include at least one 8-oxoguanine, and both of the double strands of the nucleic acid, i.e., both single strands may include at least one 8-oxoguanines within the 1st to 9th nucleotides from the 5'-ends thereof.

The RNA interference-inducing nucleic acid may be selected from a microRNA, a small interfering RNA (siRNA), a short hairpin RNA (shRNA), DsiRNA, IsiRNA, ss-siRNA, piRNA, endo-siRNA, or asiRNA, but is not limited to the above type as long as it is a nucleic acid that induces RNA interference.

For example, the 1st to 9th nucleotides from the 5'-end may include a sequence of microRNA.

For example, the microRNA may be any of microRNAs of Group 1:
[Group 1]
miR-1, miR-184, let-7f-5p, miR-1-3p, miR-122, let-7, and miR-124.

For example, the micro RNA may be miR-1, miR-122, let-7, or miR-124.

For example, the 8-oxoguanine (o⁸G) may be present at a position corresponding to the 2nd, 3rd, 4th, 7th, or a combination thereof from the 5'-end of the microRNA.

For example, the RNA interference-inducing nucleic acid may include at least one single strand of double strand thereof that includes the 1st to 9th nucleotides from the 5'-end of miR-1, and among them, at least one guanine (G) may be modified to 8-oxoguanine (o⁸G), and for example, the 8-oxoguanine (o⁸G) may be present at a position corresponding to the 2nd, 3rd, or 7th position, or a combination thereof, from the 5'-end of miR-1.

For example, the RNA interference-inducing nucleic acid may include at least one single strand of double strands thereof that includes the 1st to 9th nucleotides from the 5'-end of miR-122, and among them, at least one guanine (G) may be modified to 8-oxoguanine (o⁸G), and for example, the 8-oxoguanine (o⁸G) may be present at a position corresponding to the 2^{nd} or 3rd position, or a combination thereof, from the 5'-end of miR-122.

For example, the RNA interference-inducing nucleic acid may include at least one single strand of double strands thereof that includes the 1st to 9th nucleotides from the 5'-end of let-7 or miR-124, and among them, at least one guanine (G) may be modified to 8-oxoguanine (o⁸G), and for example, the 8-oxoguanine (o⁸G) may be present at a position corresponding to the 4th position from the 5'-end of let-7 or miR-124.

In the RNA interference-inducing nucleic acid, an o⁸G:A arrangement occurs at the position of 8-oxoguanine (o⁸G) to recognize a target site.

For example, the RNA interference inducing nucleic acid may include any one of the following polynucleotides of Group 2:
[Group 2]
a polynucleotide of SEQ ID NO: 1 (5'p-Uo⁸GGAAUGUAAAGAAGUAUGUAU-3');
a polynucleotide of SEQ ID NO: 2 (5'p-UGo⁸GAAUGUAAAGAAGUAUGUAU-3');
a polynucleotide of SEQ ID NO: 3 (5'p-UGGAAUo⁸GUAAAGAAGUAUGUAU-3');
a polynucleotide of SEQ ID NO: 65 (5'p-Uo⁸Go⁸GAGUGUGACAAUGGUGUUUG-3');
a polynucleotide of SEQ ID NO: 66 (5'p-UGAo⁸GUAGUAGGUUGUAUAGdTdT-3'); and
a polynucleotide of SEQ ID NO: 67 (5'p-UAAo⁸GGCACGCGGUGAAUGCdTdT-3').

When the aforementioned RNA interference-inducing nucleic acid is injected into a cell or an animal, various pathophysiological phenomena may be controlled, and for example, myocardial hypertrophy, inhibition of migration of liver cancer cells, or apoptosis may be induced.

For example, the RNA interference-inducing nucleic acid including the 1st to 9th nucleotides from the 5'-end of miR-1, wherein at least one guanine (G) modified to 8-oxoguanine (o⁸G) may induce myocardial hypertrophy when injected into a cell or an animal.

For example, when the RNA interference-inducing nucleic acid including the 1st to 9th nucleotides from the 5'-end of miR-122 and wherein at least one guanine (G) is modified to 8-oxoguanine (o⁸G) is injected into liver cancer cells, inhibition of the migration of liver cancer cells may be induced.

For example, when the RNA interference-inducing nucleic acid including the 1st to 9th nucleotides from the 5'-end of let-7 or miR-124, wherein at least one guanine (G) is modified to 8-oxoguanine (o⁸G) is injected into glioma cells, apoptosis may be induced.

The present invention provides a composition including the aforementioned RNA interference-inducing nucleic acid and an antioxidant.

The antioxidant may prevent further oxidation of the RNA interference-inducing nucleic acid, that is, the oxidation of additional guanine (G) to 8-oxoguanine (o⁸G), so that the RNA interference-inducing nucleic acid may more effectively control pathophysiological phenomena. As the antioxidant, all antioxidants commonly used in the art may be used.

In addition, the present invention provides a method for identifying a position of 8-oxoguanine (o⁸G) including:
(a) extracting RNA from the cell;
(b) isolating RNA that includes 8-oxoguanine (o⁸G) from the extracted RNA by immunoprecipitation (IP) using an anti-o⁸G antibody;
(c) producing cDNA by reverse transcription of the isolated RNA that includes 8-oxoguanine (o⁸G) to produce and sequence a sequencing library for determining the position of 8-oxoguanine (o⁸G); and
(d) identifying the position at which guanine (G) is substituted with thymine (T) as the position where guanine (G) is modified to 8-oxoguanine (o⁸G).

The method for identifying the position of the 8-oxoguanine (o⁸G) may identify the position where guanine (G) is modified to 8-oxoguanine (o⁸G) more accurately than the existing method for identifying the position by reverse transcription of RNA to prepare cDNA and sequencing it.

In addition, the present invention provides a modified nucleic acid that specifically binds to a microRNA in which at least one guanine (G) of the 1st to 9th nucleotides from the 5'-end is modified to 8-oxoguanine (o⁸G).

The modified nucleic acid may include polynucleotide complementary to 6 or more, for example, 6, 7, or 8 consecutive polynucleotides starting from the 2nd or 3rd nucleotide from the 5'-end of the modified microRNA, and
the modified nucleic acid may include adenine (A) that binds to at least one 8-oxoguanine (o⁸G) among the 1st to 9th nucleotides from the 5'-end of the modified microRNA.

For example, the modified nucleic acid may specifically bind to a microRNA in which at least one guanine (G) of the 2nd, 3rd, or 7th nucleotide from the 5'-end is modified to 8-oxoguanine (o⁸G).

The modified nucleic acid may include a polynucleotide complementary to 6 or more consecutive polynucleotides starting from the 2nd or 3rd nucleotide from the 5'-end of the modified microRNA, and
the modified nucleic acid may include adenine (A) that binds to at least one 8-oxoguanine (o⁸G) among the 2nd, 3rd, or 7th nucleotide from the 5'-end of the modified microRNA.

For example, the modified nucleic acid may include at least one base sequence among 5'-ACAUUCA-3', 5'-ACAUUAC-3', and 5'-AAAUUCC-3', but is not limited thereto.

In addition, the present invention provides a recombinant vector including a gene encoding the aforementioned modified nucleic acid.

The recombinant vector may include DNA, and the DNA may include a polynucleotide complementary to the polynucleotide of the modified nucleic acid for inhibiting cardiac hypertrophy of the present invention. For example, when the base of the modified nucleic acid for inhibiting cardiac hypertrophy is adenine (A), it may include the thymine (T) base, when the base of the modified nucleic acid for inhibiting cardiac hypertrophy is guanine (G), it may include the cytosine (C) base, when the base of the modified nucleic acid for inhibiting cardiac hypertrophy is cytosine (C), it may include a guanine (G) base, and when the base of the modified nucleic acid for inhibiting cardiac hypertrophy is uracil (U), it may include an adenine (A) base.

In addition, the present invention provides a pharmaceutical composition for treating cardiac hypertrophy including the aforementioned modified nucleic acid or the recombinant vector.

For example, the pharmaceutical composition for treating cardiac hypertrophy may include a modified nucleic acid that specifically binds to microRNA and in which at least one guanines (G) from among the 1st to 9th nucleotides from the 5'-end are modified to 8-oxoguanine (o⁸G).

The modified microRNA to which the modified nucleic acid specifically binds may be one in which any one of miR-1, miR-184, let-7f-5p, or miR-1-3p is modified.

The modified microRNA may be, for example, a modified miR-1, in which, for example, a guanine (G) at the position corresponding to the 2nd, 3rd, 7th position, or a combination thereof from the 5'-end of miR-1, is modified.

For example, the pharmaceutical composition for treating cardiac hypertrophy may further include an antioxidant. As the antioxidant, all antioxidants commonly used in the field may be used, for example, NAC, BAH, or an antioxidant for cell culture (A1345; Sigma-Aldrich) may be further used, but is not limited thereto.

In addition, the present invention may provide a pharmaceutical composition for treating liver cancer or glioblastoma including the aforementioned RNA interference-inducing nucleic acid.

For example, the pharmaceutical composition for treating liver cancer may include an RNA interference-inducing nucleic acid in which at least one single strand of the double strands thereof includes 1st to 9th nucleotides from the 5'-end of miR-122, among which at least one guanine (G) is modified to 8-oxo guanine (o⁸G).

For example, the RNA interference-inducing nucleic acid may include a single strand including a base sequence in which the guanine (G) at the 2nd position, the 3rd position, or a combination thereof from the 5'-end of the 1st to 9th nucleotides from the 5'-end of miR-122, is modified to 8-oxoguanine (o⁸G).

For example, the pharmaceutical composition for treating glioblastoma may include an RNA interference inducing nucleic acid that includes at least one single strand of double strands thereof that includes the 1st to 9th nucleotides from the 5'-end of let-7 or miR-124, in which at least one guanine (G) is modified to 8-oxoguanine (o⁸G).

For example, the RNA interference-inducing nucleic acid may include a single strand including the base sequence in which guanine (G) positioned at the 4th position from the 5'-end of the 1st to 9th nucleotides from the 5'-end of let-7 or miR-124 is modified to 8-oxoguanine (o⁸G).

For example, the pharmaceutical composition for treating liver cancer may further include an antioxidant in addition to the RNA interference-inducing nucleic acid, and the pharmaceutical composition for treating glioblastoma may further include an antioxidant in addition to the RNA-interference-inducing nucleic acid. The antioxidant means a generally used antioxidant, for example, NAC and BAH, or an antioxidant for cell culture (A1345; Sigma-Aldrich) may be used, but is not limited thereto.

The pharmaceutical composition for treating liver cancer or glioblastoma further includes an antioxidant, thereby preventing the further oxidation of other guanine (G) in the RNA interference-inducing nucleic acid by active oxygen in the cell and thus maximizing its function.

The pharmaceutical composition according to the present invention may be formulated in various forms according to conventional methods. For example, it can be formulated in oral dosage forms such as powders, granules, tablets, capsules, suspensions, emulsions, syrups, creams, gels, patches, sprays, ointments, warnings, lotions, liniments, pastas, or it can be formulated and used in the form of external preparations for skin such as cataplasma, suppositories, and sterile injection solutions.

The pharmaceutical composition according to the present invention may further include suitable carriers, excipients, and diluents commonly used in the preparation of pharmaceutical compositions. In this case, carriers, excipients, and diluents included in the composition may include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propylhydroxybenzoate, talc, magnesium stearate, and mineral oil. In the case of formulation, it is prepared using commonly used diluents or excipients such as fillers, extenders, binders, wetting agents, disintegrants, and surfactants. Solid formulations for oral administration may include tablets, pills, powders, granules, capsules, etc., and these solid preparations may include at least one excipient in the extract, for example, starch, calcium carbonate, sucrose or lactose, gelatin, etc. In addition to simple excipients, lubricants such as magnesium stearate talc are also used. Liquid formulations for oral use may include suspensions, internal solutions, emulsions, syrups, etc. in addition to water and liquid paraffin, which are commonly used simple diluents, various excipients, for example, wetting agents, sweeteners, fragrances, preservatives, etc. Formulations for parenteral administration may include sterile aqueous solutions, non-aqueous solutions, suspensions, emulsions, freeze-dried formulations, and suppositories. Non-aqueous solvents and suspending agents may include propylene glycol, polyethylene glycol, vegetable oils such as olive oil, and injectable esters such as ethyl oleate. As the base of the suppositories, Witepsol, macrogol, tween 61, cacao butter, laurin butter, glycerogelatin, and the like may be used.

The pharmaceutical composition of the present invention may be administered orally or parenterally (e.g., intravenously, subcutaneously, intraperitoneally, or locally applied) according to a desired method, and the dosage may vary depending on the patient's condition and weight, and the degree of disease, depending on the drug form, and administration route and time, but may be appropriately selected by those skilled in the art.

The pharmaceutical composition of the present invention is administered in a pharmaceutically effective amount. In the present invention, "pharmaceutically effective amount" means an amount sufficient to treat a disease at a reasonable benefit/risk ratio applicable to medical treatment, and the effective dose level is determined according to factors including the type, severity, drug activity, and type of the patient's disease, sensitivity to the drug, administration time, administration route and excretion rate, treatment period, concurrent drugs and other factors well known in the medical field. The pharmaceutical composition according to the present invention may be administered as an individual therapeutic agent or may be administered in combination with other therapeutic agents, may be administered sequentially or simultaneously with conventional therapeutic agents, and may be administered singularly or multiply. Taking all of the above factors into consideration, it is important to administer an amount capable of obtaining the maximum effect with a minimum amount without side effects, which can be easily determined by those skilled in the art. Administration may be performed once a day, or may be performed in several divided doses.

In addition, the present invention provides a pharmaceutical composition for preventing or treating cardiac hypertrophy including an antioxidant as an active ingredient, wherein the antioxidant inhibits oxidative modification of at least one guanine (G) among the 1st to 9th nucleotides based on the 5'-end of the microRNA, into an 8-oxoguanine (o⁸G).

In the present invention, the antioxidant serves to inhibit the oxidative modification of the guanine (G) to 8-oxoguanine (o^{B}G), the antioxidant may be for example, at least one selected from N-acetylcysteine (NAC), butylated hydroxyanisole (BHA), ascorbic acid (Vitamin C), glutathione, urate, bilirubin, vitamin E, carotenoids, ubiquinone (ubizuinol, Co-Q10), flavonoids, antioxidant enzymes, SOD (superoxide dismutase), catalase, glutathione peroxidase (GSH-PX), glutathione reductase, glutathione transferase, SOD mimetics (CuDIOS), ebselen, lazaroids (21-aminosteroids), captodative olefins, α-lipoic acid and dihydrolipoic acid (DHLA), 5-HTP (hydroxytryptophan), DHEA (dehydroepiandrosterone), amino acids, herbal antioxidants, and minerals, and according to an embodiment of the present invention, the antioxidant may be N-acetylcysteine (NAC) or butylated hydroxyanisole (BHA), but the present invention is not limited to the type of the antioxidant.

For example, the microRNA may be miR-1, miR-184, let-7f-5p, or miR-1-3p.

In addition, the present invention provides an information provision method for diagnosing cardiac hypertrophy that includes:
determining whether a guanine (G) among nucleotides of microRNA isolated from a cardiomyocyte of an animal is modified to 8-oxoguanine (o⁸G); and
classifying as cardiac hypertrophy when a guanine (G) of the nucleotides of the microRNA is modified to 8-oxoguanine (o⁸G).

In the present invention, "diagnosing" means confirming the presence or characteristics of a pathological condition. For the purposes of the present invention, diagnosing is to determine whether cardiac hypertrophy has developed.

For example, the nucleotide may be the 1st to 9th nucleotides from the 5'-end of the microRNA.

For example, the nucleotide may be the 2nd, 3rd, or 7th nucleotide from the 5'-end of the microRNA.

For example, the microRNA may be miR-1, miR-184, let-7f-5p, or miR-1-3p.
in addition, the present invention provides a method for producing a non-human animal model resistant to cardiac hypertrophy and a non-human animal model resistant to cardiac hypertrophy produced by the method, wherein the method includes:
(a) operably linking a gene encoding a modified nucleic acid for the inhibition of cardiac hypertrophy to a promoter to construct a recombinant vector;
(b) introducing the recombinant vector into a fertilized egg of an animal; and
(c) generating the fertilized egg after transplanting it into a surrogate mother to obtain a transgenic animal model.

For example, the microRNA may be miR-1, miR-184, let-7f-5p, or miR-1-3p, for example, miR-1.

For example, the animal may be a non-human primate, a mouse, a dog, a cat, a rabbit, a horse, or a cow.

According to an embodiment of the present invention, the animal may be a mouse, but if it is a mammal other than a human, the type is not particularly limited.

In the present invention, the method of introducing vector-form DNA into cells is not particularly limited, and may be, for example, performed by nucleofection, transient transfection, cell fusion, liposome-mediated transfection, polybrene-mediated transfection, transfection with calcium phosphate (Graham, FL et al., Virology, 52:456 (1973)), transfection with DEAE dextran, transfection by microinjection (Capecchi, MR, Cell, 22:479 (1980)), transfection with cationic lipids (Wong, TK et al., Gene, 10:87 (1980)), electroporation (Neumann E et al., EMBO) J, 1:841 (1982)), transduction, or transfection, or methods well known to those of ordinary skill in the art as described in Basic Methods in Molecular Biology, Davis et al., 1986 and Molecular Cloning: A Laboratory Manual, Davis et al., (1986).

In addition, the present invention provides a method for screening a candidate substance for the treatment of cardiac hypertrophy, which includes:
(a) treating a cardiomyocyte of an animal model of cardiac hypertrophy with a candidate substance;
(b) analyzing frequency of modification of guanine (G) to 8-oxoguanine (o⁸G) among nucleotides of microRNA expressed in the cardiomyocyte of the animal model of cardiac hypertrophy; and
(c) selecting the candidate substance as a cardiac hypertrophy therapeutic agent, when the frequency of modification of guanine (G) to 8-oxoguanine (o⁸G) decreases compared to the case in which the candidate substance is not treated.

In the present invention, the candidate substance refers to an unknown substance used for screening to examine whether or not it affects the binding frequency of the polynucleotide with the o⁸G:A arrangement target site in cardiomyocytes of an animal model of cardiac hypertrophy, and may include chemicals, proteins, (poly)nucleotides, antisense-RNA, siRNA (small interference RNA), or natural product extracts, etc., but is not limited thereto.

In the present invention, the candidate substance may be an antioxidant, but is not limited thereto.

In addition, the present invention provides a method for inhibiting cardiac hypertrophy that includes administering the modified nucleic acid to an individual.

In addition, the present invention provides a method for inhibiting liver cancer metastasis or treating glioblastoma that includes administering the RNA interference-inducing nucleic acid to an individual.

In addition, the present invention provides a method for preventing or treating cardiac hypertrophy that includes administering a composition including an antioxidant as an active ingredient to an individual.

In the present invention, "individual" refers to a subject requiring administration of a composition including a modified nucleic acid for inhibition of cardiac hypertrophy or an RNA interference-inducing nucleic acid or an antioxidant for treatment of liver cancer or glioblastoma, and more specifically refers to mammals such as human or non-human primates, mice, dogs, cats, horses, and cattle.

In the present invention, "administration" refers to a composition including a modified nucleic acid for inhibiting cardiac hypertrophy or an RNA interference-inducing nucleic acid for the treatment of liver cancer or glioblastoma or an antioxidant of the present invention to an individual by any suitable method.

In addition, the present invention provides a use of the modified nucleic acid for inhibiting cardiac hypertrophy.

In addition, the present invention provides a use of the RAN interference-inducing nucleic acid for inhibiting liver cancer metastasis and treating liver cancer or glioblastoma.

In addition, the present invention provides a use for preventing or treating cardiac hypertrophy of a composition including an antioxidant as an active ingredient.

### Mode for Invention

Hereinafter, preferred examples are presented to help the understanding of the present invention. However, the following examples are only provided for easier understanding of the present invention, and the contents of the present invention are not limited by the following examples.

### Example 1. Cell Culture, Drug Treatment, and Transfection

### 1. Cell Culture

Rat cardiomyocyte line H9c2 (H9c2(2-1), ATCC CRL-1446; H9c2, Korea Cell Line Bank), human ventricular cardiomyocyte line AC16 (provided by J. Han, H.W. Lee, and W.J. Park), and human cervical adenocarcinoma cell line HeLa (ATCC CCL-2) were grown in Dulbecco's modified Eagle's medium (DMEM; Hyclone) supplemented with 10 % fetal bovine serum (FBS; Gibco), 100 U/ml penicillin, and 100 µg/ml streptomycin (Welgene) at 37 °C and 5 % CO₂ culture conditions.

For consistency of results, several different batches of H9c2 maintained at two different facilities (ATCC and Korea Cell Line Bank) were used, and AC16 was obtained from each of different batches derived from Dr. M.M. Davidson (scc109, Millipore).

Primary cultures of rat neonatal cardiomyocytes (rCMC) were prepared using the Neomyt kit (nc-6031; Cellutron) according to the manufacturer's protocol, as described in Seok, H. Y. et al. Circ Res 114, 1585-1595, and Huang, Z. P. et al. Circ Res 112, 1234-1243.

That is, ventricular tissue derived from Sprague Dawley rat heart was collected on postnatal day 1 (P1), washed and cut into 3 to 4 mm pieces, and stirred at 37 °C in a 50 ml flask, and cells derived from ventricular slices were completely isolated by repeated enzymatic reactions (solution provided by the Neomyt kit). Undigested tissue was removed from the obtained supernatant using a strainer, and then plated for 2 hours to remove easily attached non-cardiomyocytes. Finally, non-adherent rCMC was harvested and treated with 10 % FBS (Gibco), 5 % horse serum (HS; Welgene), 2 % L-glutamine (Welgene), 0.1 mM 5-bromo-2'-deoxyuridine (Sigma-Aldrich), 100 U ml⁻¹ penicillin, and a 4:1 mixture of DMEM:M199 (Welgene) supplemented with 100 µg ml⁻¹ streptomycin (Welgene) in SureCoat (sc-9035; Cellutron) culture plates.

### 2. Drug Treatment

To induce adrenergic hypertrophy, unless otherwise indicated, rCMC, H9c2, or AC16 were usually treated with 200 µM phenylephrine (PE) or 10 µM isoproterenol (ISO) and tested 48 hours after treatment. Since serum deficiency is also used as a pretreatment condition for myocardial hypertrophy, the same medium without FBS and HS was replaced 24 hours before treatment. For treatment with antioxidants, 2 mM N-acetylcysteine (NAC) was applied during treatment with PE or ISO.

### 3. Transfection

In general, transfection or cotransfection of vectors with double miRNA into H9c2, AC16, rCMC, or HeLa cells was performed using Lipofectamine 2000 or 3000 (Invitrogen), whereas transfection of miRNA or miRNA inhibitors (50 nM) was performed using RNAiMAX (Invitrogen) or Lipofectamine 3000 (Invitrogen) according to the general protocol provided by the manufacturer. To achieve maximum efficiency, the correct number of cells was counted using a Countess II Automated Cell Counter (Invitrogen) and aliquoted into plates prior to transfection.

### Example 2. ROS and Cell Size Measurement

For quantitative measurement of cellular ROS levels, flow cytometry was based on ROS fluorescent dye and dihydroethidium (DHE) was applied using Muse Cell Analyzer (Milipore) with Muse Oxidative Stress Kit (Milipore). That is, the day before drug treatment, H9c2 or AC16 cells were aliquoted at a density of 10⁶ cells/6 well-plate. From 10 minutes to 48 hours after treatment, cells were harvested, fixed, stained, and measured after repeating according to the manufacturer's protocol (n=3). For analysis, equal numbers of cells (n=10,000) were quantified for both ROS level and cell size. Forward-Scattered Light (FSC) was measured as cell size according to the manufacturer's protocol (log₁₀(FSC), y-axis; hypertrophy, cell size>3).

To determine the amount of ROS in cardiac tissue lysates, CM-H2DCFDA (Invitrogen) was used. Homogenized lysates prepared in 1x RIPA lysis buffer (DyneBio) supplemented with protease inhibitors (complete, Mini, EDTA-free; Roche) and 2.5 mM deferoxamine mesylate (DFOM; Sigma-Aldrich) were quantified regarding proteins using the Qubit Protein Assay Kit (Invitrogen). 100 µg of the lysates were incubated with 2 µM CM-H2DCFDA for 30 min at 37 °C in the dark. Fluorescence signals were detected by a Qubit 2.0 Fluorometer (Invitrogen).

To visualize cellular ROS levels, DHE (Invitrogen) was used. Initially, H9c2 cells were serum-starved for 24 h and treated with 100 µM PE until indicated time points. Then, the harvested cells were washed with serum-free DMEM and incubated with 10 µM DHE at 37 °C for 10 minutes while avoiding light, and after incubation, the cells were washed again with serum-free DMEM. ROS images were obtained using an inverted fluorescence microscope (Leica DMi8).

### Example 3. Immunofluorescence Staining

H9c2 or rCMC were fixed with 4 % paraformaldehyde (PFA; Biosesang) for 15 min at room temperature. After washing twice with PBS (Biosesang) containing 0.1 % NP-40 (Sigma-Aldrich), the sample was incubated for 1 hour at room temperature with the addition of 5 % bovine serum albumin (BSA; Bovogen). Then, it was washed twice, and the primary antibody was incubated under the following conditions; overnight at 4 °C in PBS with 0.1 % NP-40 and 3 % BSA; MF20 (1:1000, Developmental hybridoma), o⁸G (15A3, 1:1000, QED Bioscience), and Ago2 (ab5072, 1:200, Abcam). DAPI (1.5 µg/ml, Vector lab) was used for nuclear staining. Because of the different degrees of fate heterogeneity in H9c2 cardiomyocytes (although all H9c2 cells are in the same lineage as cardiomyocytes), MF20 was used to stain cardiomyocytes. For RNase treatment, 10 µg/ml of RNase A (Invitrogen) was used at room temperature for 15 minutes before incubating the primary antibody, and Alexa Fluor 488 donkey anti-mouse IgG (1:1000, Abeam) and Alexa Fluor 594 donkey anti-rabbit IgG (1:1000, Abeam) were used as a secondary antibody in PBS containing 0.1 % NP-40 at room temperature for 1 hour. Stained cells were examined with an inverted fluorescence microscope (Leica DMi8), analyzed with Leica Application Suite (LAS), and quantified with Imaged (≥100 cells, https://imagej.nih.gov/).

### Example 4. Cell Size Measurement

Cell sizes from cultures or tissue sections were quantified using the "measuring" function in ImageJ 1.51s software (https://imagej.nih.gov/). All images were converted to a size of 2560 x 1962 pixels, equivalent to 35.56 x 26.67 inch², for consistent comparison across multiple images. Then, the cell area in the converted image was measured in units of inch² (71.9 pixel/inch) and used for the next analysis.

### Example 5. RNA Synthesis and Modification

Custom synthesis and modification services from TriLink Biotechnologies (USA), Integrated DNA Technologies (USA), ST Pharm (Korea), and Bioneer (Korea) were used to produce RNA with various modifications, and their quality was monitored and confirmed.

8-oxoguanine (o⁸G) in the ribonucleotide was introduced at the indicated position of miR-1 (5'p-UGGAAUGUAAAGAAGUAUGUAU-3'; SEQ ID NO: 7); miR-1:7o⁸G, 5'p-UGGAAUo⁸GUAAAGAAGUAUGUAU-3' (SEQ ID NO: 3); miR-1:2o⁸G, 5'p-Uo⁸GGAAUGUAAAGAAGUAUGUAU-3' (SEQ ID NO: 1); and miR-1:3o⁸G, 5'p-UGo⁸GAAUGUAAAGAAGUAUGUAU-3' (SEQ ID NO: 2).

The duplex of miR-1 containing the oxidized derivative was produced in vitro under the following conditions by being annealed to the synthesized miR-1 passenger strand (5'p-ACAUACUUCUUUAUAUGCCCAUA-3' (SEQ ID NO: 8), when transfer is confirmed, fluorescein isothiocyanate (FITC) is attached to 5'-end); 90 °C for 2 minutes, 30 °C for 1 hour, and 4 °C for 5 minutes.

In addition, 8-oxoguanine (o⁸G) in the ribonucleotides was introduced at the indicated position of miR-122 (5'p-UGGAGUGUGACAAUGGUGUUUG-3'; SEQ ID NO: 68), let-7 (5'-pUGAGUAGUAGGUUGUAUAGdTdT-3'; SEQ ID NO: 69) and miR-124 (5'-p UAAGGCACGCGGUGAAUGCdTdT-3'; SEQ ID NO: 70) as follows: miR-122:2,3o⁸G, 5'p-Uo⁸Go⁸GAGUGUGACAAUGGUGUUUG-3' (SEQ ID NO: 65); let-74o⁸G: 5'p-UGAo⁸GUAGUAGGUUGUAUAGdTdT-3' (SEQ ID NO: 66); miR-124:4o⁸G, and 5'-p UAAo⁸GGCACGCGGUGAAUGCdTdT-3' (SEQ ID NO: 67).

Duplexes of miR-122, let-7, and miR-124 containing oxidized derivatives were prepared in the same method as the duplex of miR-1.
miR-1 containing the substitution of G>U at the indicated positions was also synthesized (miR-1:2GU, 5'p-UUGAAUGUAAAGAAGUAUGUAU-3' (SEQ ID NO: 9); miR-1:3GU, 5'p-UGUAAUGUAAAGAAGUAUGUAU-3' (SEQ ID NO: 10); miR-1:7GU, and 5'p-UGGAAUUUAAAGAAGUAUGUAU-3' (SEQ ID NO: 11)) and was duplexed with the passenger strand of miR-1. As a control miRNA, a non-target miRNA (NT) (5'-UCACAACCUCCUAGAAAGAGUA-3'; SEQ ID NO: 12) derived from cel-miR-67 (C. elegans-specific miRNA provided as negative control by D. Dharmacon) was synthesized as siRNA and was used as a duplex (guide strand: 5'p-UCACAACCUCCUAGAAAGAGUAdTdT-3' (SEQ ID NO: 13), passenger strand: 5'p-UACUCUUUCUAGGAGGUUGUGAdTdT-3' (SEQ ID NO: 14), and 'dT' represents thymidine deoxynucleotide). When excluding seed-mediated off-target effects, NT was further modified to include a dSpacer (abasic deoxynucleotide; Ø) at the 6th position in both the guide and passenger strands or 2'-O-methyl at 1st and 2nd positions as previously reported.

To confirm the o⁸G-induced G>T mutation, 39-oxo-R (o⁸G; 5'-CCUGGUCCCAGACUAAAGAAUo⁸GCUUGACAGUUAUCUCGUAUGCCGUC UUCUCGAGGUAGCGGAACCGUGAGCUUUGAAGU-3'; SEQ ID NO: 15), which potentially generates EcoR1 sites during RT-PCR via o⁸G:A base binding; and 39R (G; 5'-CCUGGUCCCAGACUAAAGAAUGCUUGACAGUUAUCUCGUAUGCCGUCUU CUCGAGGUAGCGGAACCGUGAGCUUUGAAGU-3'; SEQ ID NO: 16) as a control thereof were synthesized. As a spike-in control, o⁸G spike-in (5'p-Uo⁸Go⁸GAAUGUAAAGAAGUAUGUAU-3'; SEQ ID NO: 17), G spike-in (5'p-UGGAAUGUAAAGAAGUAUGUAU-3'; SEQ ID NO: 18), and miRNA:o^{B}G spike-in (5'p-UAAGo⁸GCACGCGGUGAAUGCCAA-3'; SEQ ID NO: 19) were synthesized.

For competitive miR-1 and miR-1:7o⁸G inhibitors, anti-seed (2x: 5'-dTdTØACAUUCCAØACAUUCCAØdTdT-3' (5'-ACAUUCC-3' is a miR-1 seed site; SEQ ID NO: 20)), anti-7oxo (2x: 5'-dTdTØAAAUUCCAØAAAUUCCAØdTdT-3', 4x: 5'-dTAAAUUCC AAAUUCCAGAAAUUCCACAAAUUCCAdT-3', 9x: 5'-dNdNØAAAUUCCAØAAAUUCCAØAAAUUCCAØAAAUUCCAØAAAUUCCAØ AAAUUCCAØAAAUUCCAØAAAUUCCAØAAAUUCCAØdNdN-3'; 'dN' represents deoxynucleotide, A, T, C, or G (5'-AAAUUCC-3' is a miR-1 7oxo site; SEQ ID NO: 6)), cont (anti-NT; 2x: 5'-dTdTØGGUUGUGAØGGUUGUGAØdTdT-3', 4x: 5'-dTGGUUGUG GGUUGUGAGGGUUGUGACGGUUGUGAdT-3', 9x: 5'-dNdNØGGUUGUGAØGGUUGUGAØGGUUGUGAØGGUUGUGAØGGUUGU GAØGGUUGUGAØGGUUGUGAØGGUUGUGAØGGUUGUGAØdNdN-3' (5'-GGUUGUG-3' is cont NT site; SEQ ID NO: 21) were synthesized. At both the 5'- and 3'-ends of the inhibitors, "dT" or "dN" was intentionally attached to contribute to RNA stability. By introducing an abasic deoxynucleotide (Ø) into the junction of the target site in anti-seed (2x) or anti-7oxo (2x) to prevent off-target effects through the putative sequence at the junction, it was confirmed that there was no difference in the phenotype from the inhibitor without the abasic deoxynucleotide. Underlines in the sequence indicate the corresponding target inhibition sites.

### Example 6. RNA Extraction

Small (<-200 nt) and large (>-200nt) RNA fractions were isolated using the miRNeasy Mini Kit (Qiagen) to purify RNA according to size according to the protocol provided by the manufacturer. For cardiac tissue samples, a Minilys Personal Homogenizer (Bertin) or a glass homogenizer was used in the presence of 700 µl Qiazol Lysis Reagent (Qiagen), supplemented with 2.5 mM DFOM (Sigma-Aldrich) to prevent oxidation in vitro as reported previously. For total RNA purification, only Qiazol Lysis Reagent with 2.5 mM DFOM was used after addition of 20 % chloroform (Merck), followed by RNA precipitation with isopropyl alcohol and treatment with RQ1 RNase free DNase (30 min at 37 °C; solution was stopped with heat inactivation, 65 °C for 10 min; Promega). For accurate quantification of RNA, a spectrophotometer (Denovix) and Qubit fluorescence quantitation (Invitrogen) were used.

To purify miRNA, gel extraction of about 20 nt was performed from total RNA. Total RNA denatured in gel loading buffer II (2 min at 90 °C; Ambion) was isolated by 15 % Urea-PAGE gel with the marking of about 20 nt miRNA marker (synthesized miR-1), and the size thereof was cross-confirmed with a 14 to 30 ssRNA Ladder marker (Takara). Then, after staining with SYBR Gold, about 20 nt size was cut from the gel, was incubated in a thermomixer (Thriller, Peqlab) overnight at 65 °C with gel extraction buffer (0.5 M ammonium acetate, 10 mM magnesium acetate, 0.1 % SDS, 1 mM EDTA, pH 8.0), and was further purified with an Oligo Clean & Concentrator kit (Zymo).

### Example 7. Quantification of o⁸G by ELISA

Colorimetric detection and quantification of o⁸G was performed using the OxiSelect Oxidative RNA Damage ELISA kit (Cell Biolabs) according to the manufacturer's protocol. As sample preparation, purified small RNA or large RNA (0.4-5 µg) was treated with Nuclease P1 (Wako) at 37 °C for 1 hour, extracted through ethanol precipitation, and was added to compete o⁸G/BSA conjugate for binding of anti-o⁸G antibody, pre-coated on the plate, and then reacted with HRP-conjugated secondary antibody provided in the kit for detection. The absorbance of the sample was measured with a spectrophotometer (450 nm, GloMax-Multi Detection System; Promega), and the concentration of o⁸G was estimated by comparison with a predetermined o⁸G standard curve and expressed as a relative quantity.

### Example 8. Drug Treatment

All experimental procedures were approved by the Korea University Laboratory Animal Steering Committee and were performed in compliance with the Animal Protection and Laboratory Animal Use Guidelines. To induce adrenergic cardiac hypertrophy, ISO (75 mg/kg) was administered via intraperitoneal injection (IP) to 8 to 12-week old male C57BL/6J mice (Koatech) every 2 days for a total of 29 days.

As a mock-injection control, an equal volume of PBS was used, and 100 mg/kg NAC was administered to investigate the antioxidant effect. Mice were divided into groups according to drug injected (n=4 in each group, and sample size was selected based on the minimum number used in a previous study (Lee, H. S. et al. Nature Communications 6, 10154)): Mock, ISO, ISO+NAC, and NAC. 29 days after the first injection, cardiac tissue was collected by measuring each heart weight (HW), body weight (BW), and tibia length (TL), and HW/BW or HW/TL was used as normalized heart sizes. All samples were stored at -80 °C until analyzed for ROS measurement, CLEAR-CLIP and RNA extraction followed by o⁸G ELISA, dot blot, Northwestern, IP, and qPCR. For paraquat (Sigma-Aldrich) treatment, 10 mg/kg paraquat was administered to mice via IP every week for a total of 4 weeks. Then, RNA was extracted and used for ELISA. In the case of RNA-Seq, ISO was injected 3 times IP on days 1, 3, and 5, and mice (n=3) were sacrificed one week later, and the same volume of PBS was injected as a control. All samples were stored at -80 °C until used to construct RNA-Seq libraries and qPCR analysis.

### Example 9. Echocardiography

Echocardiograms were monitored and recorded under the control of the Animal Imaging Core Facility at Samsung Medical Center. Specifically, mice were initially exposed to 2 to 5 % isoflurane to induce anesthesia and maintained with 1 % isoflurane while measuring echocardiography (Visual Sonics Vevo 2100 Imaging System) for about 5 min. Under M-mode tracing, a 2-D short-axis was applied to fix individual recording positions, the papillary region of the mouse. Heart rate including diastolic and systolic wall thickness, left ventricular (LV) dimensions, and LV end-diastolic and end-systolic chamber dimensions were measured.

### Example 10. Dot Blot and Northwestern Blot Analysis

For dot blots, size-specific RNA (80-300 ng) or gel-purified miRNA (50 ng) were plotted on a Zeta-Probe blotting membrane (Bio-Rad) and were crosslinked by UV irradiation with an optimal crosslinking (120mJ/cm²) option on a Spectrolinker XL-1000 (Spectroline). For Northwestern blots, total RNA was denatured in Gel Loading Buffer II (2 min at 90 °C; Ambion), isolated with 15 % Novex TBE-Urea gel (Invitrogen) using 22 nt miRNA size marker (synthesized miR-1), transferred to a Zeta-Probe blotting membrane, and crosslinked with UV (120 mJ/cm², Spectrolinker XL-1000). The membranes were blocked with 1 × TBST (Biosesang) with 5 % BSA for 1 h at room temperature and incubated with anti-o⁸G antibody (15A3, 1:2000; QED Bioscience) in 1 × TBST for 1 h at room temperature. Then, HRP-conjugated goat anti-mouse IgG (1:5000, Pierce) was used in TBST at room temperature for 1 hour, followed by reaction with ECL (SuperSignal West Pico PLUS, Pierce) for detection. When using a fluorophore-conjugated secondary antibody (Alexa Fluor 680 Goat anti-mouse IgG, 1:15000 in 1 × TBST, Invitrogen), blocker FL Fluorescence Blocking Buffer (ThermoFisher) was used for both blocking and incubation with the anti-o⁸G antibody. The fluorescence signal was directly quantified by the iBright FL100 imaging system (Invitrogen) and calculated as the intensity relative to the total amount of RNA stained as dots by SYBR Gold (1:10000, Invitrogen).

### Example 11. Optimization of o⁸G Immunoprecipitation (IP)

To optimize the immunoprecipitation (IP) conditions of o⁸G against the anti-o⁸G antibody (15A3, QED Bioscience), o⁸G spike-in (5'p-Uo⁸Go⁸GAAUGUAAAGAAGUAUGUAU-3'), a synthetic 20nt-length RNA containing two o⁸G bases) was compared with G spike-in without o⁸G (5'p-UGGAAUGUAAAGAAGUAUGUAU-3'). To prepare anti-o⁸G antibody-attached beads, 2.5 µg of anti-o⁸G antibody (15A3, QED Bioscience) was incubated in 25 µl of Dynabeads Protein G (Invitrogen) and 200 µl of PBS at room temperature for 1 hour. To prevent non-specific interactions of anti-o⁸G antibodies, the addition of 250 µg of deoxyguanosine (dG, Sigma-Aldrich) was also tested during bead preparation. For washing and purification, Dynabeads Protein G was isolated using a DynaMag-2 magnet (Invitrogen). After washing beads three times with PBS, different conditions of IP buffer were checked for o⁸G spike-in vs. G spike-in (10 pg or 100 pg, in the presence of 300 ng small RNA); PBS containing 0.04 % NP-40; low detergent, PBS containing 0.004 % NP-40; high detergent, PXL (PBS, 0.1 % SDS, 0.5 % deoxycholate, 0.5 % NP-40). For reference, all IP processes were performed at 4 °C for 2 hours in the presence of 2.5 mM DFOM and 40U recombinant RNase inhibitor (Takara).

Other conditions of wash buffer were also tested; high detergent, IP buffer (2 times) > PXL (4 times) > PBS (2 times); addition of a salt, PBS supplemented with 100 mM NaCl (3 times) > PBS (5 times); serial wash with high salt, TE (15 mM Tris-HCl pH 7.5, 5 mM EDTA) > TE containing a high detergent (1 % Triton X-100, 1 % deoxycholate, 0.1 % SDS, and 2.5 mM EGTA) and a high salt (1M NaCl) > TE containing a high detergent and a salt (120 mM NaCl) > wash buffer (50 mM Tris-HCl pH 7.5, 1 mM MgCl, 150 mM NaCl, 0.05 % NP-40); serial wash with extreme salt, TE > TE containing a high detergent and a high salt > TE containing a high detergent and an extreme salt (2M NaCl) > TE containing a high detergent and a salt > wash buffer. RNA in beads was purified with 700 µl of Qiazol Lysis Reagent supplemented with 2.5 mM DFOM and 20 % chloroform, and an RNA Clean & Concentrator-5 kit (Zymo) was used. Since the size of the spike-in control was 22 nt, the amounts of o⁸G spike-in and G spike-in were measured by quantitative RT-PCR (qPCR) for miRNA, and their relative ratios were used to estimate the noise in the signal:IP process.

### Example 12. Quantitative RT-PCR for miRNA (qRT-PCR)

To quantify miRNA, an miRNA qPCR method or a TaqMan MicroRNA assay (Applied Biosystems) was used. Specifically, 1 µg of small RNA or IP purified RNA purified by a miRNeasy Mini Kit (Qiagen) was polyadenylated in a total volume of 10 µl using Poly(A) Polymerase (Ambion). Subsequently, polyadenylated RNA was reverse transcribed using SuperScript III reverse transcriptase (Invitrogen) together with 2 µM of oligo (dT) adapter primer (5'-GCGAGCACAGAATTAATACGACTCACTATAGGTTTTTTTTTTTTVN-3'; SEQ ID NO: 22). qPCR reactions were performed in SYBR Green PCR Master Mix (Applied Biosystems) with forward (same sequence as target miRNA) and reverse primers (5'-GCGAGCACAGAATTAATACGACTCAC-3'; SEQ ID NO: 23); cycling conditions (95 °C for 5 minutes; 95 °C for 15 seconds, 55 °C for 15 seconds, and 72 °C for 20 seconds for 45 cycles; and 72 °C for 5 minutes). U6 snRNA was generally measured as a reference control (forward: 5'-CGCTTCGGCAGCACATATAC-3' (SEQ ID NO: 28), reverse: 5'-TTCACGAATTTGCGTGTCAT-3' (SEQ ID NO: 29)), except using o⁸G or G spike-in (forward: 5'-TGGAATGTAAAGAAGTATGTAT-3' (SEQ ID NO: 24), reverse: 5'-GCGAGCACAGAATTAATACGACTCAC-3' (SEQ ID NO: 25)) or miRNA:o⁸G spike-in (forward: 5'-TAAGGCACGCGGTGAATGCCAA-3' (SEQ ID NO: 26), reverse: 5'-GCGAGCACAGAATTAATACGACTCAC -3' (SEQ ID NO: 27)).

When using the TaqMan MicroRNA Assay, it was performed according to the protocol provided by the manufacturer. That is, 100 ng of small RNA was reverse transcribed using a MicroRNA reverse transcription kit (Applied Biosystems) together with a specific RT probe that recognizes each miRNA sequence. With the RT product, qPCR reactions were performed with specific qPCR primers and a TaqMan Universal PCR Master Mix (No AmpErase UNG; Invitrogen). RT probes and qPCR primers used to detect specific miRNAs were supplied by the following kits: miR-1b and hsa-miR-1 (ID: 002222) in rCMC; miR-1-3p and rno-miR-1 (ID: 002064) in rCMC; miR-184 with -miR-184 (ID: 000485) in rCMC; let-7f and hsa-let-7f (ID: 000382) in rCMC; miR-1 and hsa-miR-1 (ID: 00222) in H9c2, AC16, rCMC and mouse hearts; U6 and U6 snRNA (ID: 001973); o⁸G or G spike-in, hsa-miR-1 (ID: 002222); miRNA:o⁸G spike-in, mmu-miR-124a (ID: 001182); and NT, cel-miR-67-3p (ID: 000224). All qPCR analyses were performed by Rotor-Gene Q (Qiagen) with technical replicates (n=3) and parallel reactions without reverse transcriptase (negative control).

### Example 13. Analysis of o⁸G>T Conversion in RT-PCR

Because o⁸G can pair with A, the efficacy of inducing G>T conversion was investigated based on the change from GAAUo⁸GC to GAATTC (EcoRI site) in the synthesized 39-oxo-R. Reverse transcription of o⁸G control (39-oxo-R) and G control (39R) was performed by SuperScript III reverse transcriptase (Invitrogen) with RT primers (5'-ACTTCAAAGCTCACGGTTCCGCTACCTCGAGAAGACGGCATACGA-3'(SEQ ID NO: 30), Bioneer). cDNA was amplified with Ex Taq (Takara) by PCR reaction (30 cycles of 98 °C for 10 seconds; 98 °C for 10 seconds, 40 °C for 30 seconds, 72 °C for 20 seconds; and 72 °C for 10 min) together with primers (forward: 5'-CCTGGTCCCAGACTAAAGAAT-3'(SEQ ID NO: 31), reverse: 5'-ACTTCAAAGCTCACGGTTCCG-3'(SEQ ID NO: 32); Bioneer).

After purification with the Oligo Clean & Concentrator kit (Zymo), the RT-PCR product was digested with EcoRl (NEB) and isolated on a 12 % PAGE gel in TBE.

The o⁸G-induced G>T conversion in RT-PCR was also directly confirmed by sequencing. After each ligation step, a sequencing library for 400 ng of o⁸G including 22nt synthetic RNA (5'p-UGGAAUo⁸GUAAAGAAGUAUGUAU-3'; SEQ ID NO: 33) according to the protocol for constructing a small RNA-Seq library with modifications (see Example 14), except using the RNA Clean & Concentrator-5 kit (Zymo) was constructed. In particular, it is possible to analyze the nucleotide frequency of the o⁸G position by calculating a unique read value due to a degenerate barcode (4 random nucleotides) in the 5' adapter, thereby eliminating confounding bias in PCR amplification.

### Example 14. o⁸G-miSeq

Based on the optimization of o⁸G IP conditions and o⁸G-induced G>T conversion in RT-PCR, a sequencing method (o⁸G-miSeq) was developed to identify oxidized miRNAs and their o⁸G positions. 12.5 µg of anti-o⁸G antibody (15A3, QED) alternately with 125 µl of Dynabeads Protein G (Invitrogen) in PBS (total volume 500 µl) supplemented with 1.2 mg deoxyguanosine (Sigma) at room temperature for 1 hour Bioscience) was incubated to prepare magnetic beads with an antibody attached thereto. For washing and purification, Dynabeads Protein G was isolated using a DynaMag-2 magnet (Invitrogen). After washing with PBS twice, 7 to 10 µg of small RNA sample purified from miRNeasy Mini Kit (Qiagen) was mixed with beads prepared in 200 µl of PXL containing 2.5 mM DFOM (Sigma) and 40U recombinant RNase inhibitor (Takara). After 2 hours of alternating incubation at 4 °C, a series of washes with extreme salt were applied; TE (15 mM Tris-HCl pH 7.5, 5 mM EDTA); TE containing a high detergent (1 % Triton X-100, 1 % deoxycholate, 0.1 % SDS, and 2.5 mM EGTA) and a high salt (1M NaCl); TE containing a high detergent and an extreme salt (2 M NaCl); TE containing a high detergent and a salt (120 mM NaCl); wash buffer (50 mM Tris-HCl pH 7.5, 1 mM MgCl, 150 mM NaCl, 0.05 % NP-40). Purified oxidized small RNA on the beads was further processed as it is or after extraction.

For treatment with intact beads, RNA of the beads was washed twice with PNK buffer (50 mM Tris-Cl pH 7.4, 10 mM MgCl₂, 0.5 % NP-40), was incubated using 8 µl of T4 RNA ligase 2 cleavage KQ (NEB) in 80 µl T4 RNA ligase buffer (NEB) supplemented with 1 mg/ml BSA and 40U recombinant RNase inhibitor (Takara) at 70 °C for 2 minutes, and was ligated with 0.25 µM of a 3'adaptor (5'-(rApp)T(PMe)GGAATTCTCGGGTGCCAAGG(ddC)-3'(SEQ ID NO: 34); rApp: adenylate, PMe: methyl-phosphonate, ddC: dideoxy-cytosine; Trilink) which was prepared on ice.

After incubation for 1 hour at 28 °C in a thermomixer (Thriller, Peqlab), the beads were washed once with PXL (2.5 mM DFOM) and twice with PNK buffer. For 5' adapter ligation, 0.25 µM of 5'adaptor (5'-GUUCAGAGUUCUACAGUCCGACGAUCNNNN (2'-methoxy-C)-3' (SEQ ID NO: 35); Trilink) prepared on ice after incubation at 70 °C for 2 min was incubated with RNA for 1 hour at 28 °C in a thermomixer (Thriller, Peqlab) on beads of a total of 80 µl of T4 RNA ligase buffer consisting of 8 µl of T4 RNA ligase (Thermo), 1 mg/ml BSA, and 40U recombinant RNase inhibitor. After washing sequentially with PXL (2.5 mM DFOM) and 1x first-strand buffer (Invitrogen), RNA on the beads was reverse transcribed as follows; 1 µl SuperScript III reverse transcriptase (Invitrogen), 0.72 µM RT primer (5'-GCCTTGGCACCCGAGAATTCCA-3'; SEQ ID NO: 36), 375 µM dNTP, 7.25 mM DTT, and 4U recombinant RNase inhibitor in a total of 40 µl 1x first-strand buffer; and Incubation for 1 hour at 55 °C in a thermomixer (Thriller, Peqlab). The obtained cDNA was further amplified by PCR to construct a sequencing library.

Simultaneously, the cDNA sequencing library was also purified from the beads with 700 µl of Qiazol Lysis Reagent supplemented with either input small RNA (0.4-1 µg; small RNA-Seq) or o⁸G IP (2.5 mM DFOM and 20 % chloroform), and then was carried out from the product extracted from RNA (concentrated with Clean & Concentrator-5 kit).

First, a 0.25 µM 3'adaptor (prepared on ice after incubation at 70 °C for 2 min) was ligated using 1 µl T4 RNA ligase 2 cleavage KQ (NEB) in 13 µl total T4 RNA ligase buffer supplemented with 20 % PEG 8000 and 20U recombinant RNase inhibitor prepared on ice). After incubation at 28°C for 60 minutes, it was inactivated at 65 °C for 20 minutes. Then, 13 µl of T4 RNA ligase buffer containing 0.25 µM 5'adaptor, 2 µl of T4 RNA Ligase (ThermoFisher), 40U recombinant RNase inhibitor, and 1 mg/ml BSA were added to ligate the 5'adaptor, and it was incubated at 28 °C for 60 minutes and inactivated at 65 °C for 20 min. Ligated total RNA samples were denatured with 1 µl of 10 µM RT primer at 70 °C for 2 min, placed on ice for 1 min, and reverse transcribed as follows: 1 µl Superscript III reverse transcriptase (Invitrogen), 375 µM dNTP, 7.25 mM DTT and 4U recombinant RNase inhibitor in a total of 40 µl 1x first strand buffer; and it was incubated at 55 °C for 1 hour and at 70 °C for 15 minutes. The resulting cDNA was further amplified through PCR to prepare a sequencing library.

The prepared cDNA was further processed to include the Truseq index adapter sequence (Illumina). First, cDNA was amplified via PCR using Q5 high-accuracy 2x master mix (NEB) with 100 nM universal forward primer (5'-AATGATACGGCGACCACCGAGATCTACACGTTCAGAGTTCTACAGTCCGA-3'; SEQ ID NO: 37) and 100 nM RT primer; 30 seconds at 98 °C; 5 cycles of 10 seconds at 98 °C, 30 seconds at 60 °C and 15 seconds at 72 °C; and at 72 °C for 10 min. After purification of the primary PCR product using the QIAquick PCR Purification Kit (Qiagen), qPCR (Rotor-Gene Q; Qiagen) was performed by adding SYBR Geen I (1:10000, Invitrogen) to the Q5 high-accuracy 2x master mix using a portion of the elution, and the number of cycles required to reach a linear range of amplification was determined. Secondary PCR was performed for the remaining purified primary PCR products to generate multiplexing barcodes (250 nM universal forward primer, 250 nM barcode primer: 5'-CAAGCAGAAGACGGCATACGAGAT-6mer barcode-GTGACTGGAGTTCCTTGGCACCCGAGAATTCCA-3'; SEQ ID NO: 38) using Q5 high-accuracy 2x master mix with a set of optimal cycles. The expected size of the PCR product containing about 20nt insert was further purified using Pippin Prep (Sage Science) with 15 % Urea-PAGE gel extraction (see Example 6) or 3 % agarose gel cassette, quantification was performed using a Qubit RNA HS assay kit (Invitrogen) and Fragment Analyzer (Advanced analytical) together with cross-check. Finally, the prepared library was sequenced as 50 single-ended reads by the HiSeq 2500 system (Illumina) and demultiplexed using CASAVA (Illumina).

### Example 15. Bioinformatics and Statistical Data Analysis

For bioinformatics analysis, Python script (http://clip.korea.ac.kr/oxog/) and UCSC genome browser (http://genome.ucsc.edu/) were mainly used. RNA-Seq analysis was performed using the Tuxedo Suite; TopHat2 (http://ccb.jhu.edu/software/tophat), Cufflinks and Cuffdiff (http://cole-trapnelllab.github.io/cufflinks/). Mapping of sequencing reads to miRNAs was performed using Bowtie (http://bowtie-bio.sourceforge.net). Peak analysis of CLIP data was processed by CLIPick (http://clip.korea.ac.kr/clipick/). Unless otherwise indicated, GO analyzes were performed using DAVID (http://david.abcc.ncifcrf.gov/) with default parameters and visualized with REVIGO (http://revigo.irb.hr/). The reinforcement of functional annotations was analyzed using GSEA (http://software.broadinstitute.org/gsea/).

Standard laboratory practice randomization procedures were used for cell line groups and mice of the same age and sex. Experimenters were not given any information about their placement during the experiment and outcome evaluation. All statistical tests, including the Kolmogorov-Smirnov test (KS test), t-test (unpaired two-tailed), and Fisher's exact test (relative to mapped total miR-1 readings), were obtained from Scipy (http://www.scipy. org/) or Excel (two-sided test). Values represent mean±s.d unless otherwise stated. Statistical significance was set to P=0.05 by default, and the variance was similar between groups.

### Example 16. o⁸G-miSeq Data Analysis

Demultiplexed sequencing reads (FASTQ files) were aligned using Bowtie tolerant of two mismatches (Bowtie --norc -l 7 -n 2 -a -f), and reduced sequencing reads were indexed (using bowtie-build indexer with default parameters) and mapped by mature miRNA sequences of the same species in miRBase (http://www.mirbase.org/). The results were further parsed by filtering out reads with mismatch errors, resulting in a quality score of less than 20. The number of reads per miRNA was normalized to total reads (reads per million total reads; RPM) and used as log₂ values (e.g., log₂ (input) and log₂ (o⁸G IP)). In particular, the enrichment of o⁸G was estimated by measuring the RPM of o⁸G IP sequencing (o⁸G IP) with the RPM of input small RNA-Seq (input; miRNA frequency), calculated in a log₂ ratio and expressed as significance (log₁₀ (P-value), t-test) in volcano plot analysis. To investigate the o⁸G IP bias affected by the frequency of G in the miRNA sequence, the number of miRNAs classified by G-content in the sequence (number of Gs) and o⁸G enrichment value (bin size = 1) was visualized through heat map analysis (Treeview, http://rana.lbl.gov/EisenSoftware.htm). To analyze the differential o⁸G enrichment according to PE treatment, the relative o⁸G enrichment value was calculated from an o⁸G enrichment ratio between PE treatment vs. mocks (no treatment)

Considering only the cases of mismatches with the highest quality score in base calling, the percentage of mismatches occurring at each position in the miRNA sequence was calculated relative to the total match frequency: Inconsistency (%). In particular, the seed sequences from miR-1:2U and miR-1:3U were not overlapped with other mammalian miRNAs (n=17,948; miRBase, http://www.mirbase.org/) except miR-1:7U with species-specific miRNAs, mdo-miR-12320-3p and mmu-miR-7216-3p (<80 sequencing reads) showing very low expression that can be treated as negligible.

### Example 17. Quantification of Oxidized miRNA by o⁸G IP and qPCR

After inducing hypertrophic hearts in mice by treatment of rCMC (n=3) with 100 µM PE for 48 hours or by chronic injection of ISO (75 mg kg⁻¹) (n=3), small RNA was extracted using miRNeasy Mini Kit (Qiagen). Then, as cross-confirmation, the same amount of small RNA (2 µg, PE treated vs. no treatment) measured accurately by both spectrophotometer (Denovix) and Qubit fluorescence quantification (Invitrogen) was used for o⁸G immunoprecipitation in the presence of 10 pg miRNA:o⁸G spike-in control. After RNA was extracted from the beads using Qiazol Lysis Reagent and RNA Clean & Concentrator-5 kit (Zymo), the amount of specific miRNA of o⁸G IP was evaluated using a TaqMan MicroRNA Assay kit (Applied Biosystems). For reference, all procedures before reverse transcription were performed in the presence of 2.5 mM DFOM to prevent oxidation of RNA in vitro. To normalize the mutation from the IP process, the amount of miRNA: o⁸G spike-in in o⁸G IP was measured by qPCR and used as a denominator to quantify the relative o⁸G value of a specific miRNA. In addition, by measuring the U6 snRNA in o⁸G IP, the relative level was estimated and used for normalization to confirm the enrichment of oxidation in specific miRNAs.

### Example 18. Construction of Luciferase Reporter

To measure miRNA-mediated gene suppression activity, psiCheck-2 (Promega) or pmirGLO vector (Promega) was used. In particular, in the case of the miR-1 oxo site, the detection sensitivity was increased by repeating the target site side by side (n=5) and inserting it into the 3'UTR of Renilla luciferase (hRLuc) of psiCheck-2.

For the construction of this vector, synthetic duplex oligos (Macrogen) containing various miR-1 oxo sites were cloned into the psiCheck-2 plasmid via Xhol and Notl sites as indicated; miR-1-seed site, forward: 5'-TCGAGACATTCCACATTCCACATTCCACATTCCACATTCCGC-3' (SEQ ID NO: 39), reverse: 5'-GGCCGCGGAATGTGGAATGTGGAATGTGGAATGTGGAATGTC-3'(SEQ ID NO: 40); miR-1-2oxo site, forward: 5'-TCGAGACATTCAACATTCAACATTCAACATTCAACATTCAGC-3' (SEQ ID NO: 41), reverse: 5'-GGCCGCTGAATGTTGAATGTTGAATGTTGAATGTTGAATGTC-3' (SEQ ID NO: 42); miR-1-3oxo site, forward: 5'-TCGAGACATTACACATTACACATTACACATTACACATTACGC-3' (SEQ ID NO: 43), reverse: 5'-GGCCGCGTAATGTGTAATGTGTAATGTGTAATGTGTAATGTC-3' (SEQ ID NO: 44); miR-1-7oxo site, forward: 5'-TCGAGAAATTCCAAATTCCAAATTCCAAATTCCAAATTCCGC-3' (SEQ ID NO: 45), reverse: 5'-GGCCGCGGAATTTGGAATTTGGAATTTGGAATTTGGAATTTC-3'(SEQ ID NO: 46); miR-1 control site, forward: 5'-TCGAGACTTTCCACTTTCCACTTTCCACTTTCCACTTTCCGC-3' (SEQ ID NO: 47), reverse: 5'-GGCCGCGGAAAGTGGAAAGTGGAAAGTGGAAAGTGGAAAGTC-3' (SEQ ID NO: 48)). Importantly, the miR-1 control site has a mismatch at position 6 (pivot) of the miR-1 seed site by having the same base, where an impaired pivot is reported to lose seed-mediated target inhibition, and thus it was constructed to be used as a negative control.

In the same manner as above, the sequence recognizable when generating o⁸G modifications at the seed site of miR-122, let-7, and miR-124 was prepared by substituting the site where the o⁸G modification occurs to the 8th complementary sequence based on the 5'-end with A.

To sensitively identify the 7oxo site in the identified miR-1:7o⁸G target mRNA, the psiCheck-2 vector was modified and subcloned to contain 2 or 3 repeats of the target site as follows. First, the firefly gene (hluc+) containing the predicted offset 7oxo site (3:8) was replaced by a different firefly gene (Luc2) that lacks the 7oxo site and is located in the pmirGLO vector (Promega), via the Apal and Xbal sites.

### Example 19. Luciferase Reporter Analysis

Various luciferase reporter vectors (pmirGLO, psiCheck-2, or a modified plasmid thereof containing the desired site) and/or synthetic duplex miRNAs (50-75 nM, otherwise specified) were transfected into H9c2, rCMC, and AC16. Twenty-four hours after transfection, the activity of the expressed luciferase was generally measured. In the case of drug treatment, PE, H₂O₂, and/or NAC at the indicated concentrations were treated for the specified time, starting at 24 hours post-transfection and obtained post-treatment. Relative activity (Renilla luciferase activity normalized to firefly luciferase) was measured using a dual-luciferase reporter assay system (Promega) with a GloMax-Multi Detection System (Promega) in replicates (n=6) according to the manufacturer's protocol. To evaluate the efficiency of miRNA-mediated inhibition, different concentrations of miRNA (0 to 100 nM) were transfected into HeLa. Then, using Scipy (scipy.optimize.curve_fit), nonlinear least squares fitting was performed on the sigmoid function to calculate the IC₅₀. An approximate IC₅₀ was calculated from the regression line if the least squares did not fit the function.

### Example 20. Dual Fluorescent Reporter Preparation

A double fluorescent protein (dFP) reporter vector was constructed based on psiCheck-2 (Promega), and the luciferase gene was replaced by a fluorescent protein gene. The eGFP gene was amplified in pUlta (Addgene; provided by Malcolm Moore) and hRLuc was replaced via Nhel and Xhol sites; forward primer: 5'-TAGGCTAGCCACCATGGTGAGCAAGGGCGA-3' (SEQ ID NO: 49), reverse primer: 5'-GGGCTCGAGCGATCGCCTAGAATTACTTGTACAGCTCGTCCATGC-3' (SEQ ID NO: 50). TurboRFP gene was amplified in pTRIPZ (Thermo Scientific), hluc+ was replaced via Apal and Xbal site (forward primer: 5'-GAAGGGCCCTATGAGCGAGCTGATCAAGGAGA-3' (SEQ ID NO: 51), reverse primer: 5'-GACTCTAGAATTATTATCTGTGCCCCAGTTTGCTAG-3' (SEQ ID NO: 52)), and further processed to remove the residual 33bp of the hluc+ sequence through PCR-mediated deletion (forward primer: 5'-GTACTGTTGGTAAAGCCACCATGAGCGAGCTGATCA-3' (SEQ ID NO: 53), reverse primer: 5'-TCCTTGATCAGCTCGCTCATGGTGGCTTTACCAACA-3' (SEQ ID NO: 54)), then the non-deleted backbone plasmid was removed (Dpnl treatment). All PCR reactions were performed using PfuUltra High-Fidelity DNA Polymerase (Agilent Technologies) according to the manufacturer's protocol. In addition, a converted dFP vector (p.UTA.3.0 Empty provided by Jens Gruber; Addgene plasmid #82447) was used, which was derived from the psiCheck-2 vector, but acGFP replaced hluc+ and RFP replaced hRLuc as if the reporter and control fluorescent genes were exchanged in dFP. Finally, the miR-1 seed site, 7oxo site, 2oxo site, and 3oxo site were inserted into the dFP or converted dFP vector (p.UTA.3.0) according to the same subcloning method used for psiCheck-2.

### Example 21. Single Cell Reporter Assay Using Flow Cytometry

A fluorescent protein reporter vector was used to measure miRNA-mediated gene inhibition at the single cell level using flow cytometry. First, dFP (RFP:GFP-site) or converted dFP (GFP:RFP-site) containing miR-1 seed, 7oxo, 3oxo, or 2oxo site was transfected into H9c2, and cells were collected 24 hours after transfection. As a positive control, a cognate miRNA (50 nM) for each target site was cotransfected. To confirm that inhibition of miR-1 oxo sites is mediated by endogenous miR-1, 50 nM miR-1 specific inhibitor obtained from miRIDIAN microRNA hairpin inhibitor (Dharmacon) was also cotransfected.

For cotransfection, the same amount of NT was used as a control. For drug treatment, either 200 µM PE (serum-depleted H9c2) or 2 mM NAC was treated for 24 h starting at the time of transfection. Cells were harvested in PBS (Biosesang) with 4 % BSA (Bovogen) and 5 mM EDTA (Sigma) and analyzed by flow cytometry.

For dFP (RFP:GFP-site) transfected H9c2, BD Accuri C6 Plus (BD Biosciences) operated in a 4-blue configuration initially was used (only blue laser equipment, excitation wavelength: 488 nm; standard filter, GFP: 533/30, RFP: 670 LP). After gating of live single cells (based on FSC and SSC), cells without reporter expression were further filtered out based on the basal level of autofluorescence, estimated by psi-Check2 transfected cells. Then, scatter plots of GFP vs. the RFP signal (n=10,000 cells) were analyzed. The cell size of H9c2 was estimated by the FSC value. To test the effect of PE treatment on oxidized miR-1 mediated inhibition, cell distribution according to GFP intensity (log₂(GFP)) or relative GFP ratio (log₂(GFP/RFP)) was shown by comparing PE treatment vs. no treatment, and was further statistically analyzed using cumulative fraction analysis (Scipy, scypy.stats.ks_2samp) KS test).

To increase detection sensitivity for both dFP (RFP:GFP-site) and converted dFP (GFP:RFP-site), GFP and GFP and full excitation of both GFP and RFP was then achieved using an Attune NxT flow cytometer equipped with blue (488 nm) and yellow (561 nm) lasers. For this analysis, the ranges of GFP and RFP signals were initially selected in scatter-plot analysis, and reporter values (log₁₀(GFP) for dFP and log₁₀(GFP) for dFP) derived from a similar range of vector expression (estimated by log₁₀(RFP) for dFP and log₁₀(GFP) for converted dFP; entering the same) log₁₀(RFP)) were averaged and quantified as a relative ratio. Ultimately, the relative fold change was calculated by normalizing the relative proportions to the values derived from the same reporter without the site (e.g., the average of the GFP values in each bin of dFP and RFP values is expressed as the value from the control without the site). To narrow the analysis down to only the lowest 25 % in reporter expression, the relative fold change was recalculated by using only the cells with the lowest 25 % of the reporter value in the bin of each vector expression value.

### Example 22. Induction of Cardiac Cell Hypertrophy using miR-1:o⁸G

NT-6Ø, miR-1, miR-1:7o⁸G, miR-1:2o⁸G, miR-1:3o⁸G, miR-1:7U, miR-1:2U, and miR-1:3U were transfected into H9c2 or rCMC cell with RNAiMax or Lipofectamine 3000 (Invitrogen) according to the manufacturer's protocol, and in order to obtain still images, an inverted microscope (Leica DMi8) was used and analyzed with imaged (≥ 100 cells, https://imagej.nih.gov/). To generate time-lapse images, after transfection with miR-1:7o8G or miR-1:7U, a Lumascope 400 (Etaluma) was used at 20-minute intervals between 0 and 50 h.

### Example 23. Administration of miRNA to Mice

In vivo delivery of miRNA to mice was performed. Specifically, duplex miRNA (5 mg kg⁻¹) was administered into 12-week-old male C57BL/6 mice (Koatech) via intravenous injection (I.V.) using in vivo jetPEI (N/P ratio=5; Polyplus) according to the manufacturer's protocol. Two sets of experiments were designed by distributing mice into two groups according to the injected miRNA (NT vs. miR-1:7o⁸G, NT vs. miR-1:7U; n=4). When miR-1:7o⁸G (4 mg kg⁻¹) or miR-1:7U (4 mg kg⁻¹) was injected, and NT (1 mg kg⁻¹) was also injected to confirm delivery to the mouse heart. In particular, the duplex of miR-1:7o⁸G or miR-1:7U used for injection was generated by confirming delivery to cardiac tissue using the miR-1 passenger strand containing FITC at the 5'-end. Injections were performed twice on days 1 and 2 and mice were sacrificed on day 7. For each cardiac measurement, cardiac tissue was collected: HW, BW, and TL. The cut cardiac tissue was immediately used for RNA extraction (small RNA and large RNA; miRNeasy Mini Kit, Qiagen), and the amount of delivered NT was investigated by analysis through qPCR (see detailed method of qPCR for miRNA section) followed by RNA-Seq and qPCR.

Portions of the amputated hearts were also prepared as tissue sections on slides for immunohistochemical analysis.

### Example 24. Quantitative RT-PCR for mRNA

Total RNA was isolated using RNeasy Mini Kit (Qiagen) through column DNA digestion using RNase-Free DNase Set (Qiagen). Reverse transcription was performed with SuperScript III reverse transcriptase (Invitrogen) and oligo (dT) primers. qPCR analysis was performed with SYBR Green PCR Master Mix (Applied Biosystems). All reactions were performed in triplicate with a standard two-step cycling protocol, and relative quantitation was calculated by the ΔCT method using ACTB and GAPDH as controls.

### Example 25. Immunohistochemistry of Cardiac Tissue

The dissected mouse cardiac tissue was fixed (4 % paraformaldehyde, 4 °C overnight), stored (70 % ethanol), embedded in paraffin, and cut (cross-section, continuous section; 5 µm width), and were stained with haematoxylin and eosin (H&E). Paraffin-embedded section preparation, H&E staining, and Masson's trichrome staining were performed by the Cardiovascular Product Evaluation Center (Yonsei University School of Medicine, Korea) or the pathology core facility (Seoul National University College of Medicine, Korea) through service subscription. Then, immunofluorescence staining was performed on the prepared slides. After deparaffinizing the sections with Histo-Clear (National Diagnostics), the sections were hydrolyzed with serially diluted ethanol and stored in PBS. Antigens were recovered using BD Retrievagen Antigen Retrieval Systems (BD Biosciences) according to the manufacturer's protocol. Then, the target staining area defined by the PAP pen (Sigma) was immunostained as specified; blocking solution (PBS with 10 % normal goat serum and 1 % BSA), RT for 1 h; MF20 antibody (1:200, Developmental hybridoma), blocking solution, overnight at 4 °C; Alexa Fluor 488 donkey antimouse IgG (1:1000, Abeam), blocking solution, 1 h at room temperature. In addition, Alex Fluor 594-conjugated WGA (50 µg/ml; Invitrogen) and DAPI (1.5 µg/ml; Vector lab) were applied to visualize the cell membrane and nucleus, respectively. Stained tissues were observed with an inverted fluorescence microscope (Leica DMi8; 20x or 40x magnification), analyzed with Leica Application Suite (LAS), and quantified with imaged (≥ 100 cells, https://imagej.nih.gov/).

### Example 26. Transformed Mice Generation

To produce cardiomyocyte-specific transformed mice (TG), the anti-7oxo (α-MHC 13x) plasmid was digested using BamHl to separate the transformation cassette. Gel-purified transformation cassettes were injected into the nucleus of mouse fertilized eggs prepared from superovulated C57BL/6 female mice (induced by PMSG and HCG) after mating. Thereafter, mouse embryos (fertilized single-celled fertilized eggs) were transplanted into pseudopregnant female mice (Korea Bio Co. Ltd, Seoul, Korea). After delivery, both anti-7oxo TG (+) mice were identified by PCR analysis of genomic DNA (purified from the ends) using a primer set corresponding to hGH poly(A) (forward: 5'-CCACCAGCCTTGTCCTAATAAA-3'(SEQ ID NO: 55), reverse: 5'-CAGCTTGGTTCCCAATAGA-3'(SEQ ID NO: 56)) using PCR analysis of genomic DNA (purified from the ends). For anit-7oxo TG, four independent founders (F₀; #44, #65, #68, and #69) were established and studied. All mice were given food and water ad libitum and were maintained on a 12:12 h light:dark cycle.

### Example 27. RNA-Seq library construction and analysis

RNA-Seq libraries were generated from whole or large RNA using adapter ligation-based methods or strand-displacement stop/ligation methods. For the adapter ligation-based method, 100 ng of total RNA (miRNeasy Mini Kit; purified from Qiagen) was applied to the TruSeq Stranded mRNA Library Prep Kit (Illumina) using NeoPrep (Illumina) according to the manufacturer's protocol; sample names: H9C2-NT-N1, H9C2-miR-1-2o⁸G-N1, and H9C2-miR-1-3o⁸G-N1. In addition, TruSeq Stranded mRNA Libraries were constructed, sequenced, and demultiplexed by Omega Bioservices (Norcross, GA, USA); sample names: H9C2-NT-O1, H9C2-NT-O2, H9C2-miR-1-7o⁸G-O1, and H9C2-miR-1-7o⁸G-O2.

The remaining samples were processed using the strand displacement stop/ligation method as follows. That is, from 1.5 µg of large RNA (> 200nt, RNeasy or miRNeasy Mini Kit; extracted by Qiagen), polyadenylated mRNA was purified using 10 µl of Dynabead Oligo (dT)₂₅ (Invitrogen) according to the protocol provided by the manufacturer. Then, an RNA-Seq library was prepared for 10 ng of purified mRNA using the SENSE Total RNA-Seq library kit (Lexogen) according to the manufacturer's instructions. After the libraries were generated, they were amplified by PCR with the lowest optimal cycle, which was determined by comparing the results of different cycles or by performing qPCR as described in the method of o⁸G-miSeq in Example 14. The quality of the amplified library was confirmed by using a Fragment Analyzer (Advanced Analytical) for size distribution and quantity.

If necessary, urea-PAGE gel extraction or Pippin Prep (Sage Science) was further used for size selection. Finally, the prepared multiplexed library was accurately quantified using the Qubit RNA HS analysis kit (Thermo Fisher) and the Fragment Analyzer and applied to be sequenced by the HiSeq 2500 system (Illumina) as 50 single-ended reads. For sequencing, samples named as H9C2-cont, H9C2-anti-7oxo, mHT-ISO-cont, mHT-ISO-anti-7oxo, mHT-ISO-anti-7oxo-TG(-), and mHT-ISO-anti-7oxo-TG(+) and the MiniSeq system (Illumina) were used. To ensure consistency with the results of the HiSeq 2500 system, only SE50 information was used for the MiniSeq system.

Demultiplexed sequencing reads (CASAVA; obtained using Illumina) were aligned to the mouse genome (mm9) or rat genome (rn5) using TopHat2 (tophat2 -a 4 -g 1 --b2-sensitive -r 100 --mate-std-dev=50 --library-type fr-firststrand) with the feed of the RefSeq gene annotation. When the sequencing library was prepared using the strand displacement stop/ligation method, the first 9 nucleotides from the start side of the read were trimmed against TopHat2 according to the manufacturer's instructions. The transcription level was quantified using Cufflinks (cufflinks-N-b), and differential transcriptional profiles were analyzed by Cuffdiff (Cuffdiff --FDR=0.1 -b -N --min-alignment-count=10 - library-norm-method=geometric) with the feed of mapping results in groups with identical experimental conditions. Only valid status values were selected and used in addition to fold change and statistical significance (p-value). If duplicates were derived from two different library construction methods (adapter ligation-based method and strand displacement stop/ligation method), cufflinks were used only for the same type of library, and then median RPKM and p-value (t-test, two-tailed method) were calculated from all replicates.

### Example 28. Cumulative Fraction Analysis

To test the propensity of putative target transcript inhibition or de-repression according to a given miRNA or miRNA inhibitor, the cumulative fraction with fold change (log₂ ratio) was analyzed. The putative miR-1:o⁸G target was selected as a transcript containing the miR-1 oxo site at the 3'UTR (defined by RefSeq downloaded from the UCSC genome browser), where all 6mers matching position 2-8 of miR-1:o⁸G (matches of o⁸G:A instead of o⁸G:C) were normally investigated unless otherwise indicated. "Site-free" refers to transcripts that do not contain miR-1 seed sites and oxo sites in the mRNA sequence. "Cont site" refers to a transcript containing a miR-1 control site in the 3'UTR, and the nucleotide binding to o⁸G in the oxo site was substituted with G as used as a negative control in the previous study. KS test was performed using Scipy (scypy.stats.ks_2samp) for total mRNA or cont sites. The Volcano plot was analyzed by calculating the fold change and significance (-log₁₀ (p-value)), and among them, the differentially expressed gene (DEG) was selected using the cutoff.

### Example 29. Ago HITS-CLIP Data Analysis

Ago HITS-CLIP results from left ventricular tissue of cardiomyopathy patients were retrieved from the GEO database (GSE83410). Reads containing miRNA sequences were identified with two mismatch tolerances by 'reverse' mapping from Bowtie (alignment of mature miRNA sequences to sample reads), and positional mismatches of miRNA reads were analyzed for miR-1. For enrichment analysis of miR-1 oxo sites, compared to miR-1 seed sites mediated by G:U binding, as provided by a previous study (Spengler, R. M. et al., Nucleic Acids Res 44, 7120-7131), Ago2 binding clusters were analyzed. The seed site of miR-124, a brain-specific miRNA, was used as a negative control because it was not present in cardiac tissue. A non-nucleated bulge site of miR-1 was also used as a negative control.

To identify the miR-1 7oxo site in cardiomyopathy patients, raw sequencing data of Ago HITS-CLIP was processed by CLIPIck. Specifically, the FASTQ file was initially filtered and reduced (fastq2collapse.pl) based on the quality score (fastq_filter.pl -f mean:0-24:20). The pretreated reads were then aligned to the human genome (hg19) using the NovoAlign program (http://www.novocraft.com) with the same parameters. After selecting reads from the annotated transcript (RefSeq), the aligned Ago CLIP reads were generated as BED or BAM files using BedTools and SAMtools (http://samtools.sourceforge.net/) and human heart (derived from RNA-Seq Atlas, http://medicalgenomics.org/rna_seq_atlas) was used for peak analysis with expression profile feed. To visualize peaks, all edited reads, and putative miR-1 7oxo sites (6mers at positions 2 to 8), the UCSC genome browser (http://genome.ucsc.edu/) was applied.

### Example 30. CLEAR-CLIP

Mouse cardiac tissue (about 150 mg) dissected from ISO-treated or PBS-treated mice was crushed and covalent crosslinking was induced in RNA-protein complexes in vivo by UV irradiation (400 mJ/cm², 3 times; Spectrolinker XL-1000). Ago-related fragment mRNA, processed by treatment with 50 µU/µl RNAse A (Affymetrix) at 37 °C for 5 minutes, was immunoprecipitated with 10 µg of two different anti-Ago antibodies (2A8; Diagenode, 2E12; Abnova) attached to 60 mg of Dynabeads Protein A (Invitrogen). Ligation to generate miRNA-targeted mRNA chimeras was performed by treatment with 0.625 U/µl T4 RNA ligase 1 (NEB) at 16 °C overnight, and was further enhanced by treatment with 1U/µl T4 RNA ligase 1 (NEB) in the presence of 3 % DMSO (Sigma) and 18 % PEG8000 (Sigma) at 25 °C for 75 min. The isotope-labeled RNA-protein complex was isolated by size by operating NuPAGE 10 % Bis-Tris Gel (Invitrogen), transferred to a nitrocellulose membrane (BA85; Whatman), cut, treated with 4 mg/ml proteinase K (Roche) and 7M urea (Sigma), and extracted with acid phenol:chloroform:IAA (125:24:1; Invitrogen) and RNA Clean & Concentrator-5 kit (Zymo). The purified RNA was subjected to adapter ligation and RT-PCR to generate a library for high-throughput sequencing by using the small RNA-Seq Library Prep kit (Lexogen), and finally sequenced with the HiSeq 2500 system (Illumina).

Sequencing reads were pretreated and mapped, and after adapter sequences were removed, potential chimeric reads containing miRNA sequences were identified by 'reverse' mapping, where the mature miRNA sequences were reverse-aligned to the sample library using Bowtie. The remaining sequences were then mapped to the mouse genome (mm9), only those overlapped with the combined exons from both the 2A8 and 2E12 antibodies were further selected, and only miR-1 containing the chimeric reads was ultimately focused. For miR-1 seed and oxo site investigations, comparing enrichment results between ISO-injected and non-injected mouse hearts, upstream (5', -) and downstream (3', +) were extended from the mapped chimeric reads (-/+ 0, -/+ 25, -/+ 50, -/+ 100).

### Example 31. Construction of Cardiomyocyte-specific miR-1:7o⁸G inhibitors

To specifically inhibit miR-1:7o⁸G in cardiomyocytes, a competitive inhibitor containing multiple miR-1 7oxo target sites was constructed based on the pJG/ALPHA MHC plasmid carrying the cardiomyocyte-specific α-MHC promoter. Synthetic double oligos (Macrogen) containing 13 miR-1 7oxo sites (5'-AAAUUCC-3'; SEQ ID NO: 6) or control NT target sites (5'-GGUUGUG-3'; SEQ ID NO: 21) were cloned into the pJG/ALPHA MHC vector as indicated via the Sall and Hindlll sites; anti-7oxo, α-MHC 13x, forward: 5'-TCGACAAATTCCAAAAATTCCATAAATTCCAGAAATTCCACAAATTCCAAAA ATTCCACAAATTCCATAAATTCCAGAAATTCCAAAAATTCCATAAATTCCAG AAATTCCACAAATTCCAA-3' (SEQ ID NO: 57), reverse: 5'-AGCTTTGGAATTTGTGGAATTTCTGGAATTTATGGAATTTTTGGAATTTCTG GAATTTATGGAATTTGTGGAATTTTTGGAATTTGTGGAATTTCTGGAATTTA TGGAATTTTTGGAATTTG-3' (SEQ ID NO: 58); cont, anti-NT, forward: 5'-TCGACGGTTGTGAAGGTTGTGATGGTTGTGAGGGTTGTGACGGTTGTGAA GGTTGTGACGGTTGTGATGGTTGTGAGGGTTGTGAAGGTTGTGATGGTTG TGAGGGTTGTGACGGTTGTGAA-3' (SEQ ID NO: 59), reverse: 5'-AGCTTTCACAACCGTCACAACCCTCACAACCATCACAACCTTCACAACCCT CACAACCATCACAACCGTCACAACCTTCACAACCGTCACAACCCTCACAAC CATCACAACCTTCACAACCG-3' (SEQ ID NO: 60). Of note, the control vector (anti-NT) was the same plasmid used for anti-7oxo (α-MHC 13x) except that it contained the binding site of a non-targeting siRNA (NT) derived from cel-miR-67 (C. elegans-). The pJG/ALPHA MHC vector was provided by Jeffrey Robbins (Addgene plasmid #55594) to Dr. Da-Zhi Wang. Other competitive miRNA inhibitors were synthesized from RNA (see RNA Synthesis Methods).

### Example 32. Administration of miRNA inhibitors to ISO-treated Mice

To investigate whether a competitive miR-1:7o⁸G inhibitor (anti-7oxo) could attenuate ISO-induced cardiac hypertrophy in vivo, ISO (75 mg kg⁻¹) was initially administered via intraperitoneal injection (I.P) to 8 to 12 week old male C57BL/6J mice (Korea Bio Co. LTD), and at this time, an equal volume of PBS was used as a control (n=5 for each set). After 8 h, mice were intravenously injected with anti-7oxo or control (anti-NT) using in vivo jetPEI (Polyplus) in the amounts and ratios specified according to the manufacturer's protocol; anit-7oxo (α-MHC 13x) or cont (anti-NT 13x), 1.9 mg kg⁻¹, N/P ratio=8; anti-7oxo (4x) or cont (anti-NT 4x), 5 mg kg⁻¹, N/P ratio=5. Serial injections were performed three times on days 1, 2, and 5, and all mice were sacrificed on day 7 to examine the hearts. RT-qPCR of the anti-7oxo transcript including hGH poly (A) (forward: 5'-TAAATTCCAAATTCCAGAAATTCCACAAATTCCAT-3' (SEQ ID NO: 61), reverse: 5'-CCAGCTTGGTTCCCAATAGA-3' (SEQ ID NO: 62)) was performed, and thereby the delivery rate of anti-7oxo (α-MHC 13x) to cardiac tissue was measured. To confirm the delivery of anti-7oxo (4x) to the mouse heart, a poly (A) tailing-based miRNA qPCR method (see method of quantitative PCR for miRNA) was performed with anti-7oxo (4x) (forward, 5'-TAAATTCCAAATTCCAGAAATTCCACAAATTCCAT-3'; SEQ ID NO: 63) specific primers.

### Example 33. Quantification of miR-1:o⁸G in ISO time course

To investigate the change in the amount of oxidized miR-1 in the ISO time course experiment, 75 mg kg⁻¹ ISO was administered to mice via IP injection, and injected mice were sacrificed on days 1, 5, and 7 from the time point of treatment (n=4 for each time point). An equal volume of PBS was also injected and mice were immediately sacrificed and used as samples on day 0 (n=4). After dissecting the heart, small RNA was extracted using a miRNeasy Mini Kit (Qiagen). To quantify miR-1:o⁸G, o⁸G IP and then miR-1 qPCR were performed as described in the "Quantification of oxidized miRNA by o⁸G IP and qPCR" method, but with some modifications as follows. In bead preparation, 3 µg of anti-o⁸G antibody (15A3, QED Bioscience), 30 µl of Dynabeads Protein G (Invitrogen), and 300 µg of deoxyguanosine (dG, Sigma-Aldrich) in 150 µl of PXL was used; and in IP culture, 2 µg of small RNA sample and 1 pg of o⁸dG RNA spike-in (miR-124-3p:4o⁸dG: 5'p-UAAGo^{B}dGCACGCGGUGAAUGCC-3'; SEQ ID NO: 64) in 250 µl of PXL containing 2.5 mM DFOM and 40U recombinant RNase inhibitor (Takara) was used.

### Example 34. Sequence Conservation Analysis of miR-1 Target Site in 3'UTR

When counting motifs of sequences conserved in the 3'UTR, PhastCons results for mammals were obtained from the UCSC genome browser (http://genome.ucsc.edu), and were only used if the score was greater than or equal to 0.9, and were calculated as a conservation rate (number of motifs conserved /number of all motifs; %). The conservation rates at all 3'UTRs (as defined by RefSeq) were calculated with the seed sites of the miRNA family (n=103, 6mer at positions 2 to 8) conserved for four different sites of miR-1 (seed, 2oxo, 3oxo, and 7oxo sites). Sequences were conserved in most of these mammals (but generally not beyond placental mammals; http://www.targetscan.org). As a background control, conservation of all 6-mers (n=4098) was also calculated. The resulting distributions were expressed as cumulative fractions and proportions of the population.

### Example 35. Data Availability

All raw sequencing data from o⁸G-miSeq (SRP189806, SRP189807, SRP189808, SRP226125), RNA-Seq (SRP189813, SRP189117, SRP189812, SRP189811, SRP189809, SRP213998, SRP214400, SRP228274), and CLEAR-CLIP (SRP189810) are stored in Sequence Read Archives. All FASTQ files including sequencing data of o⁸G IP with spike-in were also provided on the project website (http://clip.korea.ac.kr/oxog/).

### Example 36. Measurement of miR-1:o⁸G in Plasma of Animal Model of Cardiac Hypertrophy

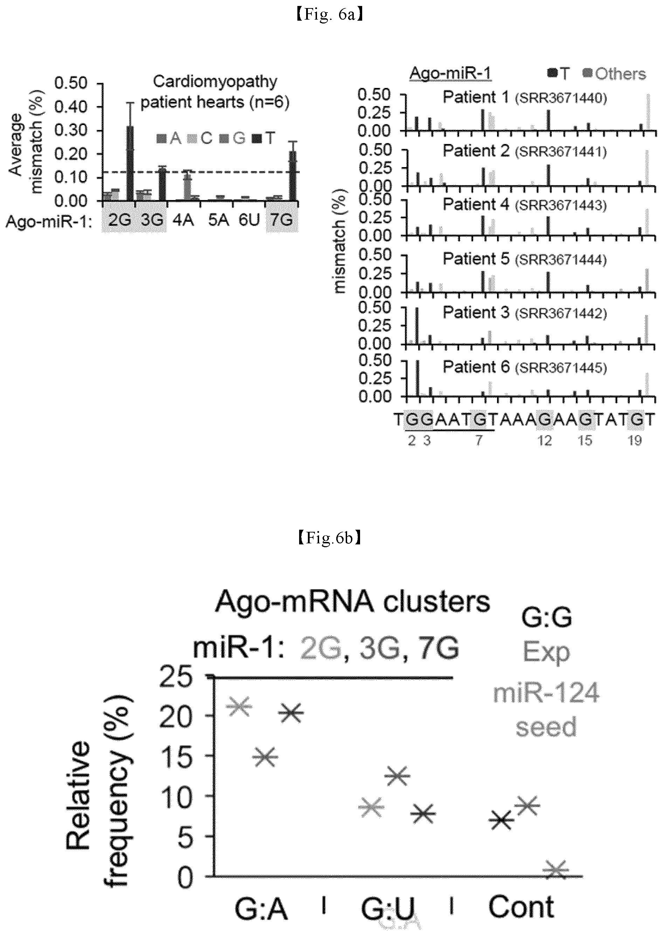

Cardiac hypertrophy in mice was induced through ISO injection in the same manner as described in Example 8 (FIG. 6a). After that, 700 µl of blood was collected from each of 3 control animals treated with PBS and 3 animals treated with ISO, and then plasma was isolated by sedimentation at 4 degrees, 1000x g, and centrifugation for 5 minutes. Small RNA was extracted using Qiagen's miRNeasy Serum/Plasma Kit from the same amount of plasma of 350 ul for each group, and the measurement of miR-1 in the extracted small RNA was quantified by correction with an amount of U6 by performing qPCR as described in Example 12. In addition, o⁸G-modified RNA was specifically isolated from 100 ng of small RNA isolated from plasma through antibody precipitation (IP) as described in Example 11, and miR-1 was quantified through qPCR. In addition, for the quantification of miR-1 modified with o⁸G, 1 pg of miR-124-3p:4o⁸dG (UAAGo⁸dGCACGCGGUGAAUGCC-3'; SEQ ID NO: 64), in which o⁸G was synthesized at 5th in a DNA form, was added in the immunoprecipitation experiment using the ⁸G antibody, measured by qPCR, and corrected.

### Experimental Example 1. miRNA Oxidation in ROS-dependent Cardiac Hypertrophy

The H9c2 rat cardiomyocyte cell line was treated with an α-adrenergic receptor (AR) agonist (phenylephrine; PE), and pathophysiological hypertrophy stimulation by PE treatment, ROS generation, and miRNA oxidation were confirmed. As a result of analysis by flow cytometry (10,000 cells, n=3) using ROS fluorescent dye (DHE), it was confirmed that ROS was increased in cells after PE treatment. In particular, 93 % of hypertrophic cells generated after treatment with PE showed a 1.8-fold increase in ROS production after treatment. Serum deficiency, an established prerequisite for stimulating adrenergic hypertrophy, also enhances the expanded phenotype with high basal ROS levels.

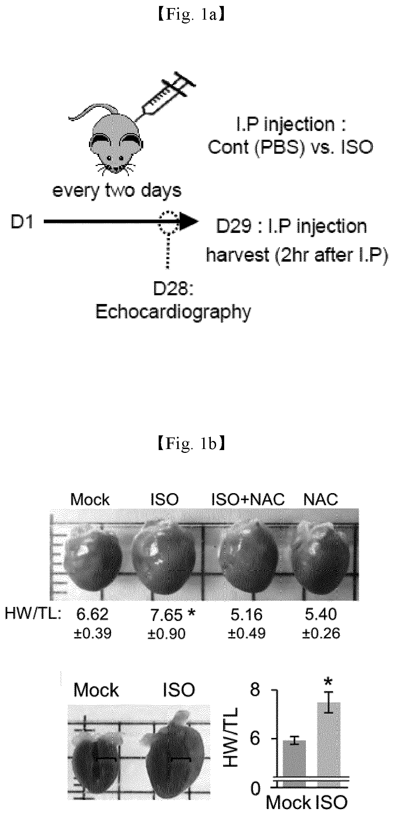

Extending further to the in vivo mouse model, chronic administration of the β-AR agonist, isoproterenol (ISO), shown in FIG. 1A, induces cardiac hypertrophy (about a 13 % increase), as shown in FIG. 1B. Among them, the pathology was confirmed by echocardiography, and it was confirmed that ROS was generated when treated with the ISO.

Next, it was investigated whether RNA was oxidized by ROS in the mouse model of cardiac hypertrophy, which was administered with the ISO.

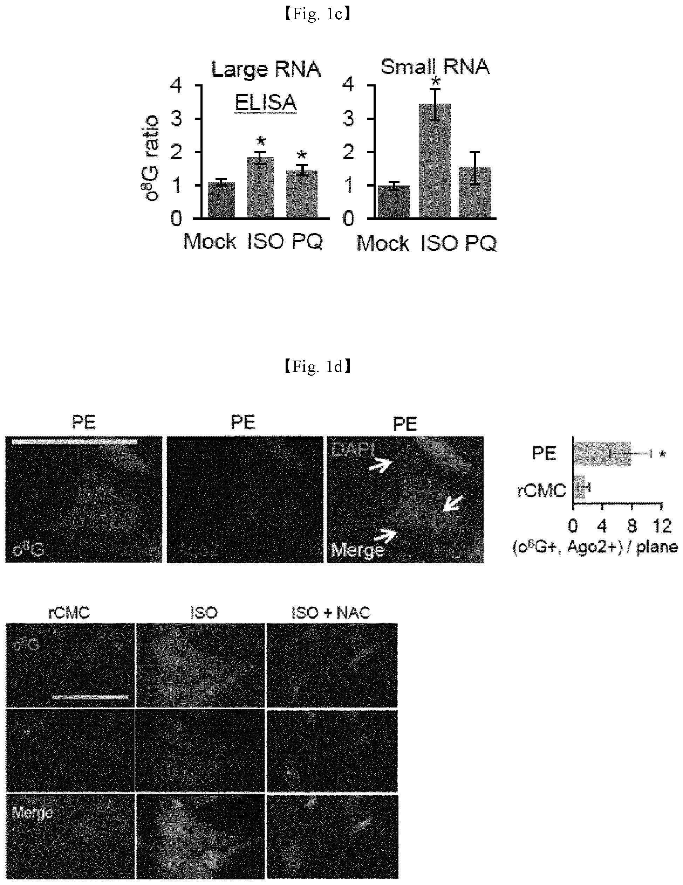

RNA was isolated according to size (based on 200 nucleotides), and 8-oxoguanine (o⁸G) induced by ISO treatment was measured using an o⁸G-specific antibody by ELISA. As a result of the measurement, it was confirmed that about 3.1 times more o⁸G was generated from small RNA than from large RNA, as shown in FIG. 1C. This was shown to be more rapid than large RNA (1.8 fold), even when large RNA was subjected to oxidative stress induced by paraquat (PQ) treatment.

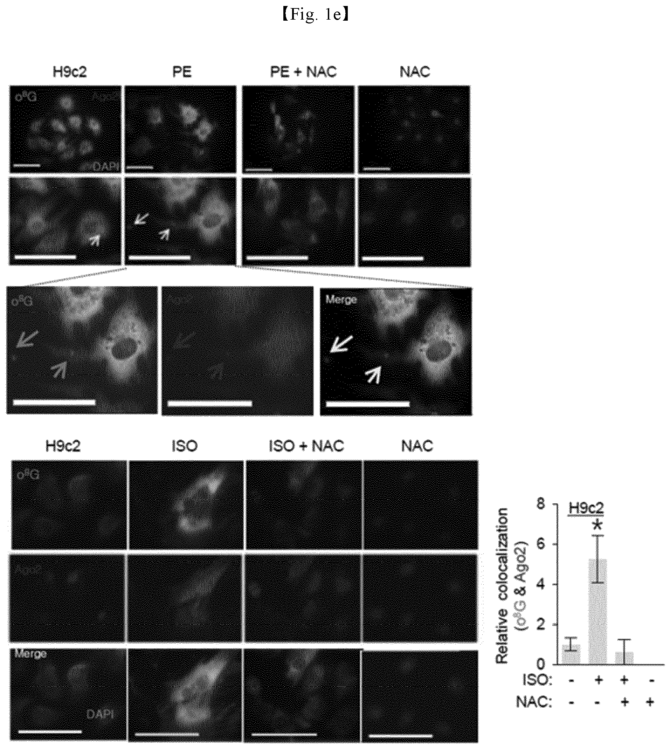

Oxidation of small RNA was further dissected into miRNA based on the colocalization of Argonaute2 (Ago2) and o⁸G, which are key proteins of the RNA-induced silencing complex, as shown in FIG. 1D was quantified and increased, it was quantified and increased in PE-treated rCMC (about 8-fold), and as shown in FIG. 1E, it was also increased in PE- or ISO-treated H9c2 (o⁸G+ per plane, Ago2; 100 cells, n=4; P=0.05).

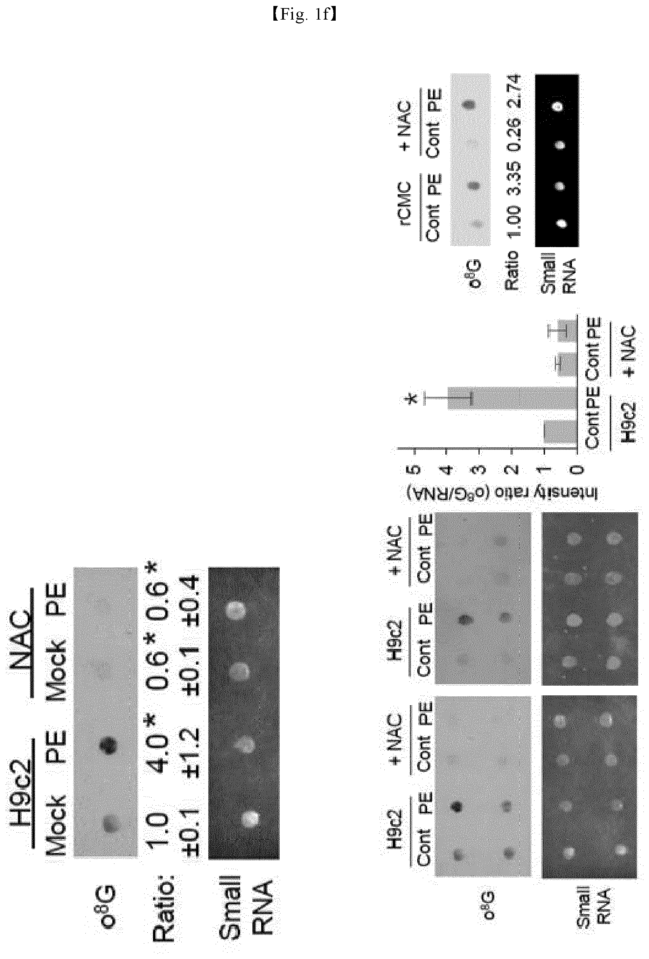

Independently, dot blot analysis using an o⁸G-specific antibody showed increased oxidation of small RNA (<200nt) from PE-treated H9c2 and rCMC in a redox-dependent manner, as shown in FIG. 1F.

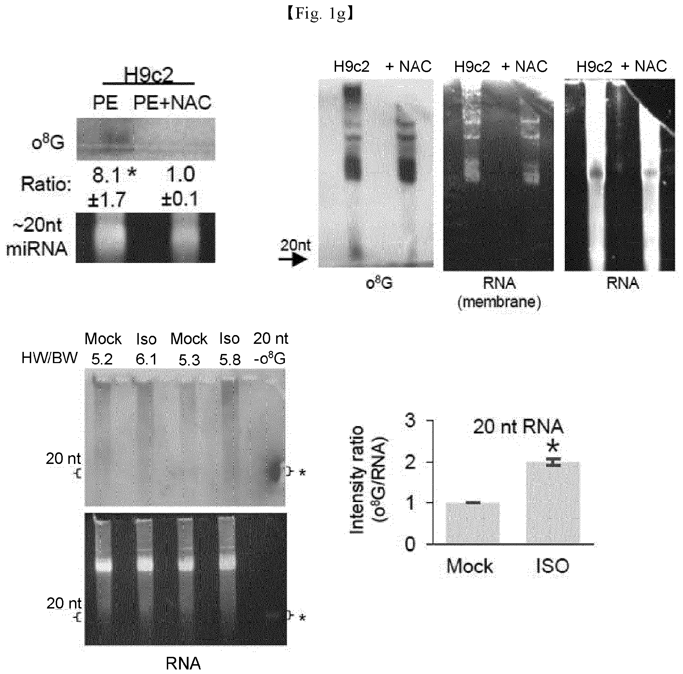

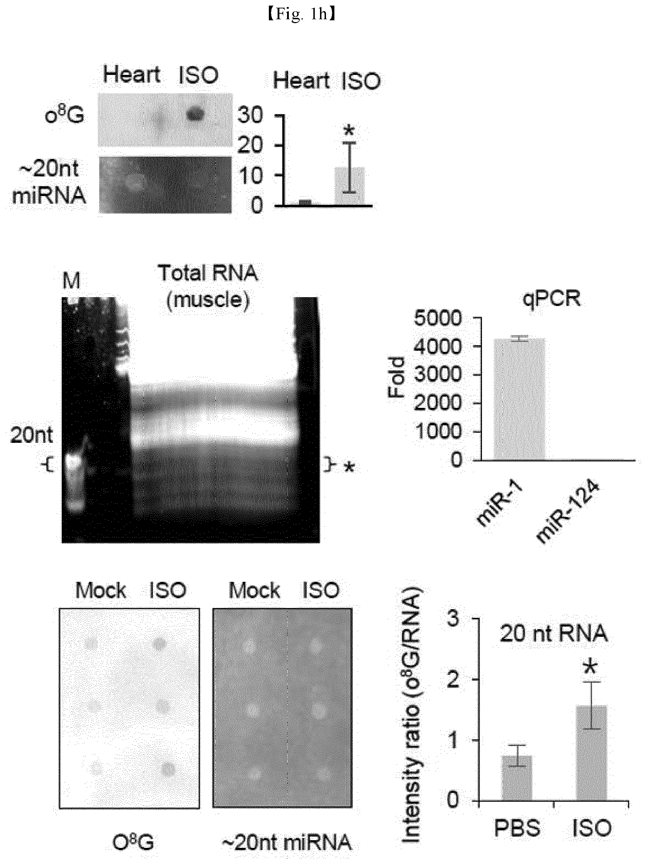

When the oxidation of miRNA was confirmed in PE-treated H9c2 and ISO-injected mouse hearts, as shown in FIG. 1G, through northwestern analysis results in PE-treated H9c2 (top of FIG. 1G) and ISO-injected mouse hearts (bottom of FIG. 1G), and the dot blot analysis of gel-extracted about 20nt miRNA shown in FIG. 1H, it was confirmed that o⁸G was generated from about 20nt miRNA by treatment with PE and ISO, respectively.

Summarizing the above experimental results, cardiac hypertrophy is redox-dependent and enables the generation of ROS, which induces o⁸G modification of miRNA.

### Experimental Example 2. Sequencing of Site-specific o⁸G in Cardiac miRNA

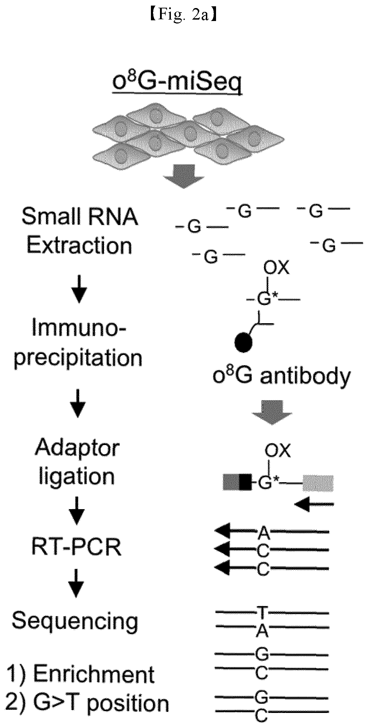

To identify oxidized miRNAs and corresponding o⁸G positions, a novel sequencing method (o⁸G-miSeq) for o⁸G in miRNAs was developed by optimizing immunoprecipitation (IP) of o⁸G and detecting o⁸G-induced G>T base conversion and a schematic diagram thereof is shown in FIG. 2A.

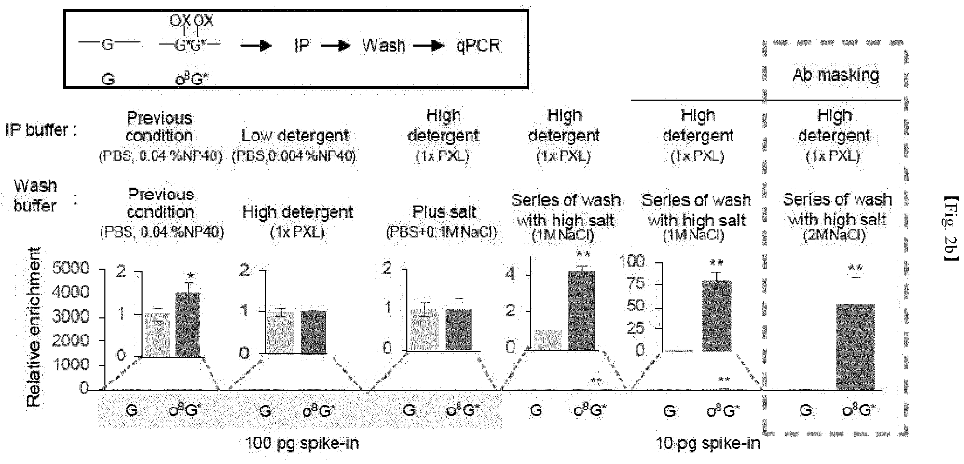

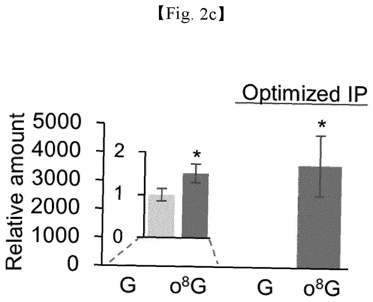

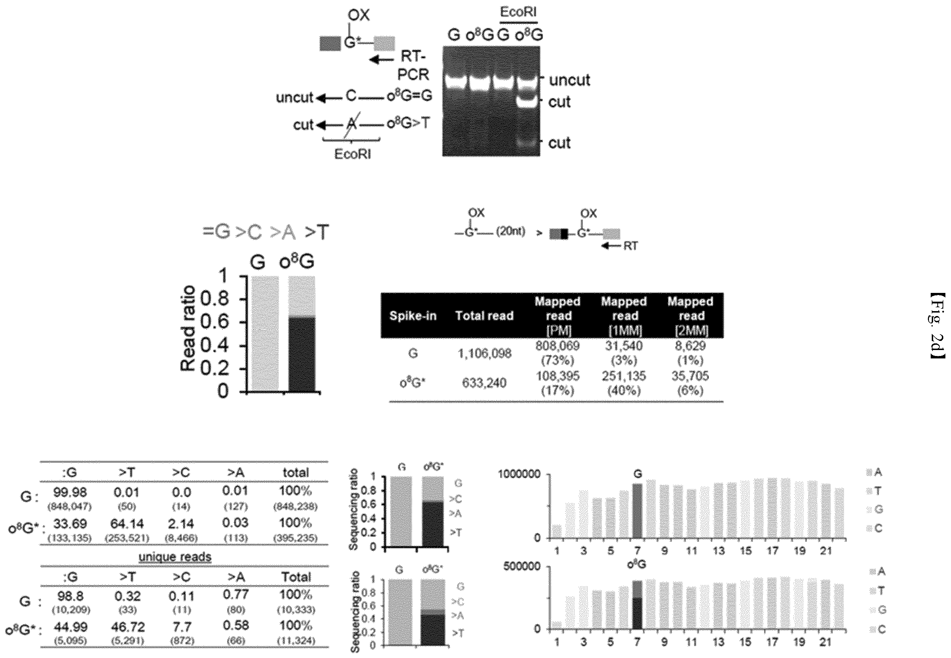

First, the IP process for o⁸G was extensively improved by adopting the conditions used for CLIP, as shown in FIG. 2B, and the optimized IP, as shown in FIG. 2C, showed an about 3000-fold amount of synthetic o⁸G compared to the non-specific background (non-oxidized G). Because o⁸G can pair with A, the efficacy of inducing G>T mutations in cDNA was enhanced to reach about 50 to 60 %, which was demonstrated indirectly by sequence-specific cleavage of the obtained restriction enzyme sites (top of FIG. 2D) and directly by sequencing (middle and bottom of FIG. 2D).

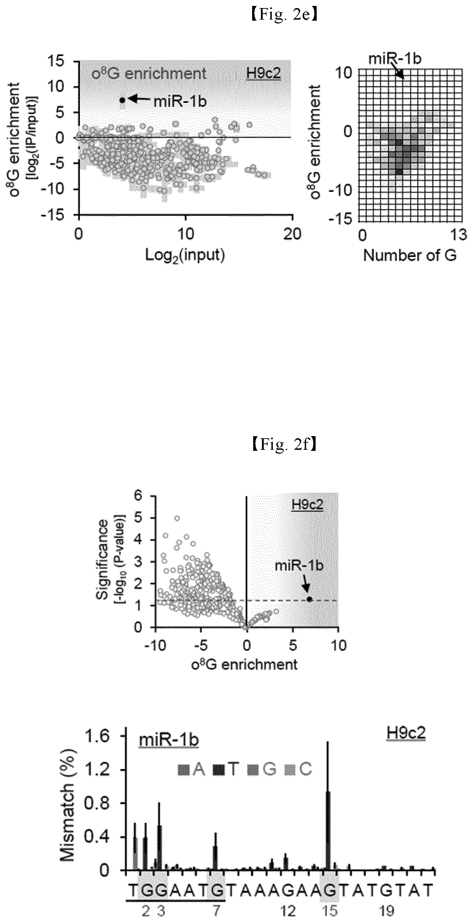

Then, as shown in Table 1, o⁸G-miSeq was initially applied to H9c2, miR-1b was identified as the most oxidized miRNA with respect to its basal expression (o⁸G enrichment, log2 (IP/input)=7; normalized by miRNA-Seq), the o⁸G enrichment, that is, the log ratio of o⁸G IP normalized to the input read count for miRNA, was indicated by dots according to the miRNA frequency and is shown in the left diagram of FIG. 2E, and the number of Gs in the sequence as heat map density is shown in the right diagram of FIG. 2E.

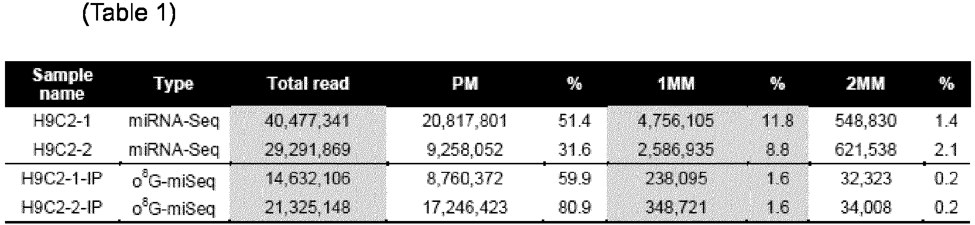

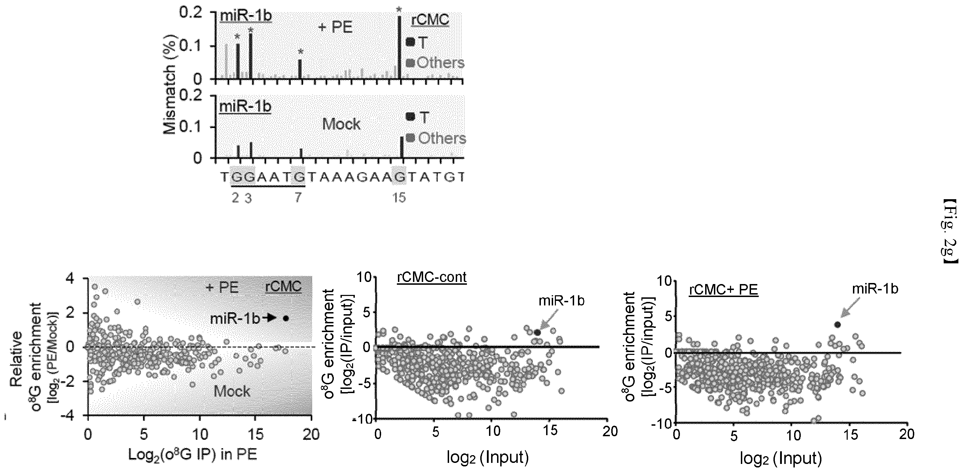

IP was demonstrated to be performed specifically by not observing any bias from the amount and G-content of miRNA, as in FIG. 2F, which showed significance (-log₁₀(P-value)) as a Volcano plot, o⁸G in miR-1b was significantly high (P <0.01), and the seed region (positions 2, 3, and 7) based on the G>T mutation rate were found to be significant. In addition, by additional application to rCMC as shown in Table 2, as shown in FIG. 2G, o8G-miSeq further indicated that miR-1b was preferentially oxidized following PE treatment with an increase in o⁸G (top of FIG. 2G) at the identified positions (positions 2, 3, and 7) of the seed region (relative o⁸G enrichment, log₂(PE/Mock)=1.66; bottom of FIG. 2G).

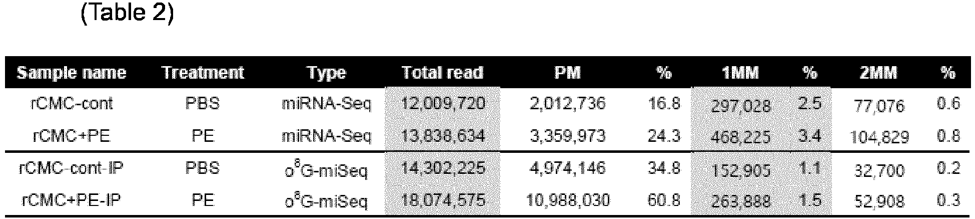

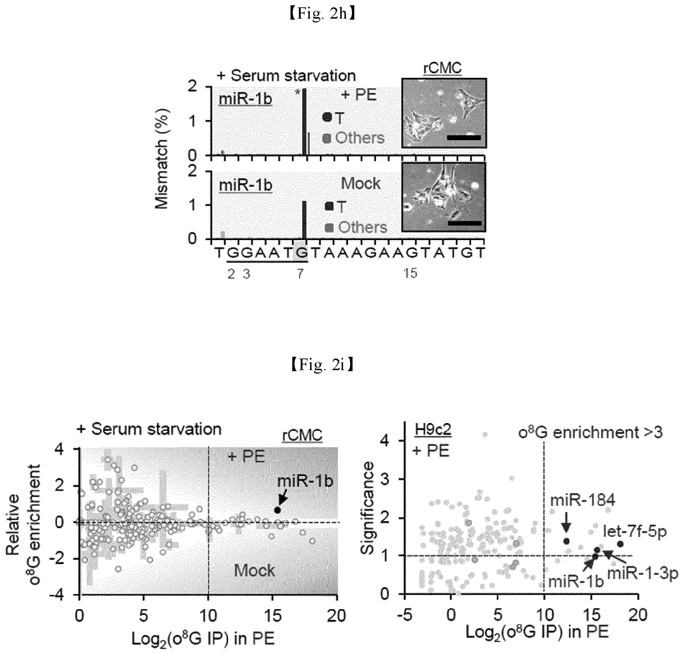

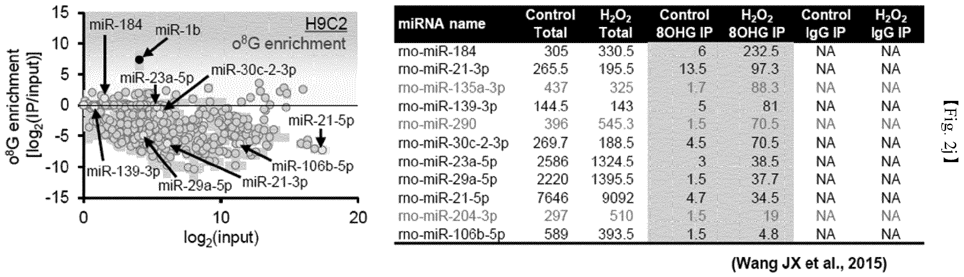

Then, in order to exacerbate the hypertrophic phenotype of rCMC, PE treatment for o⁸G-miSeq analysis was performed after exposing rCMC to serum deficiency as shown in Table 3. As a result, o⁸G at position 7 increased dramatically (miR-1:7o⁸G, about a 2-fold increase), as estimated by the G>T conversion rate in the miR-1 sequence as shown in FIG. 2H. In addition to observing enhanced miR-1b oxidation as shown in FIG. 2I and Table 4, significant oxidation was also observed for other miRNAs such as miR-184, let-7f-5p, and miR-1-3p (P<0.01) with significant amounts of o⁸G (log₂(o⁸G-IP)>10). In particular, miR-184 was confirmed to be the same as the previous H₂O₂ treatment result as shown in FIG. 2J, and other miRNAs had some inconsistencies.

Overall, by using o⁸G-miSeq, oxidized miR-1 and its specific o⁸G localization during cardiac hypertrophy may be accurately detected.

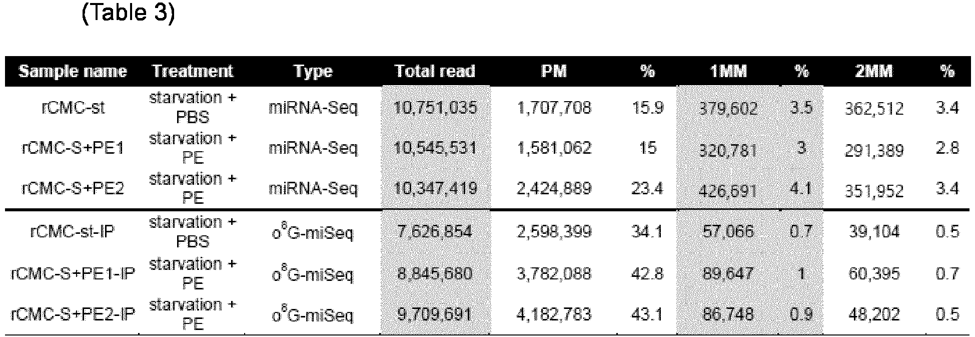

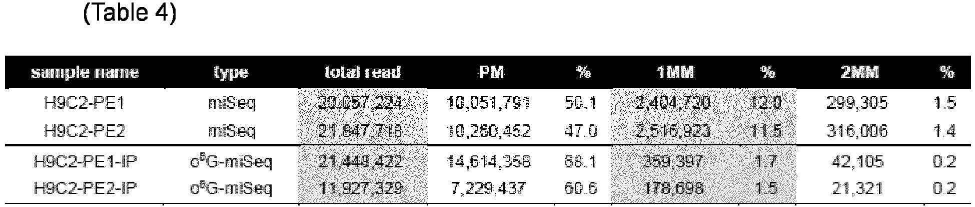

### Experimental Example 3. Target Silencing Effect through o^{B}G:A Base Pairing of Oxidized miR-1

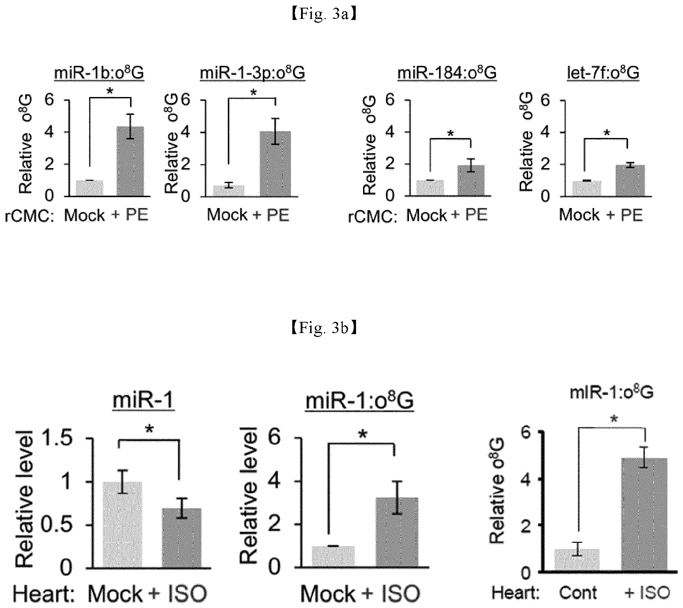

As confirmed in Experimental Example 2, oxidized miRNA (miR-1b, miR-1-3p, miR-184, and let-7f), as shown in FIG. 3A, through o⁸G IP (miRNA:o⁸G) according to PE treatment, a significant increase in their amount was observed and verified in rCMC (P<0.05, t-test). Among them, miR-1 showed the most dramatic improvement (about 2.5-5 fold), and it was also confirmed in ISO-treated hypertrophic mouse heart despite down-regulation after ISO treatment, as shown in FIG. 3B.

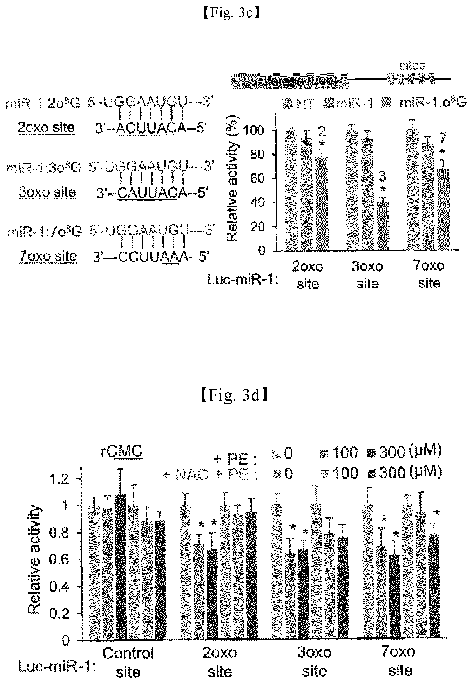

It was investigated that centering on miR-1, whether, at the o⁸G positions (positions 2, 3, and 7) identified in the seed region, miR-1 (miR-1:2o⁸G, miR-1:3o⁸G, and miR-1:7o⁸G) can recognize the corresponding new target sites (2oxo, 3oxo, and 7oxo sites) through o⁸G:A base binding, and the results are shown in FIG. 3C.

By performing the luciferase reporter analysis, it was confirmed that the synthesized miR-1:2o⁸G, miR-1:3o⁸G, and miR-1:7o⁸G could silence a target having oxo sites (2oxo, 3oxo, and 7oxo sites) recognizable as a G;A arrangement during the corresponding oxidative modification, which was not inhibited by miR-1.

Indeed, as shown in FIG. 3D, these luciferase reporters with miR-1 oxo sites were all inhibited in PE-treated rCMC but activated in the presence of the antioxidant NAC, which suggests levels of endogenous miR-1:o⁸G generated by adrenergic stimulation of rCMCs sufficient to alter target recognition and perform silencing.

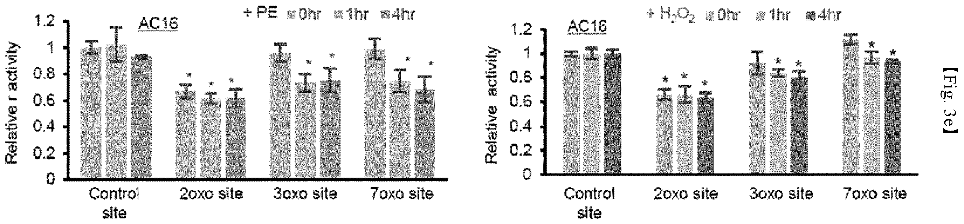

Also, consistent results were observed in AC16 immediately after treatment with PE or H₂O₂, as shown in FIG. 3E.

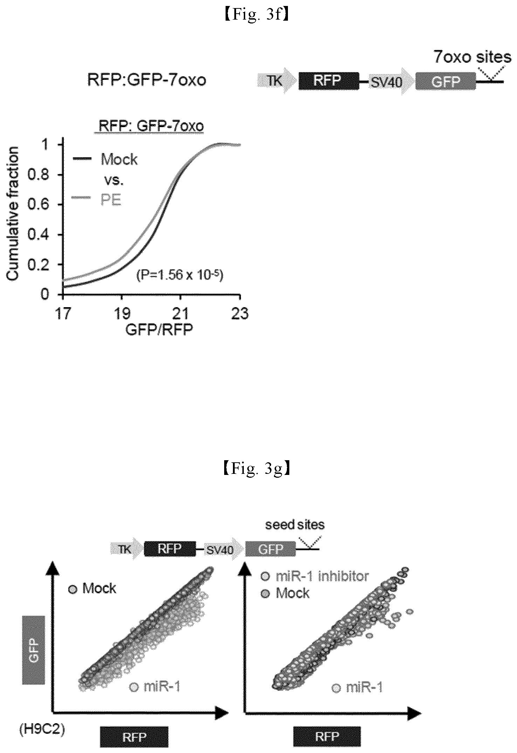

Next, to exclude synthetic effects from the heterogeneity of the cell population, flow cytometry was performed with a dual fluorescent protein (dFP) reporter as shown in FIG. 3F: a green fluorescent protein gene (GFP) with miRNA target sites and a red fluorescent protein gene (RFP) without the sites. The dFP reporter was observed by analysis (P=1.56 × 10⁻⁵, Kolmogorov-Smirnov test (KS test), GFP/RFP) of the cumulative fraction of relative activity in hypertrophic H9c2.

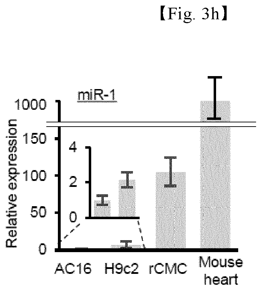

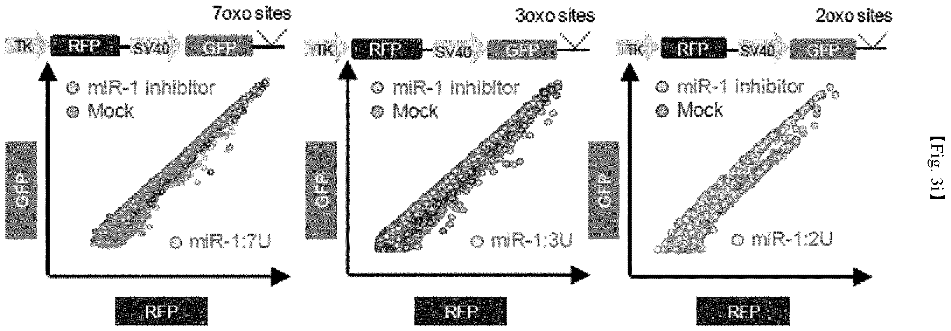

Despite the low expression level of endogenous miR-1 (probably caused by the fate heterogeneity of the cardiomyocyte line shown in FIG. 3H), miR-1:o^{B}G-dependent inhibition was detected at the level of individual H9c2 cells, as shown in FIG. 3G. It was confirmed in FIG. 3I that all miR-1 oxo sites (7oxo, 3oxo, and 2oxo sites) of the dFP reporter were consistent with the miR-1:o⁸G position (2, 3, and 7) observed by o⁸G-miSeq (FIG. 2F) and was endogenously inhibited with sensitivity to detect inhibition mediated by the basal level of miR-1:o⁸G and it was identified by transfecting miR-1 inhibitors or cognate miR-1 variants.

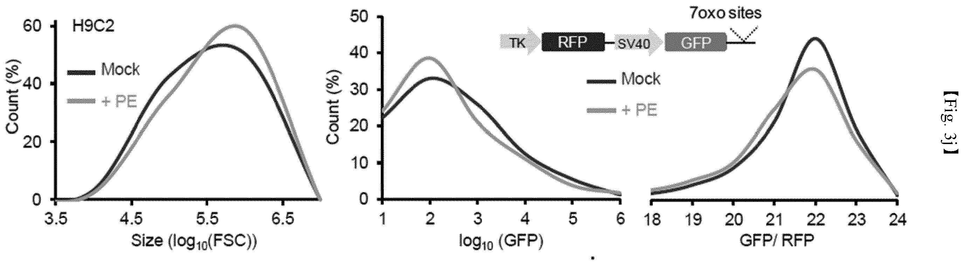

In addition, the dFP reporter detected significant PE-dependent inhibition of miR-1 7oxo sites in hypertrophic H9c2 as shown in FIG. 3J.

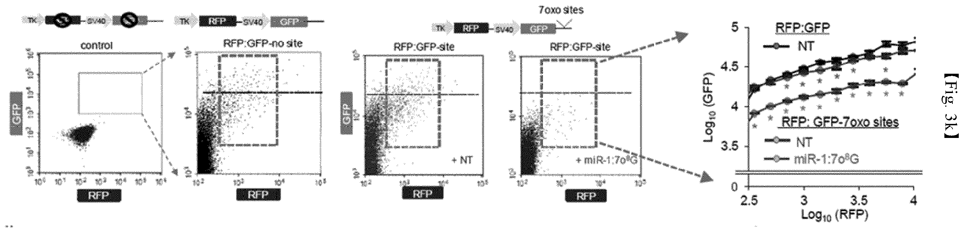

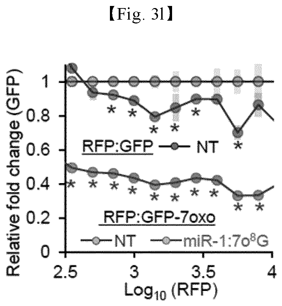

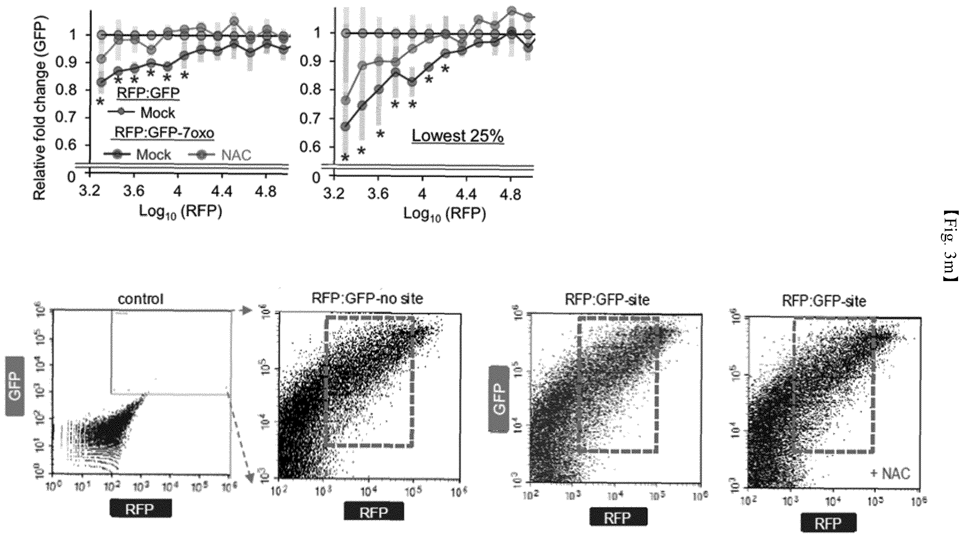

Moreover, as the sensitivity from full excitation of both fluorescent proteins increases, the values compared to the dFP reporter (RFP:GFP, NT) having no sites (RFP:GFP, NT) as shown in FIG. 3K were compared, and thereby the assay was scrutinized by validating the inhibition of miR-1 by endogenous levels of miR-1:7o⁸G (RFP:GFP-7oxo vs. RFP:GFP, NT) shown in FIG. 3L and its activation (RFP:GFP-7oxo, mock vs. NAC) by NAC treatment shown in FIG. 3M.

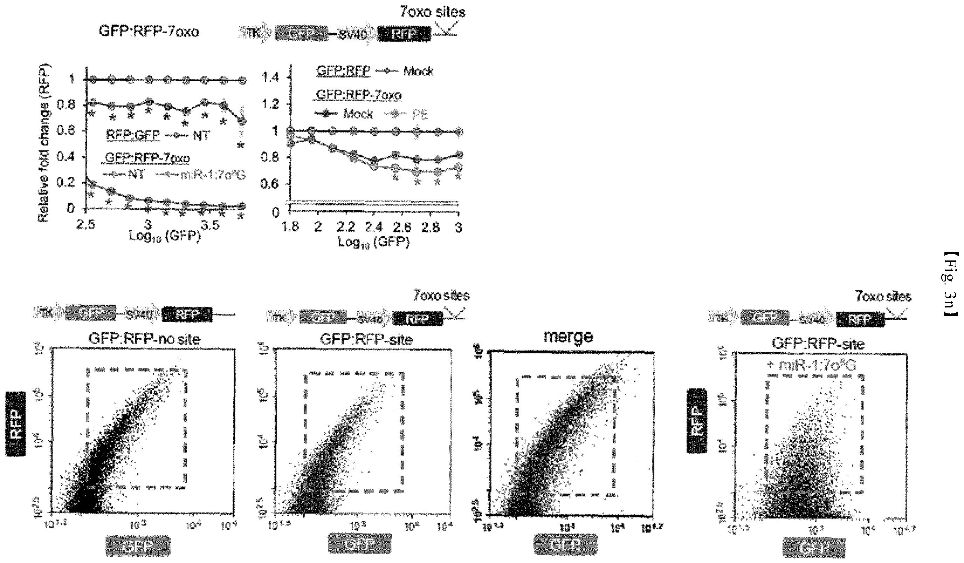

Importantly, it was considered that this relative inhibition, calculated by averaging the reporter fluorescence values (GFP-7oxo) over a similar range of control fluorescence values (RFP) in cells, was pronounced at the lowest 25 % of the reporter fluorescence values (GFP-7oxo), which suggested the presence of a cell population with high levels of miR-1:7o⁸G. In addition, as shown in FIG. 3N, the dFP reporter, in which the reporter and control fluorescent protein were exchanged, sensitively detected the inhibition of miR-1 7oxo sites by endogenous levels of miR-1:7o⁸G and PE-dependent increases in inhibition.

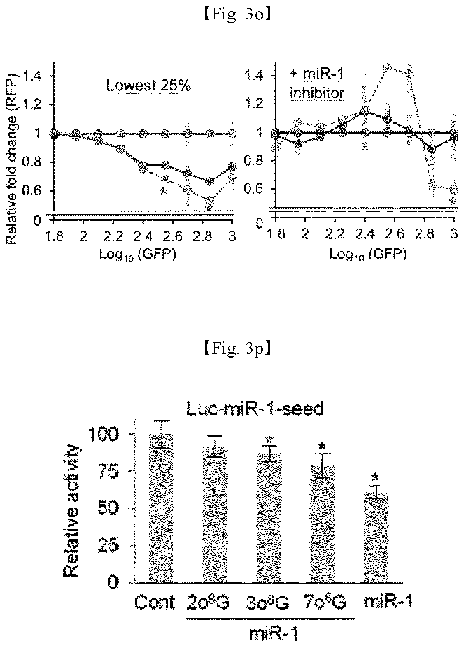

In addition, using the switched dFP reporter as shown in FIG. 3O, it was observed that PE-induced inhibition of miR-1 7oxo sites was more substantial when considering only the limited cell population with the lowest reporter value (RFP) of 25 %, and the observation of reporter activity (RFP) restored by introducing an miR-1 inhibitor further confirmed that PE-dependent inhibition of the miR-1 7oxo site was mediated by miR-1. In particular, as shown in FIG. 3P, the introduction of oxidized miR-1 (2o⁸G, 3o⁸G, and 7o⁸G) was less potent than unoxidized miR-1, but it might inhibit the seed site in the luciferase reporter because of the retained activity of o⁸G:C base binding. The miR-1:7o⁸G was observed to silence target mRNA via o⁸G:A base binding obtained in adrenergic cardiac hypertrophy.

### Experimental Example 4. Confirmation of Cardiac Hypertrophy Induction of miR-1:o⁸G

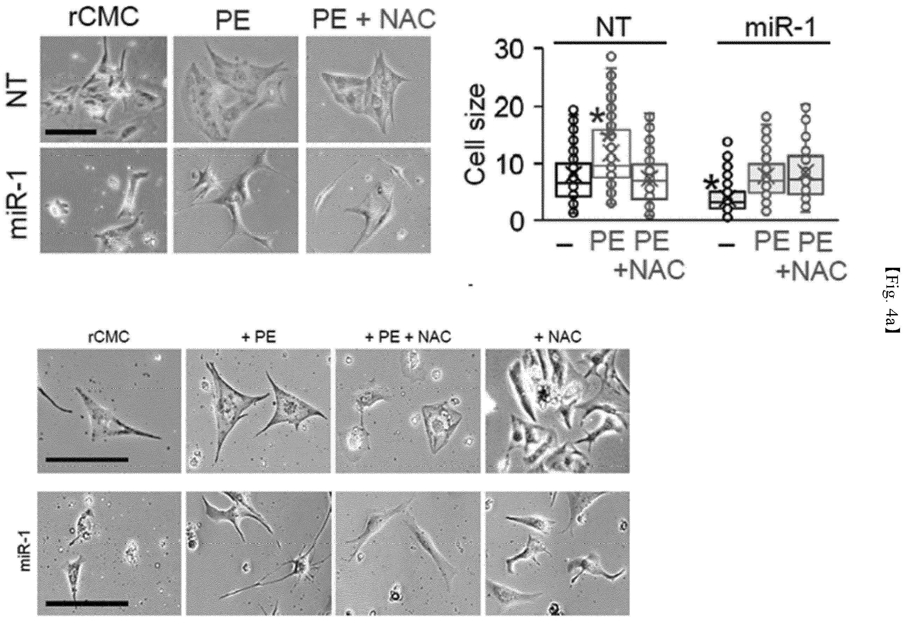

Although miR-1 was reported to have a negative function in hypertrophy, PE treatment reduced miR-1-induced atrophy of rCMC, as shown in the microscopic observation and cell size quantification results of rCMC cells shown in FIG. 4A. Cell size (inch², n=100) was quantified using ImageJ.

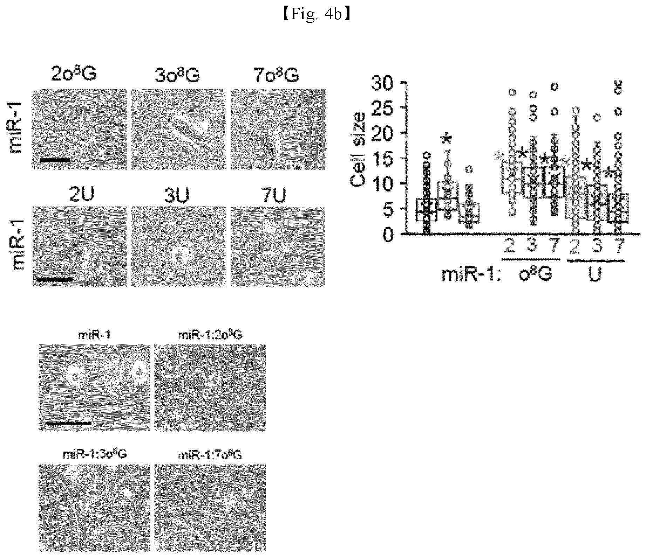

In Experimental Example 2, synthetic miR-1:2o⁸G, miR-1:3o⁸G, or miR-1:7o⁸G sustained by the discovery of miR-1:o⁸G and redox dependence of PE-induced hypertrophy was introduced, and as shown in FIG. 4B, it was observed that the hypertrophy of rCMC was induced substantially more than PE treatment.

These effects were equally shown when synthetic miR-1 (miR-1:2U, miR-1:3U, and miR-1:7U) substituted for o⁸G with U was introduced, and through this, the occurrence of hypertrophy depends on the o⁸G:A base binding.

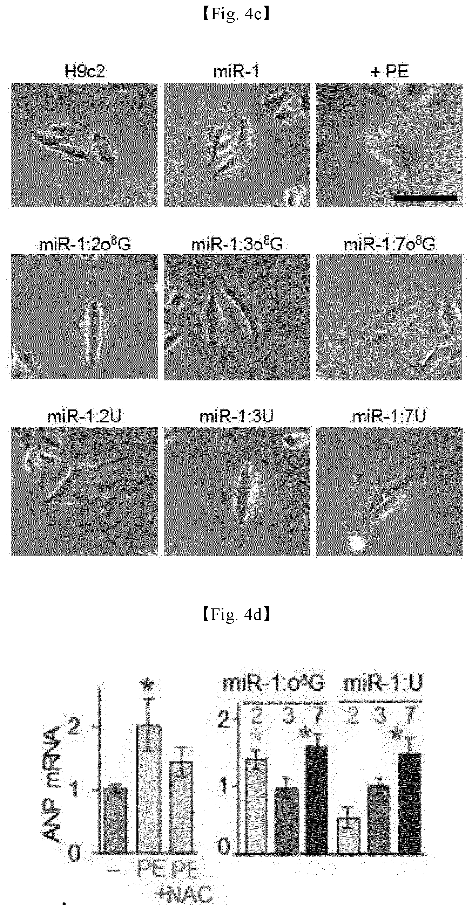

As shown in FIG. 4C, this effect was also observed in H9c2.

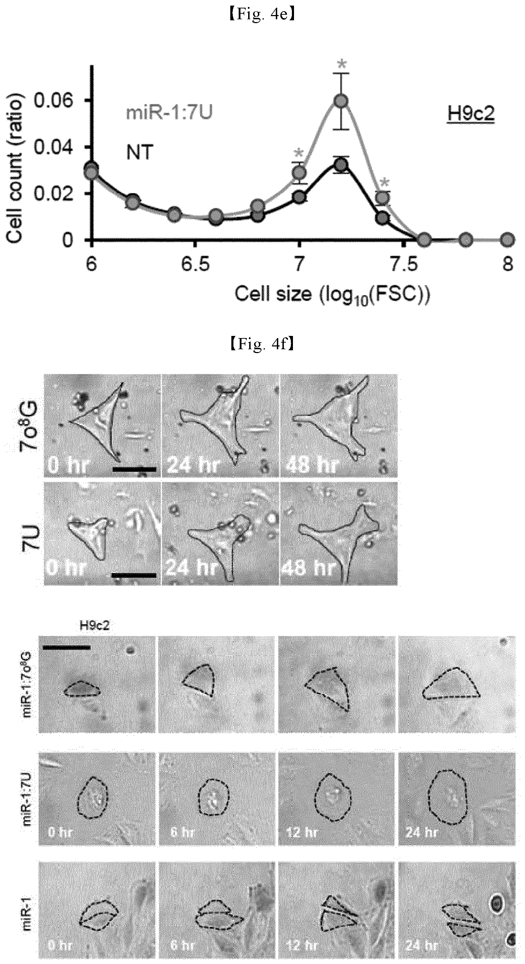

Specifically, the hypertrophy induced by miR-1:7o⁸G or miR-1:7U as shown in the qPCR measurement result shown in FIG. 4D significantly increased the expression of atrial natriuretic peptide (ANP), known as a marker of cardiac hypertrophy, as observed in PE treatment. These results were further confirmed by the flow cytometry analysis shown in FIG. 4E and time-lapse images of rCMC (top of FIG. 4F) and H9c2 (bottom of FIG. 4F).

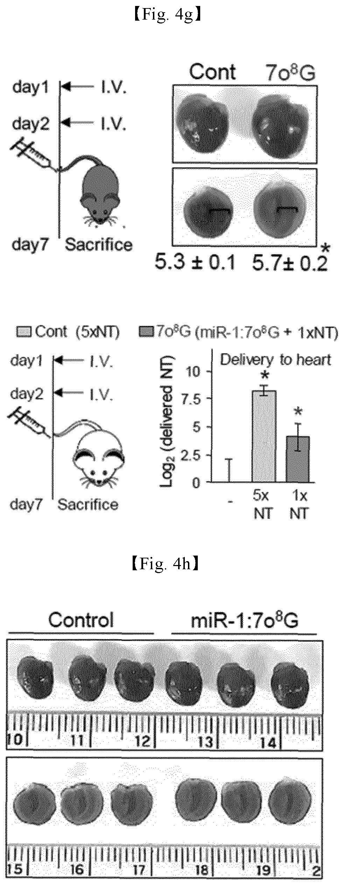

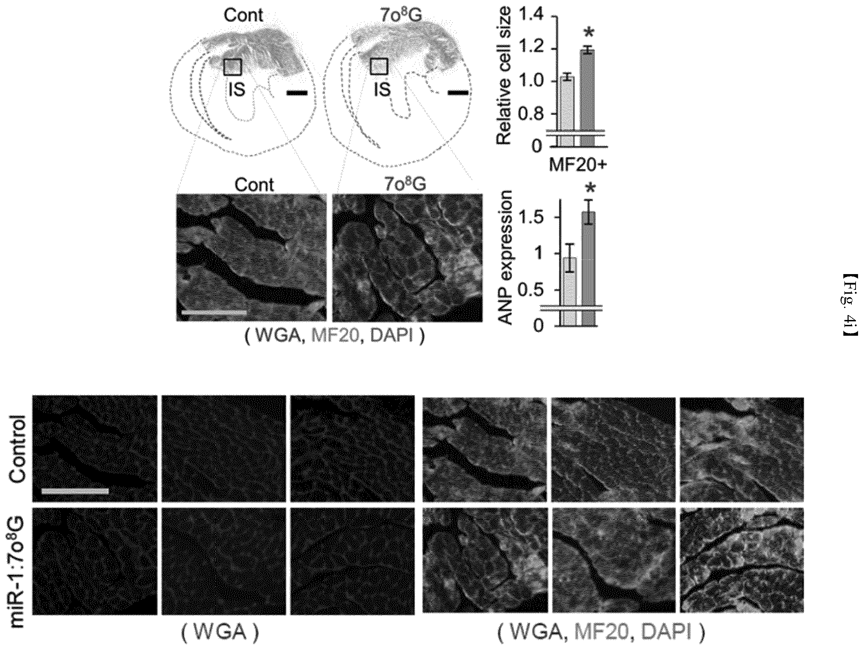

In addition, the effect of miR-1:7o⁸G on cardiac hypertrophy in vivo was tested. As shown in FIG. 4G, miR-1:7o⁸G was injected as a polyethylene imine (PEI) complex with non-targeting control (NT) via the tail vein (top of FIG. 4G), and delivery to the cardiac tissue by quantitative PCR (qPCR) was verified (bottom of FIG. 4G). As a result, at least about 10 % or more of the heart size increased significantly (P=0.001, n=3) as shown in FIG. 4H, and the interventricular septum (IS) was immunostained in H&E-stained cardiac tissue and the size of cardiomyocytes was quantified, and as a result, about a 19 % increase in cardiomyocyte size and significant upregulation of ANP expression were observed, as shown in FIG. 4I.

At this time, in FIG. 4J, WGA (wheat germ agglutinin) was used for cell boundary staining, MF20 was used for cardiomyocytes, and DAPI was used for nuclear staining.

To summarize the above experimental results, site-specific oxidation of miR-1, particularly miR-1:7o⁸G, can sufficiently induce cardiac hypertrophy in vivo through o⁸G:A base binding.

### Experimental Example 5. Discovery of Oxidized miR-122, let-7, and miR-124 and Acquisition of Functions Resulting Therefrom

In addition to myocardial hypertrophy, for other diseases known to cause oxidative stress, it was confirmed whether the o⁸G was modified at the seed region from the 5'-end to the 8th end of several microRNAs.

First, in Huh7, a liver cancer cell line derived from hepatocytes that specifically express a lot of miR-122, among tumors known to have increased free radicals, a luciferase reporter was constructed and it was confirmed whether the 2nd (2oxo), 3rd (3oxo), or the 2nd and 3rd of miR-122 guanine is o⁸G-modified (2oxo and 3oxo). At this time, the luciferase reporter experiment was performed using a psi-check2 (Promega) vector to include 5 target sites that can be recognized by binding to the o^{B}G:A arrangement at the seed region of miR-122, and it was measured after transfection into Huh7. In addition, in order to reveal that the inhibition of the corresponding site was directly caused by miR-122, a cell line (Huh7:miR-122 KO) in which the miR-122 gene was removed by gene editing using CRISPR/Cas9 in Huh7 was used with miRNA CRISPR knockout kits (Canopy, Bioscience Inc.) and tested together under the same conditions.

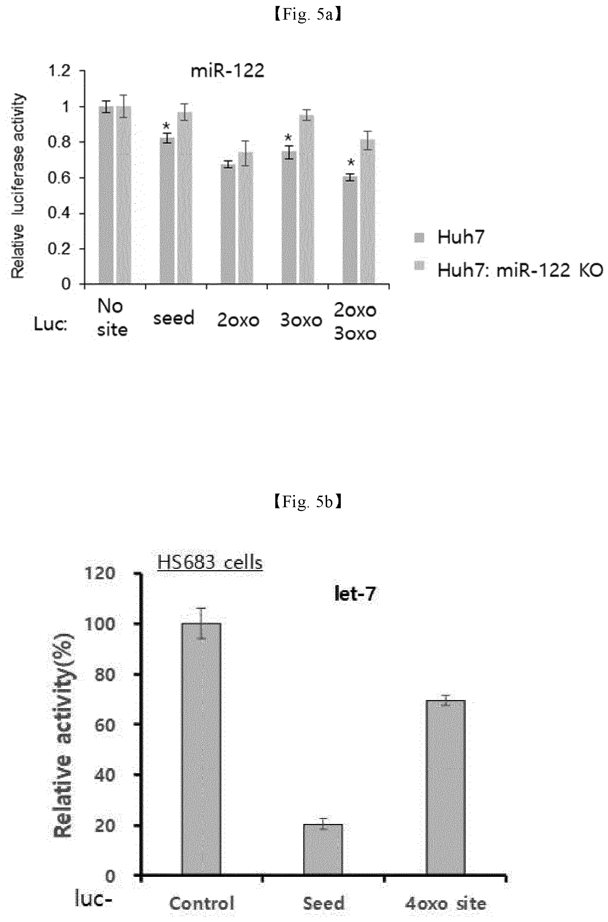

The results of the above experiments are shown in FIG. 5A.

As a result of the experiment, the seed site recognized by the existing miR-122 (seed: 5'-ACACUCCA-3') was not only inhibited, but also the 2nd o⁸G modification site (2oxo: 5'-ACACUCAA-3'), the 3rd o⁸G modification site (3oxo: 5'-ACACUACA-3'), and the 2nd and 3rd o⁸G simultaneous modification sites (2oxo, 3oxo: 5'-ACACUAAA-3') were inhibited.

In addition, in Huh7:miR-122 KO, it was observed that the inhibition of the 3rd o⁸G modification site (3oxo) and the 2nd and 3rd o⁸G simultaneous modification sites (2oxo, 3oxo) of miR-122 disappeared and it was proved that the corresponding inhibition was caused by the transformation of miR-122.

For let-7, a luciferase reporter for the 4th o⁸G site (4oxo: 5'-CUACAUCA-3') was prepared in the same way as before, and as a result of measurement in glioblastoma tumor HS683 using this, the inhibition was smaller than the seed site of let-7 (seed: 5'-CUACCUCA-3'), but significant inhibition was shown compared to the control (P<0.01), and the results are shown in FIG. 5B.

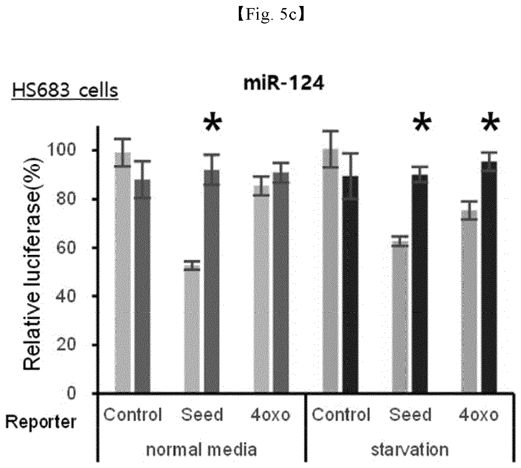

In addition, in order to find out whether o⁸G modification was also generated in the seed region for miR-124, which is known to be highly expressed in neurons and glioblasts, a luciferase reporter experiment for the 4th o⁸G modification target site of miR-124 (4oxo: 5'-GUGCAUUA-3') was performed in glioblastoma HS683 cells and the results are shown in FIG. 5C.

As a result of the experiment, the seed site of miR-124 (seed: 5'-GUGCCUUA-3') was inhibited, but inhibition of the 4oxo site was not observed. In the case of tumor cells, it is known that when nutrients or blood supply is insufficient, oxidative stress increases and intracellular oxidation increases. Therefore, in order to find out whether the o⁸G modification of miR-124 expressed in HS683 is increased in this situation, the experiment was conducted with serum removed from the cell culture medium. In this case, the 4oxo site of miR-124 was significantly (P<0.01) inhibited.

Through the above experimental results, it was confirmed that in miR-122 of liver cancer cells, the 2nd, 3rd, or 2nd and 3rd are o⁸G-modified together, in glioblastoma, the 4th base of let-7 was o⁸G-modified, and when miR-124 is subjected to oxidative stress that can occur in tumor cells, such as serum removal from a culture, o⁸G modification of the 4th base occurs to inhibit the expression of the newly recognized target gene.

In order to metastasize from liver cancer cells, the ability of liver cancer cells to migrate is preferentially required, and the expression of miR-122 is known to inhibit the migration of liver cancer cells. Since it was observed that the 2nd and 3rd of miR-122 were o⁸G-modified (miR-122:2,3⁸G) in Huh7, a hepatocarcinoma cell line, in order to examine whether the corresponding oxidized miR-122 affects the migration ability of liver cancer cells, miR-122:2,3o⁸G (5'p-Uo⁸Go⁸GAGUGUGACAAUGGUGUUUG-3', SEQ ID NO: 65) was synthesized through Trilink's RNA synthesis service, and the passenger strand (has-miR-122-5p) and the duplex were synthesized, a duplex with the passenger strand (has-miR-122-5p) was made and then was transfected into Huh7 to perform a wound-healing assay.

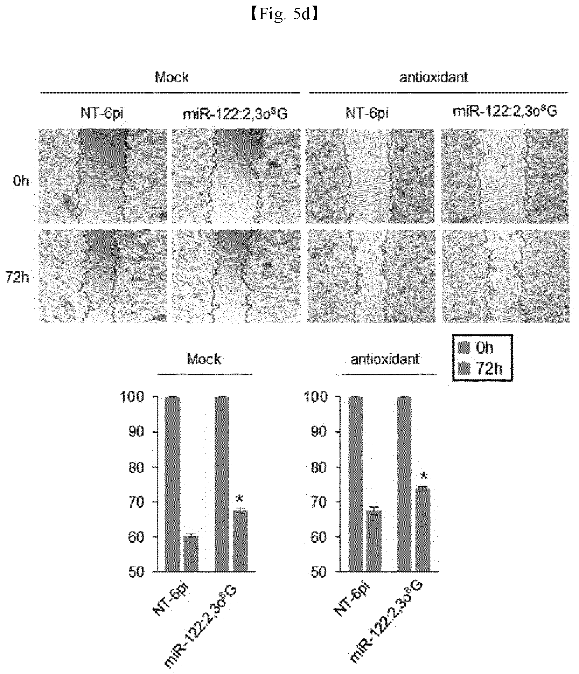

As a result of the experiment, as shown in FIG. 5D, when miR-122:2,3o⁸G was introduced into cells, it was confirmed that migration mobility of Huh7 was significantly inhibited compared to the control NT-6pi (all of the 6th base of cel-miR-67 was replaced with dSpacer).

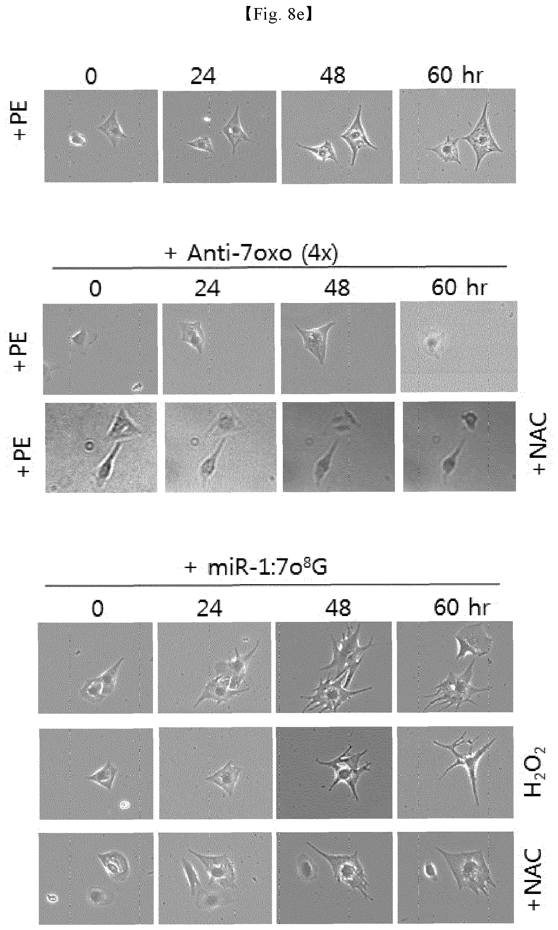

There is a possibility that in the miR-122:2,3o⁸G introduced into the cell in this way, other guanine might be additionally oxidized by the free radicals in the cell, so that its function might be somewhat reduced (see FIG. 8E). Therefore, it was confirmed that miR-122:2,3o⁸G exhibited greater inhibition of liver cancer cell migration when the same wound healing assay was performed after treatment with an antioxidant for cell culture (antioxidant supplement from Sigma-Aldrich). That is, the biological effect of the o⁸G-modified microRNA may be maximized by treating the cell with an antioxidant.

In addition, to investigate the function of let-7:4o⁸G, an o⁸G modify microRNA identified in glioblastoma, it was synthesized in the form of siRNA (let-7:4o⁸G: 5'-pUGAo⁸GUAGUAGGUUGUAUAGdTdT-3', SEQ ID NO: 66) and was introduced into HS683 cells in a duplex form. After introduction into HS683 cells, apoptosis was measured through Attune NxT flow cytometry using eBioscience Annexin V-FITC Apoptosis Detection Kit (Invitrogen).

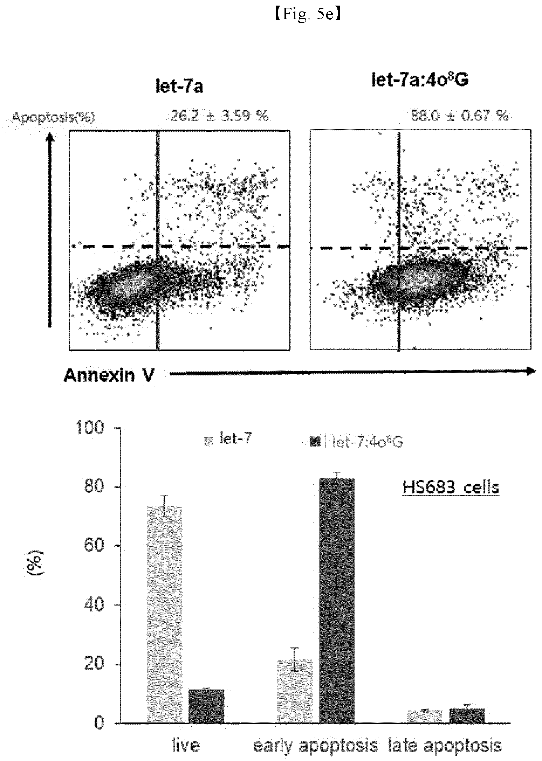

As a result of the experiment, it was confirmed that apoptosis of HS683 was significantly increased, as shown in FIG. 5E.

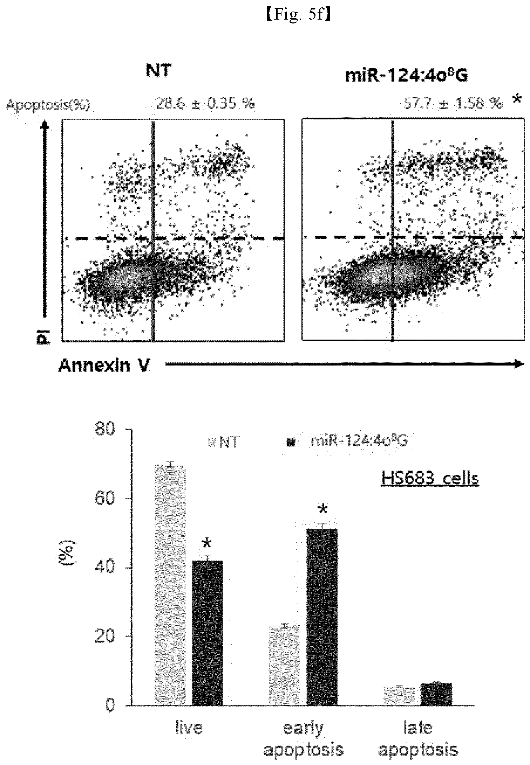

The same experiment as above was performed for the siRNA synthesis form of miR-124:4o⁸G (5'p- UAAo⁸GGCACGCGGUGAAUGCdTdT-3', SEQ ID NO: 67), and as shown in FIG. 5F, it was observed that apoptosis was induced.

Referring to the experimental results of Experimental Examples 1 to 5, the o⁸G modification occurring in the seed region (base up to the 8th base of the 5'-end) of several microRNAs can exhibit various pathophysiological functions when synthesized and introduced into the cell.

### Experimental Example 6. miR-1:7o⁸G and Loss of Function in Cardiomyopathy

The oxidation of miR-1 was investigated by analyzing the Ago HITS-CLIP results obtained from the left ventricle of the heart of a human cardiomyopathy patient (n=6, see Tables 5 to 7).

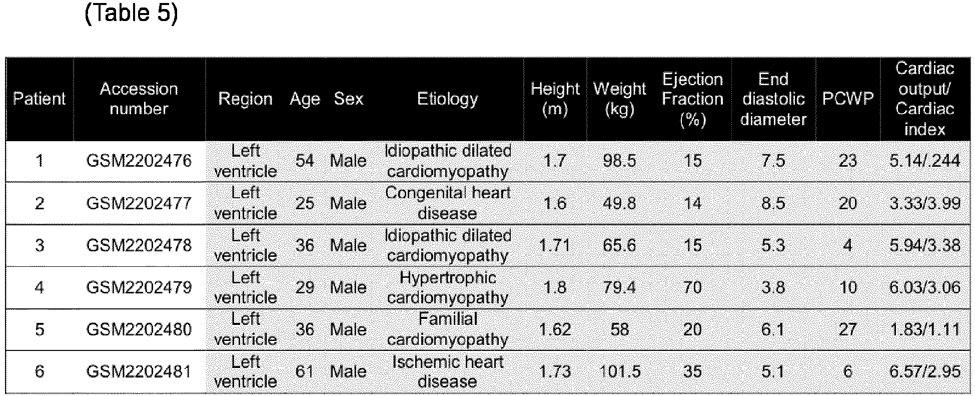

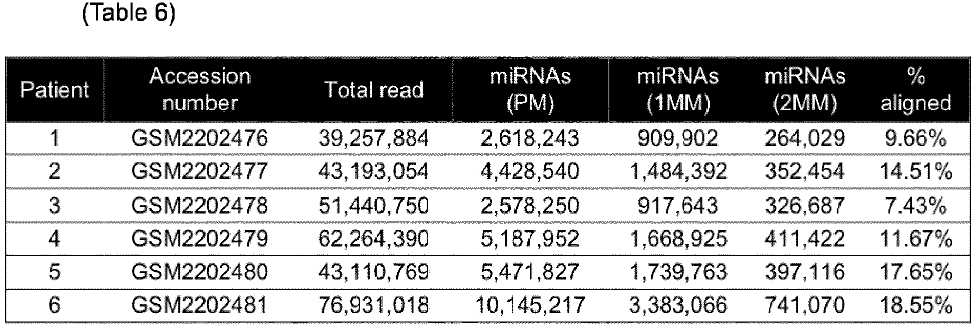

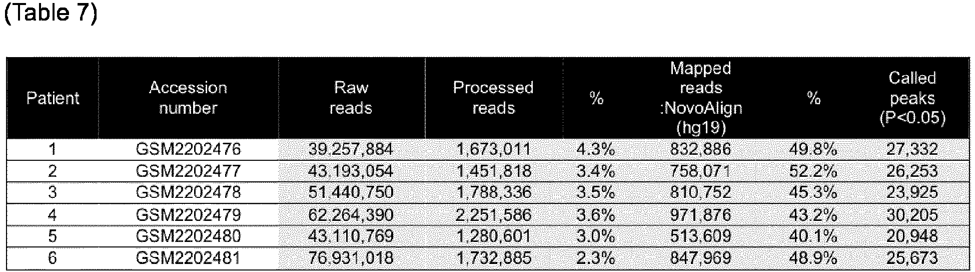

As a result, as shown in FIG. 6A, although the ratio was low, the same pattern of G>T mutations (positions 2, 3, and 7) were detected in Ago-related miR-1 (FIG. 6A, left diagram), and was clustered to include patient groups (1,2,4, and 5) with the highest frequency of position 7 as well as other smaller positions (positions 2, 3, and 12; n=4). The others (3 and 6) had the highest frequency exclusively at position 2 (n=2) ( FIG. 6A, right diagram). In the normalized Ago-mRNA cluster, the oxidized miR-1 target site represented by o⁸G:A binding through positions 2, 3, or 7 was observed significantly more than expected as shown in FIG. 6B (P<0.01, chi-square test), corresponding to an average of about 18 % that is more than G:U binding (about 10 %) and control sites (about 7 %).

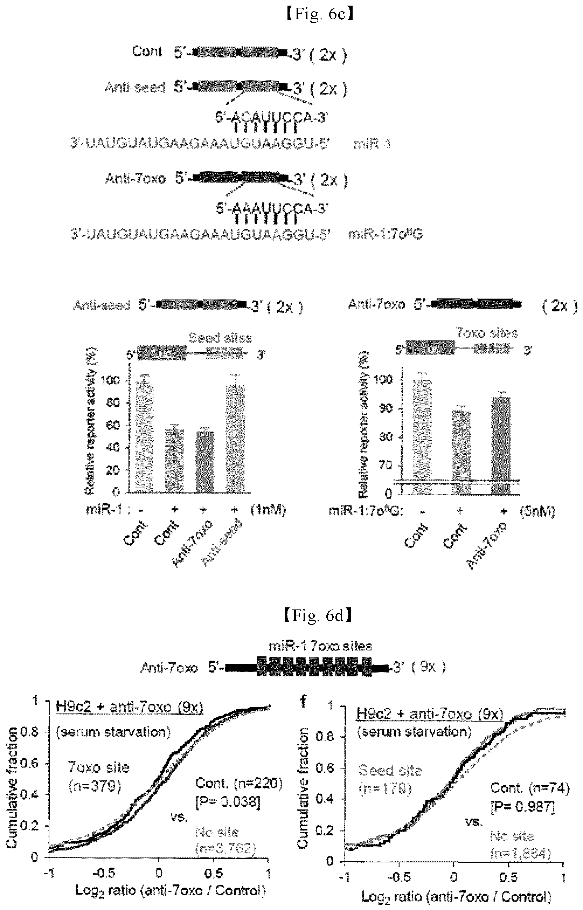

To further address the physiological relevance of miR-1:7o⁸G, loss of function was assessed. The concept from miRNA sponge, a competitive inhibitor synthesized as RNA was adopted so as to retain seed-mediated tandem repeats of target sites, and as shown in FIG. 6C, miR-1 (anti-seed) and miR-1:7o⁸G (anti-7oxo) was prepared (top of FIG. 6C) and their specific inhibition was verified (bottom of FIG. 6C).

The inhibitory activity of anti-7oxo (9x) was confirmed to completely inhibit the miR-1 7oxo target in serum-deficient H9c2 by performing RNA-Seq (see Table 8) as shown in FIG. 6D.

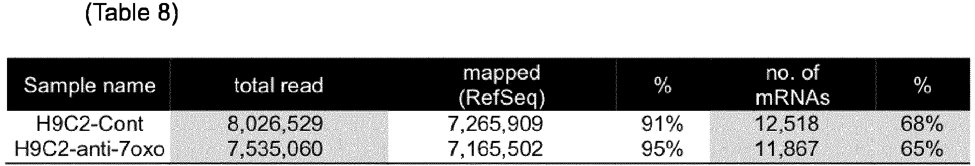

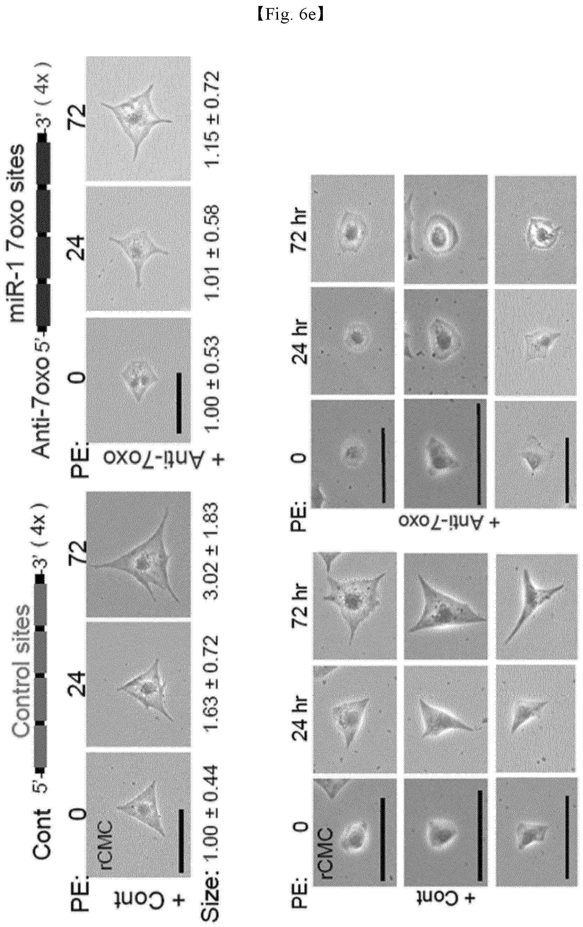

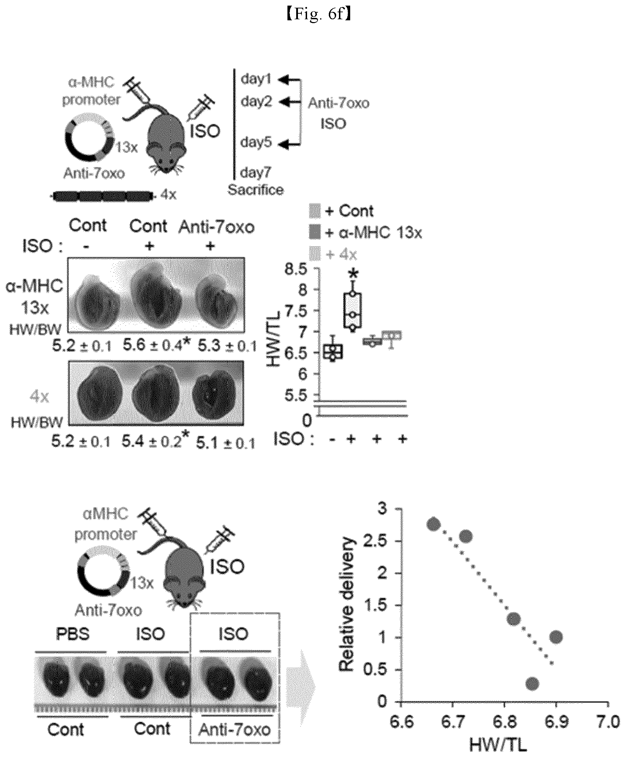

Then, as shown in FIG. 6E, anti-7oxo (4x) was introduced into rCMC and was shown to attenuate PE-induced hypertrophy. Both of anti-7oxo (4x and α-MHC 13x)) injected into ISO-treated mice also as synthesized anti-7oxo (4x) RNA or as cardiomyocyte-specific expression vector (α-MHC 13x) containing 13 target sites potently antagonized adrenergic cardiac hypertrophy as shown in FIG. 6F, and a degree of delivery of anti-7oxo (α-MHC 13x) was confirmed and was observed to correlate with their inhibitory activity.

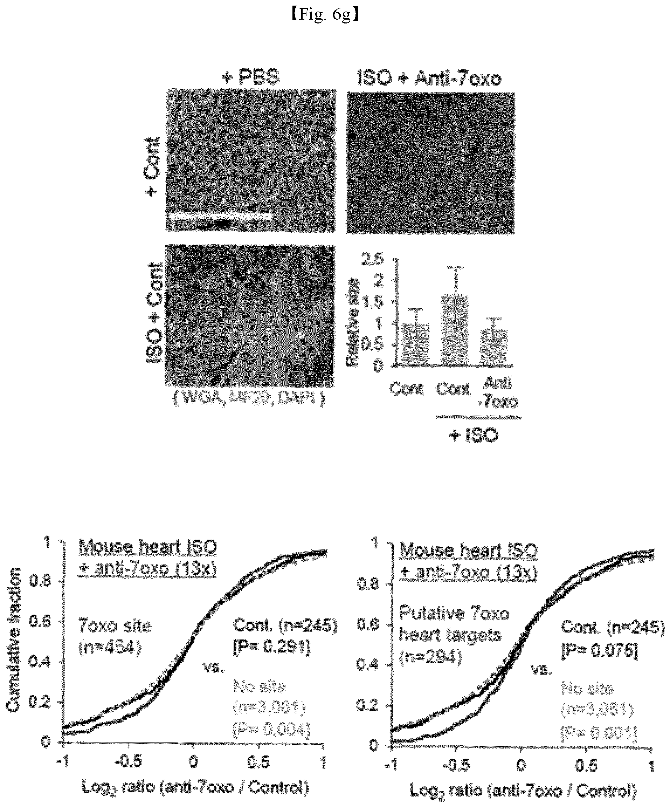

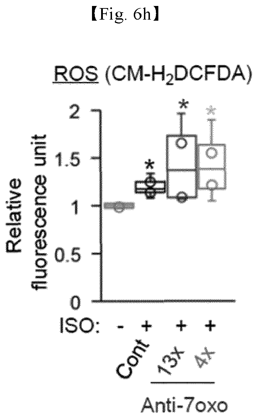

Administration of anti-7oxo (α-MHC 13x) maintained myocardial cell size as shown in FIG. 6G (top of FIG. 6G) and totally inhibited the miR-1:7o⁸G target (see bottom of FIG. 6G and Table 9). However, as shown in FIG. 6H, there was no effect on ROS increase in ISO-treated mouse hearts.

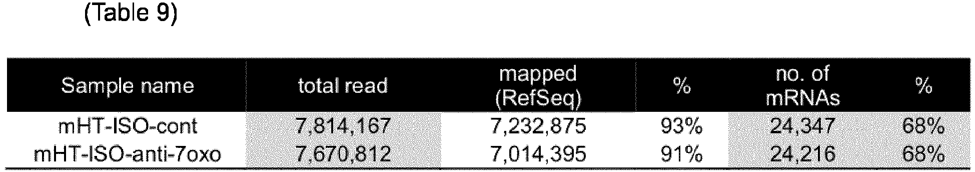

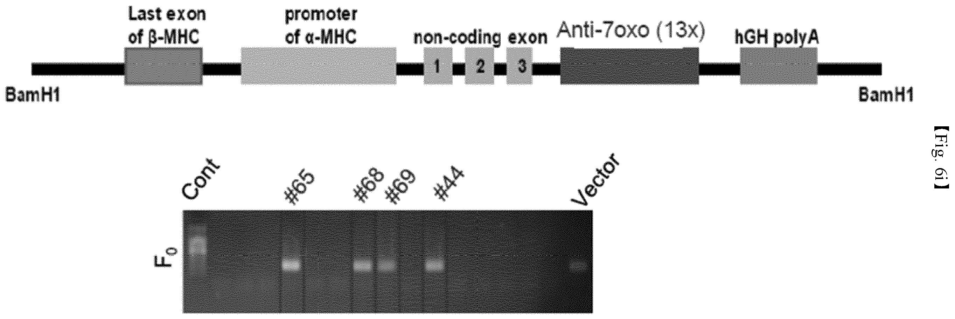

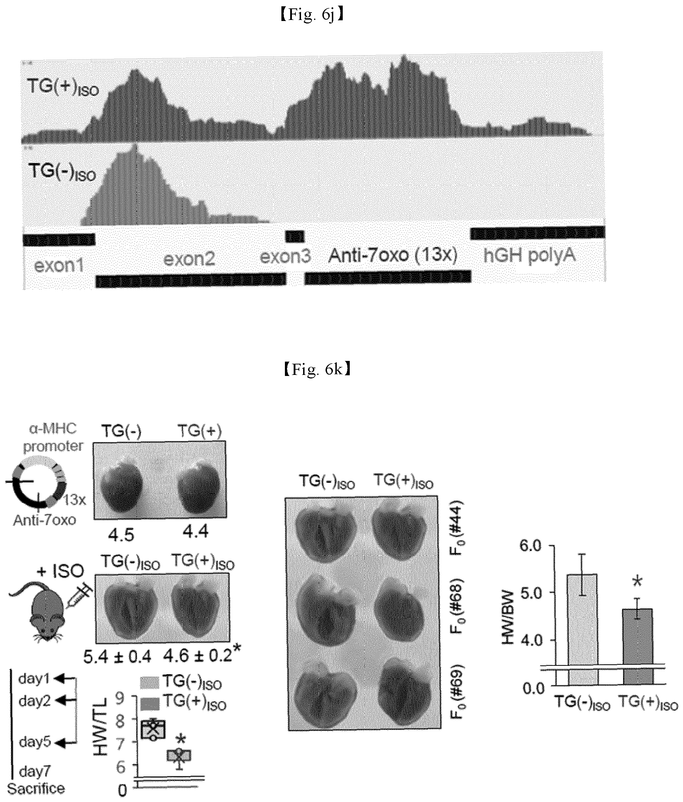

In addition, as shown in FIG. 6I, transformed mouse expressing anti-7oxo (α-MHC 13x) were generated (TG), and their expression was confirmed as shown in Table 10 and FIG. 6J (RNA-Seq).

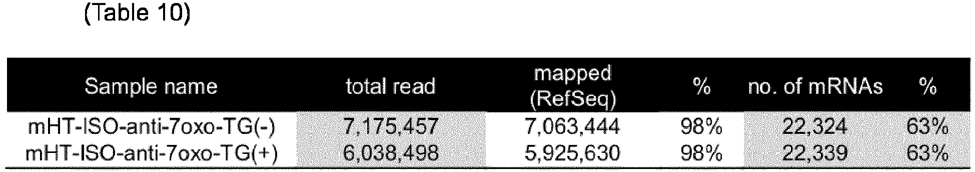

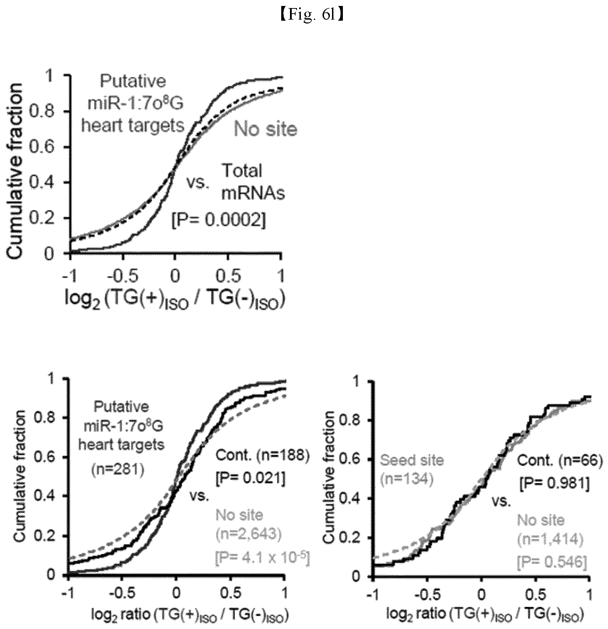

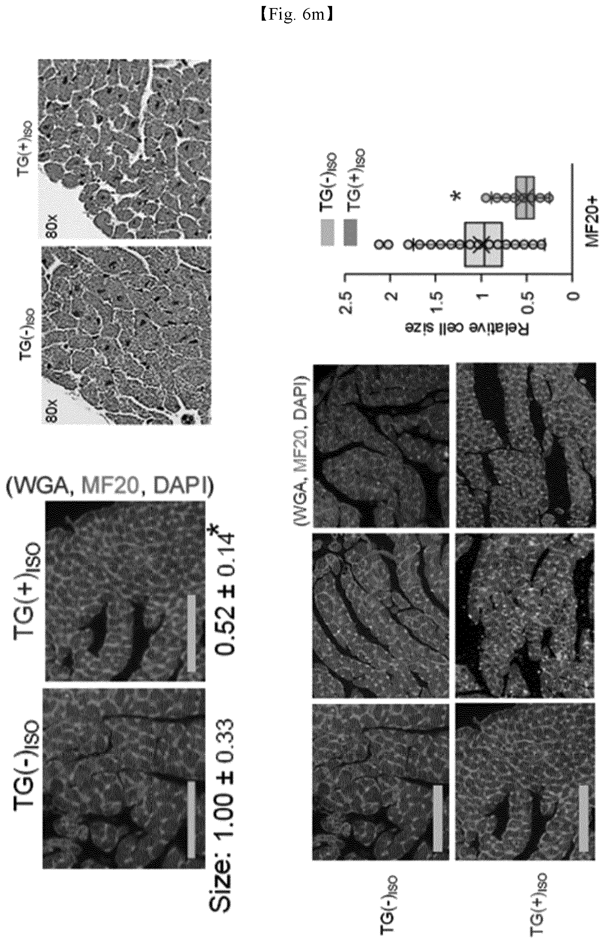

As a result, there was no basal difference in their heart size as shown in FIG. 6K (top of FIG. 6K), but ISO-induced cardiac hypertrophy was significantly prevented in all three different TG models (bottom of FIG. 6K), overall inhibition of the miR-1:7o⁸G target is shown as shown in FIG. 6L, and the sizes of cardiomyocytes were decreased in the IS even in the presence of ISO treatment (TG(+)_{ISO} vs. TG(-)_{ISO}) as shown in FIG. 6M.

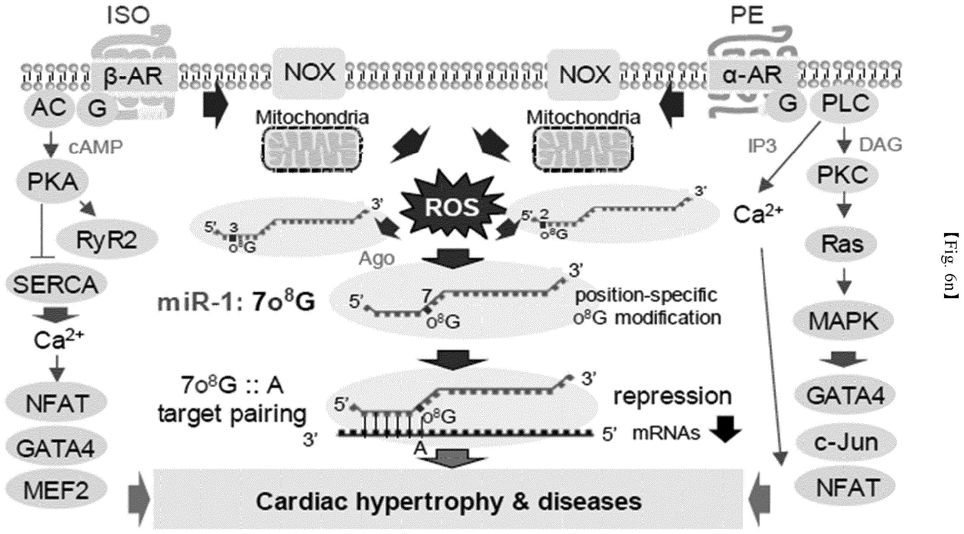

Taken together, site-specific oxidation of miR-1, specifically miR-1:7o⁸G, serves as an endogenous driver of cardiac hypertrophy and disease, suggesting that it generates and alters target interactions in cardiomyopathy patients. In this regard, schematic views of the site-specific oxidation of miR-1:7o⁸G according to the present invention induced by ROS and its cardiac hypertrophy induction process are shown in FIG. 6N.

### Experimental Example 7. Confirmation of miR-1:o⁸G increase in Plasma of Animal Model of Cardiac Hypertrophy

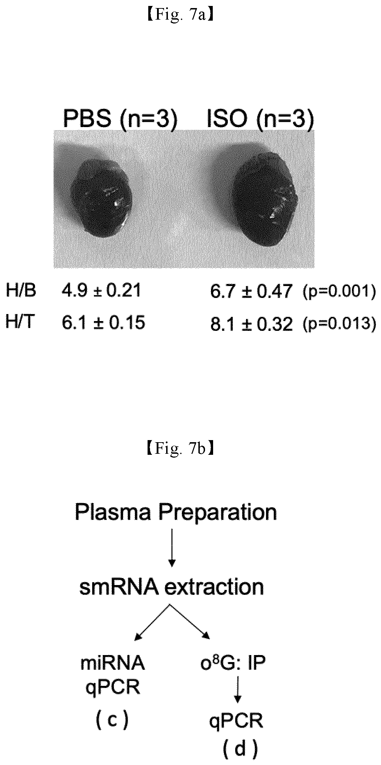

After observing that the o⁸G modification of miR-1 is increased in cardiac tissues of cardiac hypertrophy models and myocardial hypertrophy patients, in order to confirm that cardiac hypertrophy can be diagnosed by detecting it non-invasively, the o⁸G modification of miR-1 was intended to be detected in the blood of mice induced with cardiac hypertrophy. First, it was confirmed whether cardiac hypertrophy of the mouse was induced through ISO treatment. FIG. 7A is a representative cardiac photograph of cardiac hypertrophy induced through ISO, with 3 mice in each group. The heart size of the experimental group treated with ISO increased by about 30 % (36 % H/B vs. 32 % H/T) on average compared to the control. The details thereof are recorded in Table 11. In addition, to determine whether miR-1:o⁸G was measured in the blood of the animal model and whether it was enriched for cardiac hypertrophy, plasma was isolated and miR-1:o⁸G was quantified (n=3).

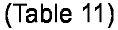

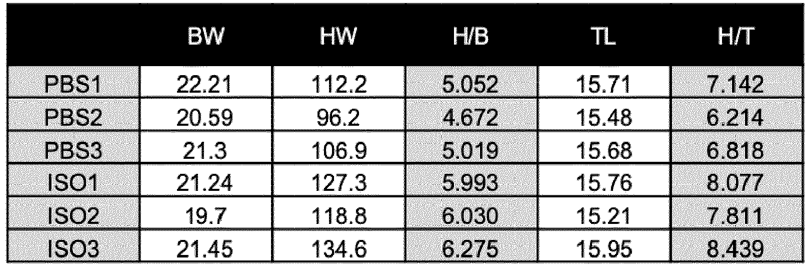

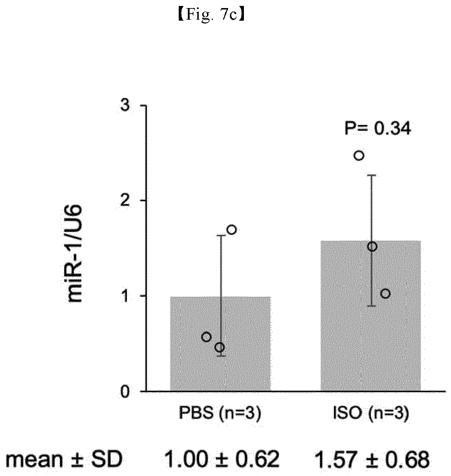

Then, after isolating plasma from the blood isolated from each animal model and extracting small RNA, an amount of miR-1 in the isolated small RNA was measured, and at the same time, an amount of o⁸G-modified miR-1 was measured through immunoprecipitation for o⁸G (FIG. 7B). As a result of measuring the amount of miR-1 from RNA extracted from plasma with the same amount, it could be confirmed that the measurement results between the experimental group and the control was not statistically significant, that miR-1 was present in the plasma, and that the corresponding amount existed regardless of the induction of cardiac hypertrophy (FIG. 7C).

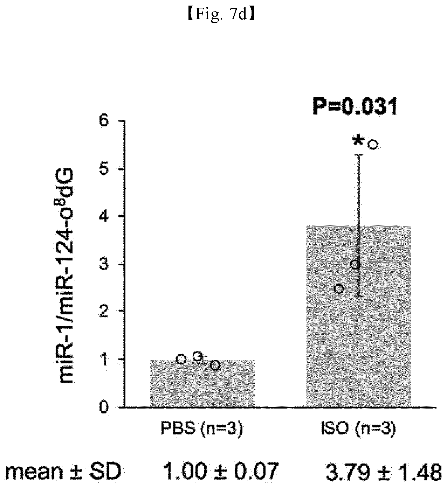

Thereafter, o⁸G-IP was performed on small RNA isolated from plasma to confirm that miR-1:o⁸G was present in plasma (FIG. 7B). At this time, in order to correct and quantify a difference in the o⁸G-IP process, human miR-124-3p synthesized as DNA at position 5 of o⁸G was added to the small RNA sample in the same amount before immunoprecipitation and used. As a result, the oxidatively modified miR-1:o⁸G observed in cardiac hypertrophy plasma increased by about 379 % on average in each of the three animals, and this change was confirmed to be statistically significant through Student's t-test (p = 0.031) (FIG. 7D). Taken together, the microRNA-1 measured by qPCR in plasma showed no difference between cardiac hypertrophy and the control, but oxidatively modified microRNA-1 (miR-1:o⁸G) measured by o⁸G-IP-qPCR showed that cardiac hypertrophy increased significantly, and through this, it was observed that cardiac hypertrophy can be diagnosed through miR-1:o⁸G measurement based on blood non-invasively.

### Experimental Example 8. Effect of antioxidants on myocardial hypertrophy and improved myocardial cell hypertrophy inhibitory ability of anti-miR-1-7oxo, which inhibits miR-1:7o⁸G during antioxidant treatment

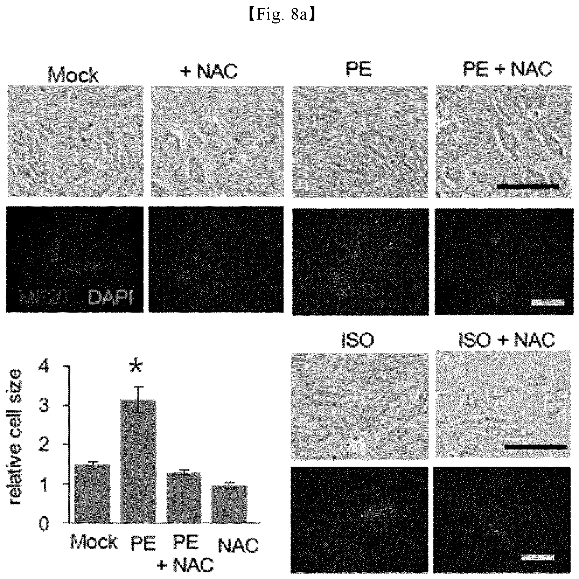

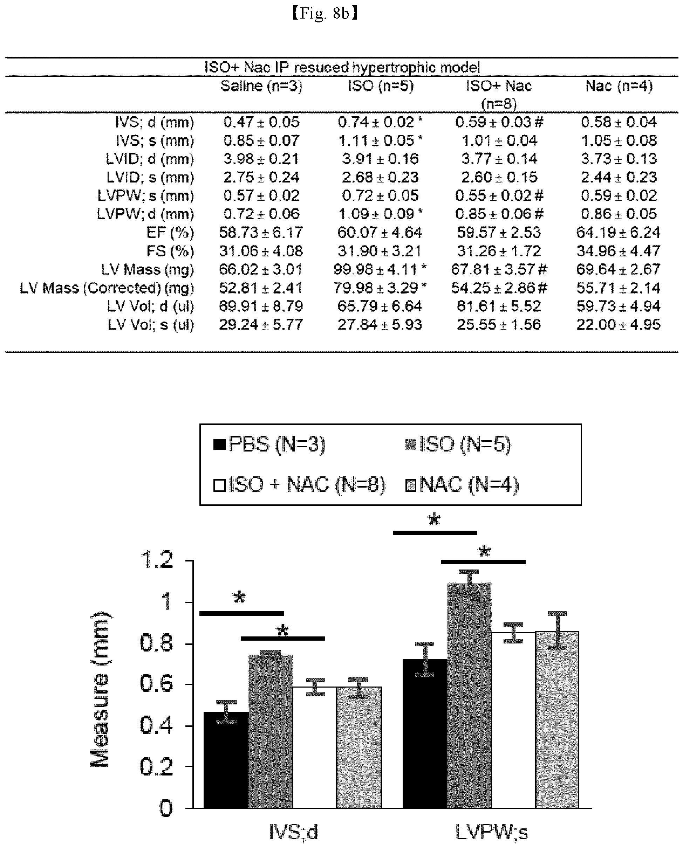

When the hypertrophy cells induced by PE treatment were treated with the antioxidant NAC, as shown in FIG. 8A, it was confirmed that the hypertrophy was reduced (n=4; inch², ImageJ), and as shown in FIG. 8B, it was confirmed that simultaneous treatment of ISO and NAC attenuated ISO-induced cardiac hypertrophy.

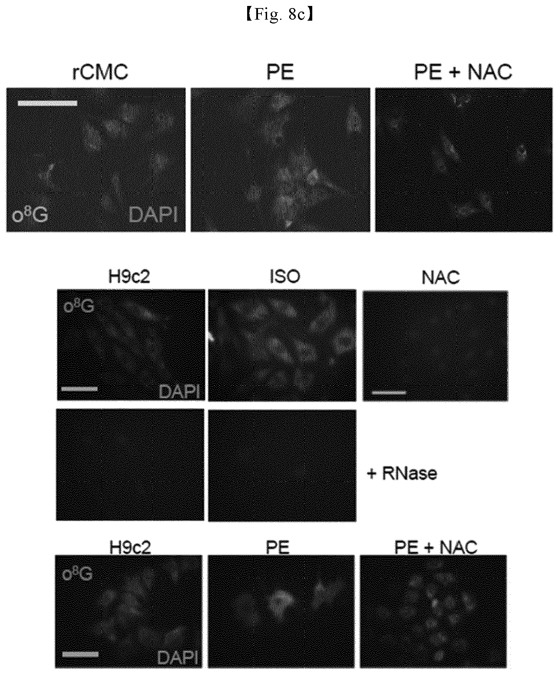

In addition, as shown in FIG. 8C, it was confirmed that the increase in o⁸G by ISO or PE treatment decreased with the treatment of NAC (scale bar, 100 µm). That is, the oxidation of miRNA was reduced according to the treatment of the antioxidant NAC.

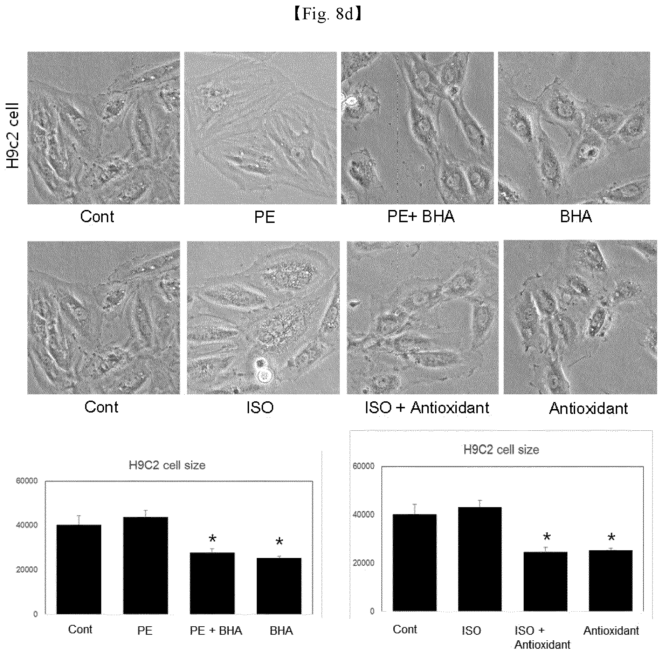

After observing the suppression of cardiac hypertrophy by ISO by NAC treatment (FIG. 1B), in order to check whether cardiac hypertrophy can be inhibited in the same way through other antioxidant treatment, H9c2 cells were treated with PE and ISO to induce hypertrophy of cardiomyocytes, and other antioxidants such as butylated hydroxyanisole (BHA) and Sigma-Aldrich's Antioxidant Supplement (A1345), which are sold as antioxidants for cell culture, were treated (FIG. 8D). As a result, regardless of the type of antioxidant, it was confirmed that the size of myocardial cells was reduced, and in particular, the size of the H9c2 cardiomyocyte line did not increase at all even when PE or ISO was treated, statistically significantly in three repeated experiments (*, P<0.01).

In addition, it was confirmed that hypertrophy occurred in the cells by PE treatment after culturing primary cardiomyocytes (rCMC) in rat embryos (FIG. 8E, upper panel). Here, when an inhibitory anti-7oxo (4x) including multiple 7oxo sites that recognizes miR-1 to inhibit the o⁸G-modified form (miR-1:7o⁸G) was introduced, it was confirmed again that myocardial cell hypertrophy was inhibited after progressing, and when NAC that is an antioxidant was additionally treated, the maximum effect in which cardiomyocytes did not enlarge at all was observed (FIG. 8E, middle panel). These results may be caused by the synergistic effect of antioxidants with anti-7oxo(4x), and may also be an effect shown by preventing o⁸G oxidation, which may additionally occur due to free radicals increased by PE treatment after introduction of anti-7oxo (4x) into cells, from occurring in anti-7oxo (4x). In fact, in order to find out whether additional o⁸G production occurs in RNA artificially introduced into cells and inhibits the effect, and thus in order to induce cardiomyocyte hypertrophy by introducing miR-1:7o⁸G rather than PE treatment, which generates active oxygen, and to simultaneously apply oxidative stress, 100 µM hydrogen peroxide (H₂O₂) was treated (FIG. 8E, lower panel). As a result, it was observed that myocardial cell hypertrophy caused by the existing miR-1:7o⁸G expression was inhibited by hydrogen peroxide treatment. On the contrary, it was confirmed that the myocardial cell hypertrophy induced by miR-1:7o⁸G appeared more efficiently when NAC, an antioxidant, was treated.

Therefore, based on these results, it is confirmed that not only NAC, but also BHA and other general antioxidants for cell culture, might inhibit the myocardial cell hypertrophy, if antioxidant treatment could exhibit antioxidant effects, and it was found that the treatment of antioxidants could effectively prevent additional oxidative modifications that may occur in the o⁸G-modified microRNA or RNA that inhibits it to induce regulation of cardiomyocyte size through artificial RNA expression. In particular, cardiac hypertrophy is characterized by physiological and pathological hypertrophy. The physiological hypertrophy may be necessary to temporarily induce it for the purpose of strengthening the function of the heart, depending on the situation, when the heart enlarges to provide a smooth supply when sufficient blood supply is needed in the case of athletes or pregnant women. Therefore, the induction of cardiac hypertrophy due to miR-1:7o⁸G introduction into cardiomyocytes may act as such physiological hypertrophy, and at this time, the antioxidant treatment can effectively induce the effect of myocardial hypertrophy, and may generally have an effect of inhibiting pathological cardiomegaly by preventing additional oxidative stress that causes pathological phenomena.

The description of the present invention described above is for illustration, and those of ordinary skill in the art to which the present invention pertains can understand that it can be easily modified into other specific forms without changing the technical spirit or essential features of the present invention. Therefore, it should be understood that the embodiments described above are illustrative in all respects and not restrictive.

## Claims

1. An RNA interference-inducing nucleic acid, comprising
at least one 8-oxoguanine (o⁸G) among the 1st to 9th nucleotides from a 5'-end of at least one single strand of double strands thereof.

2. The RNA interference-inducing nucleic acid of claim 1,
wherein the 1st to 9th nucleotides from the 5'-end comprise a sequence of a microRNA.

3. The RNA interference-inducing nucleic acid of claim 2,
Wherein the microRNA is selected from the microRNAs of Group 1:
[Group 1]
miR-1, miR-184, let-7f-5p, miR-1-3p, miR-122, let-7, and miR-124.

4. The RNA interference-inducing nucleic acid of claim 1,
wherein the RNA interference-inducing nucleic acid comprises an o⁸G:A arrangement at the position of 8-oxoguanine (o⁸G) to recognize a target site.

5. The RNA interference-inducing nucleic acid of claim 1, comprising a polynucleotide selected from Group 2:
[Group 2]
a polynucleotide of SEQ ID NO: 1 (5'p-Uo⁸GGAAUGUAAAGAAGUAUGUAU-3');
a polynucleotide of SEQ ID NO: 2 (5'p-UGo⁸GAAUGUAAAGAAGUAUGUAU-3');
a polynucleotide of SEQ ID NO: 3 (5'p-UGGAAUo⁸GUAAAGAAGUAUGUAU-3');
a polynucleotide of SEQ ID NO: 65 (5'p-Uo⁸Go⁸GAGUGUGACAAUGGUGUUUG-3');
a polynucleotide of SEQ ID NO: 66 (5'p-UGAo⁸GUAGUAGGUUGUAUAGdTdT-3'); and
a polynucleotide of SEQ ID NO: 67 (5'p-UAAo⁸GGCACGCGGUGAAUGCdTdT-3').

6. The RNA interference-inducing nucleic acid of any one of claim 1 to claim 5, wherein the RNA interference-inducing nucleic acid induces myocardial hypertrophy, inhibition of migration of liver cancer cells, or apoptosis, when being injected to a cell or an animal.

7. A composition comprising the RNA interference-inducing nucleic acid of claim 1 and an antioxidant.

8. A method for identifying a position of 8-oxoguanine (o⁸G), comprising:
(a) extracting RNA from a cell;
(b) isolating RNA that comprises 8-oxoguanine (o⁸G) from the extracted RNA by immunoprecipitation (IP) using an anti-o⁸G antibody;
(c) producing cDNA by reverse transcription of the isolated RNA that comprises 8-oxoguanine (o⁸G) to produce and sequence a sequencing library for determining the position of 8-oxoguanine; and
(d) identifying the position at which guanine (G) is substituted with thymine (T) as the position where guanine (G) is modified to 8-oxoguanine (o⁸G).

9. A modified nucleic acid that specifically binds to a modified microRNA in which at least one guanine (G) among the 1st to 9th nucleotides from the 5'-end is modified to an 8-oxoguanine (o⁸G),
wherein the modified nucleic acid comprises a polynucleotide complementary to 6 or more consecutive polynucleotides starting from the second or third nucleotide from the 5'-end of the modified microRNA, and
the modified nucleic acid comprises adenine (A) that binds to the at least one 8-oxoguanine (o⁸G) in the 1st to 9th nucleotides from the 5'-end of the modified microRNA.

10. The modified nucleic acid of claim 9, wherein
in the modified microRNA, at least one guanine (G) of the 2nd, 3rd, or 7th nucleotide from the 5'-end is modified to 8-oxoguanine (o⁸G),
the modified nucleic acid comprises a polynucleotide complementary to 6 or more consecutive polynucleotides starting from the 2nd or 3rd nucleotide from the 5'-end of the modified microRNA, and
the modified nucleic acid comprises adenine (A) that binds to the 8-oxoguanine (o8G).

11. The modified nucleic acid of claim 9, wherein
the modified nucleic acid comprises any one base sequence of 5'-ACAUUCA-3', 5'-ACAUUAC-3', or 5'-AAAUUCC-3'.

12. A recombinant vector comprising a gene encoding the modified nucleic acid of any one of claim 9 to 11.

13. A pharmaceutical composition for treating cardiac hypertrophy, comprising
a modified nucleic acid that specifically binds to a microRNA in which at least one guanine (G) among the 1st to 9th nucleotides from the 5'-end thereof is modified to 8-oxoguanine (o⁸G), or a recombinant vector comprising a gene encoding the modified nucleic acid,
wherein the modified nucleic acid comprises a polynucleotide complementary to 6 or more consecutive polynucleotides starting from the second or third nucleeotide from the 5'-end of the microRNA, and the modified nucleic acid comprises adenine (A) that binds to the 8-oxoguanine (o⁸G) of the microRNA.

14. The pharmaceutical composition for treating cardiac hypertrophy of claim 13, wherein the microRNA is miR-1, miR-184, let-7f-5p, or miR-1-3p.

15. The pharmaceutical composition for treating cardiac hypertrophy of claim 13, further comprising an antioxidant.

16. A pharmaceutical composition for treating liver cancer or glioblastoma, comprising an RNA interference-inducing nucleic acid that comprises at least one 8-oxoguanine (o⁸G) in the 1st to 9th nucleotides from a 5'-end of at least one single strand of double strands thereof.

17. The pharmaceutical composition for treating liver cancer of claim 16, wherein the micro RNA is miR-122.

18. The pharmaceutical composition for treating liver cancer of claim 16, wherein the microRNA is let-7 or miR-124.

19. The pharmaceutical composition for treating liver cancer or glioblastoma of claim 16, further comprising an antioxidant.

20. A pharmaceutical composition for preventing or treating cardiac hypertrophy comprising an antioxidant as an active ingredient,
wherein the antioxidant inhibits oxidative modification of one or more guanine (G) to 8-oxoguanine (o⁸G) among the 1st to 9th nucleotides from a 5'-end of an RNA.

21. The pharmaceutical composition of claim 20, wherein the antioxidant is N-acetylcysteine (NAC) or butylated hydroxyanisole (BHA).

22. The pharmaceutical composition of claim 20, wherein the RNA is miR-1, miR-184, let-7f-5p, or miR-1-3p.

23. A method for providing information for diagnosing cardiac hypertrophy, comprising:
determining whether a guanine (G) among nucleotides of a microRNA isolated from a cardiomyocyte of an animal is modified to 8-oxoguanine (o⁸G); and
classifying as cardiac hypertrophy when a guanine (G) of the nucleotides of the microRNA is modified to 8-oxoguanine (o⁸G).

24. The method for providing information for diagnosing cardiac hypertrophy of claim 23, wherein the nucleotides are the 1st to 9th nucleotides from the 5'-end of the microRNA.

25. The method for providing information for diagnosing cardiac hypertrophy of claim 23, wherein the nucleotides are the 2nd, 3rd, and 7th nucleotides from the 5'-end of the microRNA.

26. The method for providing information for diagnosing cardiac hypertrophy of any one of claim 23 to 25, wherein the microRNA is miR-1, miR-184, let-7f-5p, or miR-1-3p.

27. A method for producing a non-human animal model resistant to cardiac hypertrophy, comprising:
(a) operably linking a gene encoding the modified nucleic acid of claim 8 to a promoter to construct a recombinant vector;
(b) introducing the recombinant vector into a fertilized egg of an animal; and
(c) generating the fertilized egg after transplanting into a surrogate mother to obtain a transgenic animal model.

28. A non-human animal model resistant to cardiac hypertrophy produced by the method of claim 27.

29. A method for screening a candidate substance for treatment of cardiac hypertrophy, comprising:
(a) treating a cardiomyocyte of an animal model of cardiac hypertrophy with candidate substance;
(b) analyzing frequency of modification of guanine (G) to 8-oxoguanine (o⁸G) among nucleotides of microRNA expressed in the cardiomyocyte of the animal model of cardiac hypertrophy; and
(c) selecting the candidate substance as a cardiac hypertrophy therapeutic agent, when the frequency of modification of guanine (G) to 8-oxoguanine (o⁸G) decreases compared to the case in which the candidate substance is not treated.

30. The method for screening a candidate substance for the treatment of cardiac hypertrophy of claim 29, wherein the candidate substance is an antioxidant.
